# EUROPEAN PATENT SPECIFICATION

(11) **EP 3 745 862 B1**
(45) Date of publication and mention of the grant of the patent: **26.06.2024**
(21) Application number: 19747631.0
(22) Date of filing: 31.01.2019
(51) Int. Cl.: A61K 31/337, A61K 31/37, A61K 31/4188, A61K 31/137, A61K 31/4045, A61K 31/428, A61K 31/438, A61K 31/4745, A61K 31/48, A61K 31/485, A61K 31/506, A61K 31/55, A61P 11/00, A61P 43/00, A61P 9/00, A61P 13/00, A61P 17/00, C07D 311/04, C07D 311/02, A01N 43/54

(54) **D1 DOPAMINE RECEPTOR AGONISTS FOR TREATING FIBROTIC PATHOLOGIES**
D1 DOPAMIN-REZEPTOR-AGONISTEN ZUR BEHANDLUNG VON FIBROTISCHEN ERKRANKUNGEN
AGONISTES DES RÉCEPTEURS DOPAMINERGIQUES D1 POUR TRAITER LES PATHOLOGIES FIBROTIQUES

(30) Priority: 31.01.2018 US 201862624535 P
(43) Date of publication of application: 09.12.2020
(73) Proprietor: Mayo Foundation for Medical Education and Research, Rochester, MN 55905 (US)
(72) Inventor: TSCHUMPERLIN, Daniel J., Rochester, Minnesota 55906 (US); HAAK, Andrew J., Rochester, Minnesota 55902 (US)
(74) Representative: Fish & Richardson P.C.
(86) International application number: PCT/US2019/016178
(87) International publication number: WO 2019/152733

(56) References cited:
- WO-A1-96/38435
- WO-A2-2017/132661
- US-A1- 2016 032 384
- US-B2- 8 859 001
- DATABASE BIOSIS [Online] BIOSCIENCES INFORMATION SERVICE, PHILADELPHIA, PA, US; August 2004 (2004-08), TOWNSEND M ET AL: "Constrictive pericarditis and pleuropulmonary fibrosis secondary to cabergoline treatment for Parkinson's disease", XP002802205, Database accession no. PREV200400405407 & TOWNSEND M ET AL: "Constrictive pericarditis and pleuropulmonary fibrosis secondary to cabergoline treatment for Parkinson's disease", HEART (LONDON), vol. 90, no. 8, August 2004 (2004-08), ISSN: 1355-6037
- MIRONES et al.: "Dopamine Mobilizes Mesenchymal Progenitor Cells Through D2-Class Receptors and Their PI3K/AKT Pathway", Stem Cells, vol. 32, no. 9, 8 May 2014 (2014-05-08), pages 2529-2538, XP055628362, ISSN: 1066-5099, DOI: 10.1002/stem.1745

## Description

### CLAIM OF PRIORITY

This application claims priority to U.S. Patent Application Serial No. 62/624,535, filed on January 31, 2018.

### TECHNICAL FIELD

This invention relates to treating diseases and conditions associated with tissue fibrosis, for example, by using compounds that inhibit YAP/TAZ in fibroblasts.

### BACKGROUND

Tissue fibrosis across all organs affects a vast population of people. In the U.S. alone over half a million people are affected by liver (>400k) and lung (> 100k) fibrosis. These diseases remain very challenging to treat clinically. In examples such as idiopathic pulmonary fibrosis (IPF) and scleroderma, therapeutic options are extremely limited. In fact, for this group of diseases, the five year survival rate can be as bleak as many late stage aggressive cancers. WO 2017 /132661 A2 describes that imipridones selectively modulate Class A G protein-coupled receptors (GPCRs), such as the D2-like subfamily of dopamine receptors, and are useful for the treatment of conditions and disorders in need of such modulation, such as cancers, psychiatric disorders, and bacterial infections. WO 96/38435 A1 describes dopamine agonists that are useful for treating dopamine-related neurological, psychological, cardiovascular disorders, cognitive impairment, attention deficit disorder, and substance abuse disorders. Townsend et al. describe in Heart, vol. 90, no. 8, in August 2004 that an inflammatory reaction was cause by cabergoline in a patient suffering from Parkinson's disease.

### SUMMARY

The present invention is defined by the independent claim. The dependent claims depict additional embodiments of the invention.

Tissue fibrosis is characterized by uncontrolled deposition and diminished clearance of fibrous connective tissue proteins, and ultimately leads to fatal, end-stage organ scarring. Yes-associated protein 1 (YAP) and transcriptional coactivator with PDZ-binding motif (TAZ) play a role in the mesenchymal cell activation that drives tissue fibrosis¹⁻⁴. YAP and TAZ are downstream transcriptional effectors of multiple pro-fibrotic stimuli in mesenchymal cells⁵, and their expression in other cells and tissues is essential to regeneration and homeostasis⁶, complicating efforts to target them therapeutically⁷.

In one general aspect, a composition for use in a method of treating or preventing a fibrotic pathology is provided. The method includes administering to a subj ect in need thereof a therapeutically effective amount of a Gαₛ protein coupled receptor agonist, or a pharmaceutically acceptable salt thereof, wherein Gαₛ protein coupled receptor is preferentially expressed in mesenchymal cells as compared to epithelial or endothelial cells, and the agonist is specific for Gαₛ receptor that is expressed preferentially in the mesenchymal cell, the Gαₛ protein coupled receptor is a dopamine receptor, and the agonist agonizes both D1 and D5 dopamine receptors. The Gαₛ protein coupled receptor agonist is selected from dihydrexidine (DHX), SKF-81297, SKF-82958, SKF-38393, fenoldopam, 6-Br-APB, A-68930, A-77636, CY-208-243, pergolide, R(-)-2,10,11-trihydroxyaporphine, (R)-(-)-apomorphine, R(-)-propylnorapomorphine, R(+)-6-bromo-APB, R(-)-2,10,11 -trihydroxy-N-propyl-noraporphine, 6,7-ADTN, mesulergine, N-methyldopamine, 4-hydroxyphenethylamine, 3-hydroxyphenethylamine, pramipexole, PD-168077, (±)-PD 128-907, (±)-2-(N-phenylethyl-N-propyl)amino-5-hydroxytetralin, bromocriptine, ropinirole, LY-163-502, dipropyldopamine, B-HT 920, piribedil, (+)-UH 232, (-)-quinpirole, R(-)-2,11-dihydroxy-10-methoxyapomorphine, or a pharmaceutically acceptable salt thereof, or the Gαₛ protein coupled receptor agonist is a compound of Formula (I): 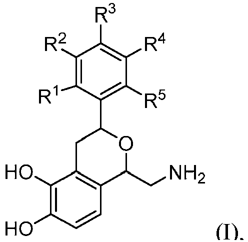 or a pharmaceutically acceptable salt thereof, where each of R¹, R², R³, R⁴ and R⁵ is independently selected from H, OH, C₁₋₆ alkoxy, C₁₋₆ haloalkoxy, HO-C₁₋₃ alkyl, NH₂-C₁₋₃ alkyl, HS-C₁₋₃ alkyl, SH, NH₂, C₁₋₆ alkylamino and di(C₁₋₆ alkyl)amino, optionally wherein at least one of R¹, R², R³, R⁴ and R⁵ is selected from OH, C₁₋₆ alkoxy, C₁₋₆ haloalkoxy, HO-C₁₋₃ alkyl, NH₂-C₁₋₃ alkyl, HS-C₁₋₃ alkyl, SH, NH₂, C₁₋₆ alkylamino and di(C₁₋₆ alkyl)amino. In some embodiments, at least two of R¹, R², R³, R⁴ and R⁵ are independently selected from OH, C₁₋₆ alkoxy, C₁₋₆ haloalkoxy, HO-C₁₋₃ alkyl, NH₂-C₁₋₃ alkyl, HS-C₁₋₃ alkyl, SH, NH₂, C₁₋₆ alkylamino and di(C₁₋₆ alkyl)amino. In some embodiments, at least one of R¹, R², R³, R⁴ and R⁵ is selected from NH₂ and OH. In some embodiments, the compound of Formula (I) is selected from any one of the following compounds: 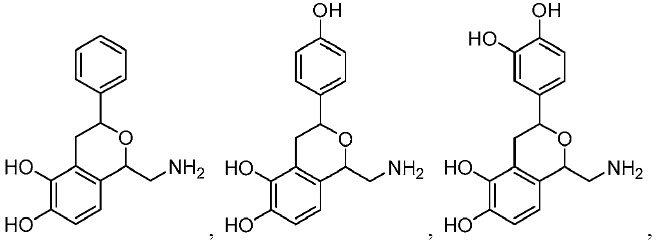 and 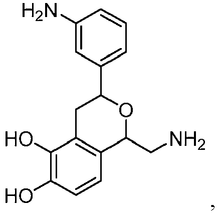 or a pharmaceutically acceptable salt thereof. In some embodiments, the mesenchymal cell is a fibroblast or a stellate cell. In some embodiments, the fibrotic pathology is selected from interstitial lung disease (ILD), pulmonary fibrosis (PF), idiopathic pulmonary fibrosis (IPF), liver tissue fibrosis, cardiac fibrosis, kidney fibrosis, and skin tissue fibrosis. In some embodiments, the method further includes administering to the subject a therapeutically effective amount of an additional therapeutic agent useful in treating a fibrotic pathology, optionally wherein the additional therapeutic agent is dopamine, or a pharmaceutically acceptable salt thereof.

In another aspect, an *in vitro* method is provided. The method includes agonizing a Gα_{S} protein coupled receptor in a cell; and/or promoting YAP/TAZ phosphorylation in a cell; and/or inhibiting YAP/TAZ function in a cell; and/or inhibiting expression of a profibrotic gene in a cell; and/or reducing nuclear localization of YAP/TAZ in a cell; and/or inhibiting expressing of α-smooth muscle actin (*αSMA*) in a cell; and/or inhibiting extra-cellular matrix production and deposition by a cell; and/or enhancing extra-cellular matrix degradation by a cell. The method further includes contacting the cell with an effective amount of a compound of Formula (I): 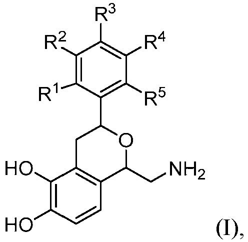 or a pharmaceutically acceptable salt thereof, where each of R¹, R², R³, R⁴ and R⁵ is independently selected from H, OH, C₁₋₆ alkoxy, C₁₋₆ haloalkoxy, HO-C₁₋₃ alkyl, NH₂-C₁₋₃ alkyl, HS-C₁₋₃ alkyl, SH, NH₂, C₁₋₆ alkylamino and di(C₁₋₆ alkyl)amino, with the proviso that the compound of Formula (I) is not any one of the following compounds:

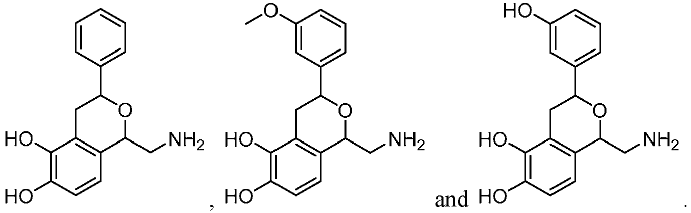

In some embodiments, at least two of R¹, R², R³, R⁴ and R⁵ are OH. In some embodiments, at least one of R¹, R², R³, R⁴ and R⁵ is NH₂. In some embodiments, the compound of Formula (I) is selected from any one of the following compounds: 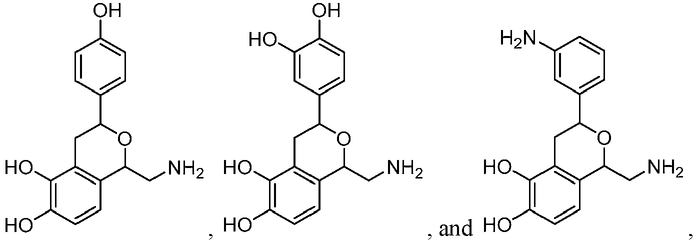 or a pharmaceutically acceptable salt thereof.

In another aspect, a compound of Formula (I): 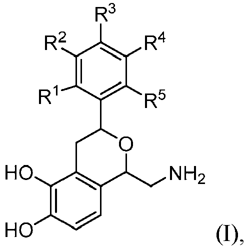 or a pharmaceutically acceptable salt thereof is provided. Each of R¹, R², R³, R⁴ and R⁵ is independently selected from H, OH, C₁₋₆ haloalkoxy, HO-C₁₋₃ alkyl, NH₂-C₁₋₃ alkyl, HS-C₁₋₃ alkyl, SH, NH₂, C₁₋₆ alkylamino and di(C₁₋₆ alkyl)amino, with the proviso that the compound of Formula (I) is not any one of the following compounds: 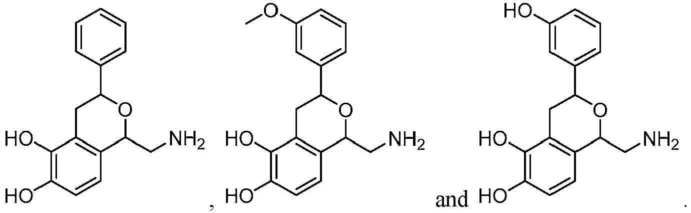 In some embodiments, the compound of Formula (I) is selected from any one of the following compounds: 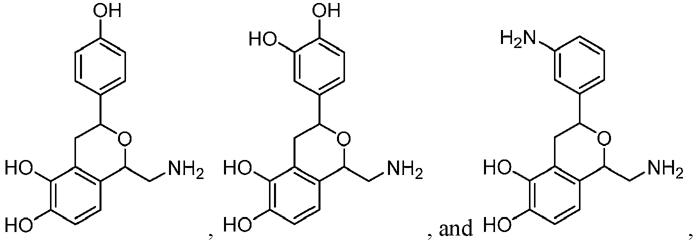 or a pharmaceutically acceptable salt thereof.
In some embodiments, R³ is OH. In some embodiments, at least two of R¹, R², R³, R⁴ and R⁵ are OH. In some embodiments, at least one of R¹, R², R³, R⁴ and R⁵ is NH₂.

Unless otherwise defined, all technical and scientific terms used herein have the same meaning as commonly understood by one of ordinary skill in the art to which the present application belongs. Methods and materials are described herein for use in the present application; other, suitable methods and materials known in the art can also be used. The materials, methods, and examples are illustrative only and not intended to be limiting. In case of conflict between the present specification and a reference mentioned herein, the present specification, including definitions, will control.

Other features and advantages of the present application will be apparent from the following detailed description and figures, and from the claims.

### DESCRIPTION OF DRAWINGS

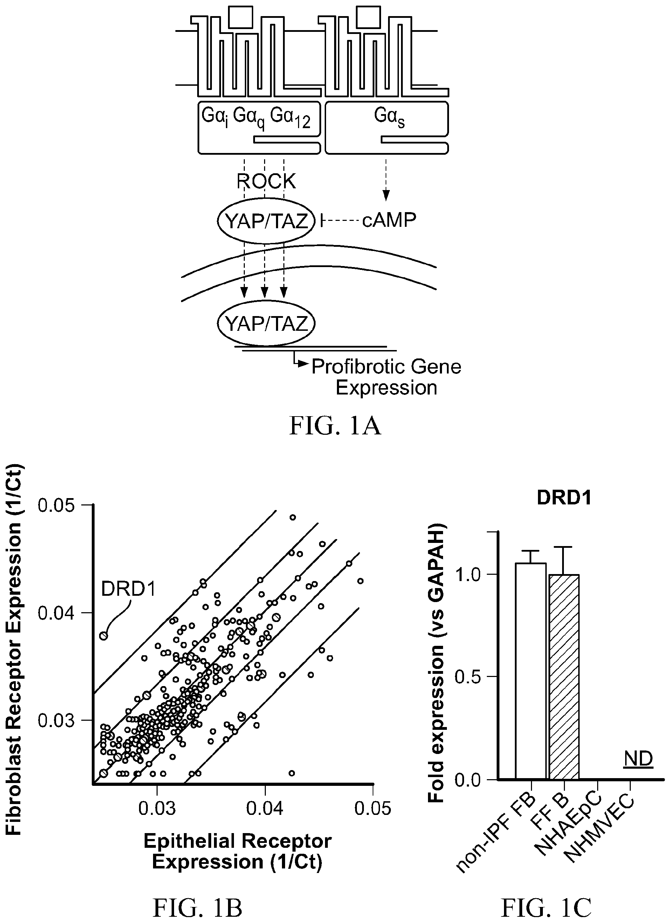

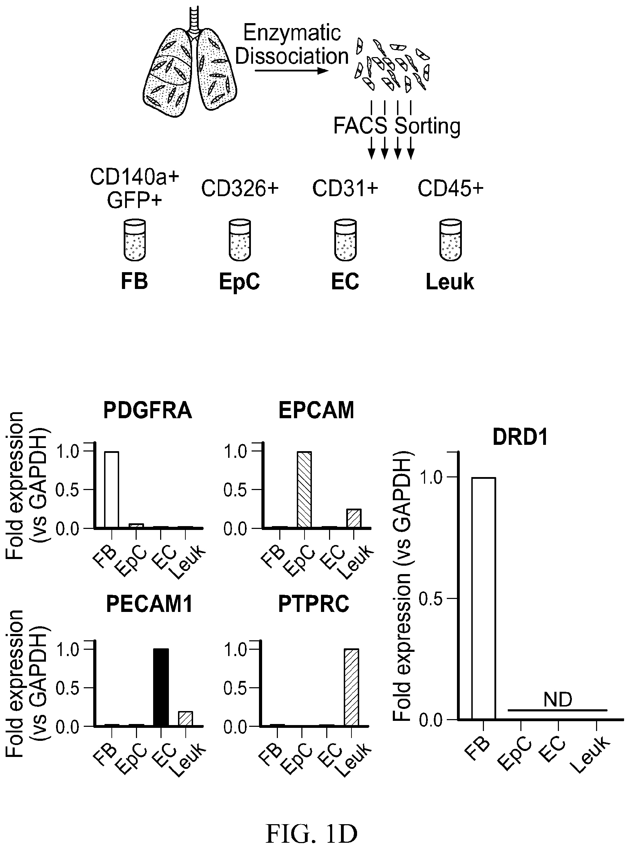

**FIG. 1** **Gaₛ-coupled Dopamine Receptor D1 is selectively expressed in pulmonary fibroblasts, a.** Regulation of YAP/TAZ transcription co-factor activity by GPCR signaling. Receptors which couple to Gαₛ elevate cAMP and induce phosphorylation of YAP/TAZ which blocks nuclear localization. Receptors which couple to Galphα_{i/q/12} promote nuclear localization and activity of YAP/TAZ, likely through Rho-kinase (ROCK). b. GPCR expression profiling of cultured human alveolar epithelial cells and normal human pulmonary fibroblasts. Dopamine Receptor D1 (*DRD1*) transcripts are highly expressed in fibroblasts and not detected in epithelial cells. Red points indicate GPCRs which selectively couple to Gαₛ. Orange diagonal line indicates 10-fold preferential expression, blue line 100-fold. **c.** *DRD1* expression in cultured non-IPF associated fibroblasts, IPF patient-derived fibroblasts, normal human alveolar epithelial cells (NHAEpC), and normal human microvasculature endothelial cells (NHMVEC), passage 6 or less. NHAEpC and NHMVEC, n=2. non-IPF FB and IPF FB, n=6. **d.** Expression of DRD1 in freshly isolated mouse lung fibroblasts (FB), epithelial (EpC), endothelial cells (EC), and leukocytes (Leuk). Lung from a healthy *COL1A1-GFP* expressing mouse was enzymatically digested then sorted for markers of fibroblasts (GFP+, CD140a+), epithelial cells (CD326+), endothelial cells (CD31+), and leukocytes (CD45+) followed by RNA isolation to validate selective populations and expression of *Drd1.*

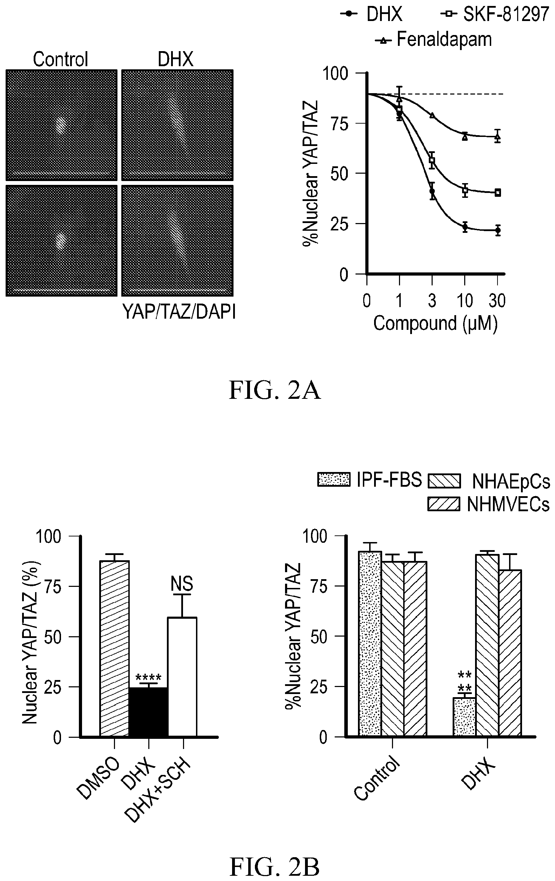

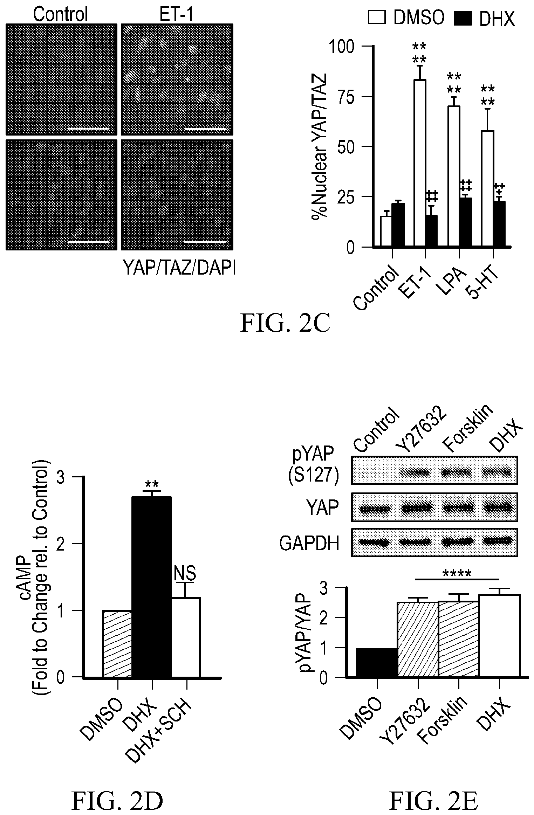

**FIG. 2** **Dopamine Receptor D1 agonism blocks YAP/TAZ nuclear localization, a.** Selective agonists of dopamine receptor D1 with varying efficacy inhibit YAP/TAZ nuclear localization; this effect can be overcome by treatment with a DRD1 receptor antagonist (SCH 39166, 3µM), (Dihydrexidine, 10µM). IMR-90 cells treated 2 hours prior to fixation. N=4 (**** *p* < 0.0001 vs. 0.1% DMSO vehicle control). Scale bar represents 100 µm. b. Consistent with DRD1 expression, DHX inhibits YAP/TAZ only in fibroblasts (IPF-FBs) but not in epithelial (NHEpCs) or endothelial (NHMVECs) cells. N=4 (**** *p* < 0.0001 vs. 0.1% DMSO vehicle control) **c.** YAP/TAZ localization is induced through multiple ligands which stimulate receptors coupled to Galphα_{i/q/12}, endothelin 1 (ET-1: 100nM), lysophosphatidic acid (LPA: 10µM), and serotonin (5-HT: 1µM). In each case DHX treatment (10µM) can reverse this effect on YAP/TAZ. IMR-90 cells plated densely onto plastic cell culture plates for 24 hours in media containing 0.1% FBS, treated for 2 hours prior to fixation. N=4 (*****p* < 0.0001 vs. 0.1% DMSO vehicle control), (++++ *p* < 0.0001 vs. the respective stimulated agonist ET-1, LPA, or 5-HT). Scale bar represents 100 µm. **d.** cAMP measured in IPF patient-derived fibroblasts treated for 20 minutes with DHX (10 µM) +/- SCH 39166 (3µM). N=3 (** *p* < 0.01 vs. 0.1% DMSO vehicle control). e. Phosphorylation of YAP by Rho-kinase inhibitor Y27632 (20µM), direct cAMP stimulator Forskolin (10µM), or DHX (10µM). IMR-90 cells cultured for 24 hours in media containing 0.1% FBS, treated 2 hours prior to fixation. N=3 (*** *p <* 0.001 vs. 0.1% DMSO vehicle control).

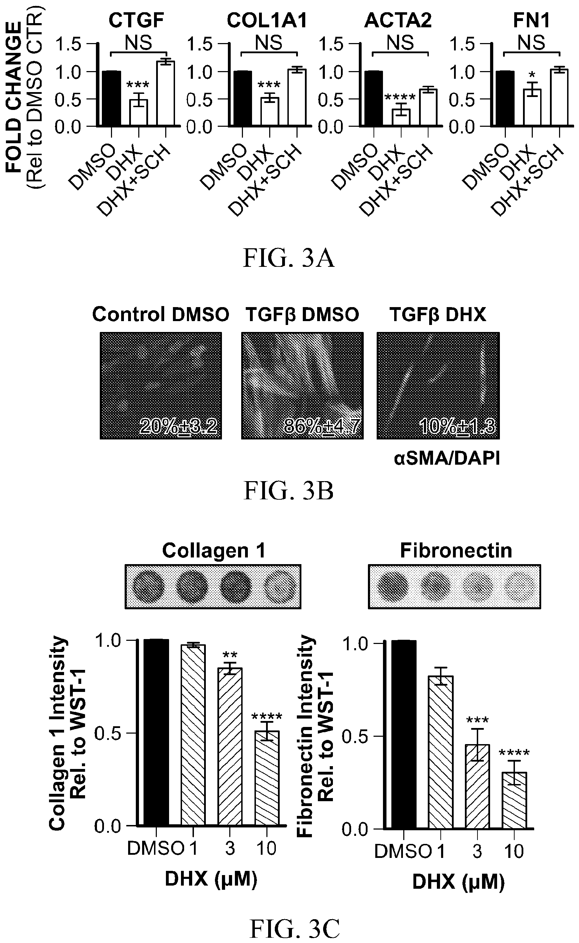

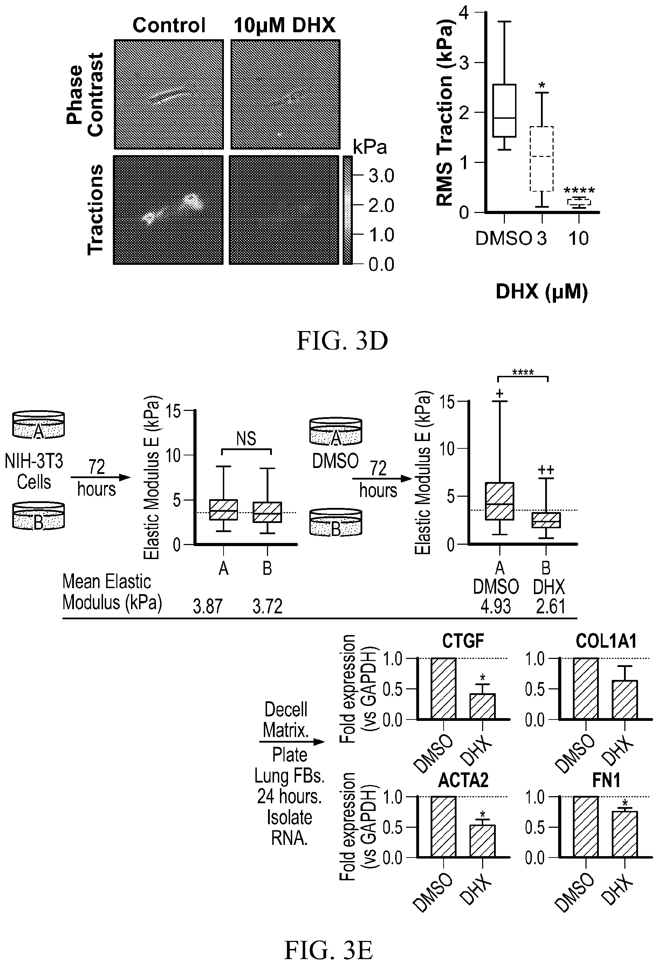

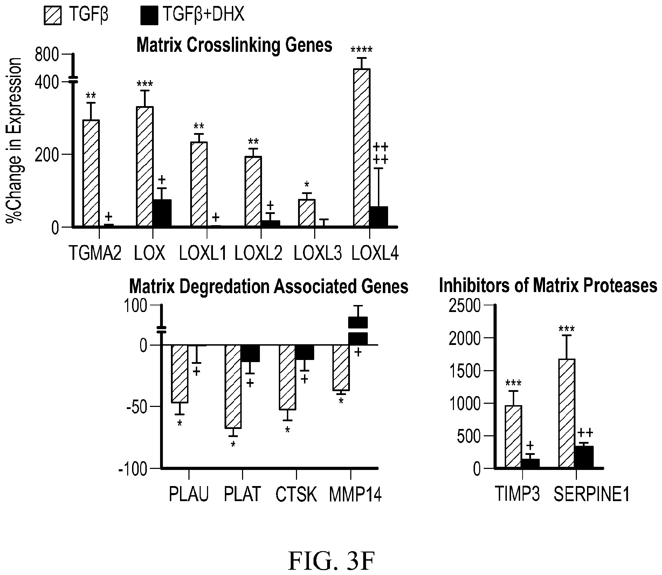

**FIG. 3** **DHX reverses fibroblast matrix deposition, contraction and stiffening, a.** DHX blocks profibrotic gene expression in IPF patient-derived fibroblasts. Genes which encode Connective tissue growth factor (*CTGF*), Collagen I *(COL1A1),* αSMA (*ACTA2*), and Fibronectin (*FN1*) are reduced by 24 hour treatment with DHX (10µM), +/- SCH 39166 (3µM). N=3 (**** *p <* 0.0001, *** *p <* 0.001, * *p* < 0.05 vs. 0.1% DMSO vehicle control) **b.** DHX reverses TGFβ-induced αSMA+ stress fiber formation. IMR-90 cells pre-stimulated with 2ng/mL TGFβ for 48 hours and then treated with DHX (10µM) +2ng/mL TGFβ for an additional 24 hours prior to fixation. Cells which are positive for αSMA were quantified by a blinded investigator and noted in the bottom right corner, a minimum of 300 cells in each experiment were quantified. N=3. c. DHX reverses TGFβ-induced extracellular matrix accumulation. IPF patient-derived fibroblasts grown at confluence were pre-stimulated with 2ng/mL TGFβ for 48 hours and then treated with DHX (10µM)+2ng/mL TGFβ for an additional 24 hours prior to fixation. Cell derived matrix is measured using antibodies for Collagen I and Fibronectin. N=3 (**** *p <* 0.0001, *** *p* < 0.001, ** *p* < 0.01 vs. 0.1% DMSO vehicle control) **d.** DHX attenuates IPF patient-derived fibroblast contractility measured by traction force microscopy. Representative traction maps are shown from cells plated onto 6.4kPa matrices treated with the indicated concentration of DHX. RMS tractions were determined in two independent experiments; box and whisker plots show min to max, quartile, and mean from one representative experiment (**** *p <* 0.0001, * *p* < 0.05 vs. 0.1% DMSO vehicle control). e. DHX reverses extracellular matrix stiffening. NIH-3T3 cells plated onto gelatin-coated tissue culture plates stimulated to deposit matrix with 2ng/mL TGFβ and 20µM ascorbic acid for 72 hours prior to AFM microindentation analysis to measure stiffness (elastic modulus). The same dishes were then treated +/- 10µM DHX in the same media for another 72 hours and AFM analysis. The matrices were decellularized and passage 3 NHLFs were plated onto the matrices; after 24 hours RNA was collected and expression of profibrotic genes was analyzed. AFM analysis N=2. 75 indentation measurements were made for each plate. The box and whisker plots show min to max, quartile, and mean from one representative experiment (**** *p <* 0.0001 vs. 0.1% DMSO vehicle control) (++ *p <* 0.01, + p < 0.05 vs. the same culture plate after the first 72 hour incubation). Measurement of RNA from cell plated onto decellularized matrices N=3. (* *p* < 0.05 vs. 0.1% DMSO vehicle control). **f.** DHX modulates matrix deposition vs. matrix degradation gene program. IPF patient-derived fibroblasts cultured in media containing 0.1% FBS were stimulated for 24 hours with 2ng/mL TGFβ and 10 µM DHX prior to RNA isolation. Genes which encode for ECM crosslinking: transglutaminase 2 (*TGM2*), lysyl oxidase and lysyl oxidase-like enzymes (*LOX* and *LOXLI-4),* ECM degradation: uPA (*PLAU*)*,* tPa (*PLAT*), cathepsin K (*CTSK*), and matrix metalloprotease-14 (*MMP14*) and ECM protease inhibitors: metalloprotease inhibitor 3 (*TIMP3*), and plasminogen activator inhibitor 1 (*SERPINEI*) were measured. N=3. (**** *p* < 0.0001, *** *p* < 0.001, ** *p* < 0.01, * *p* < 0.05 vs. 0.1% DMSO vehicle control non-TGFβ treated), (++++ *p <* 0.0001, ++ *p* < 0.01, + *p <* 0.05 vs. 0.1% DMSO vehicle control TGFβ treated).

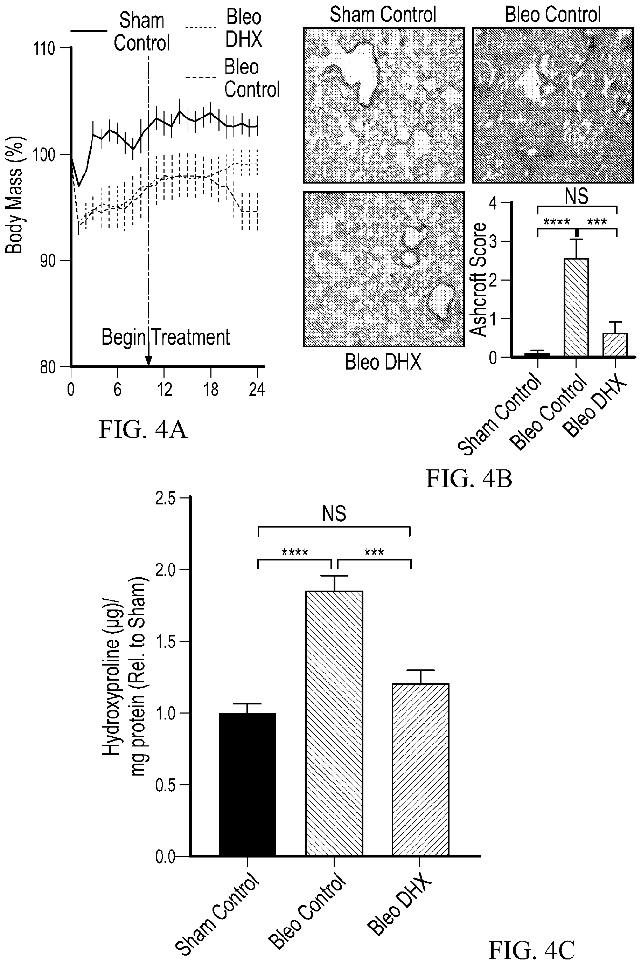

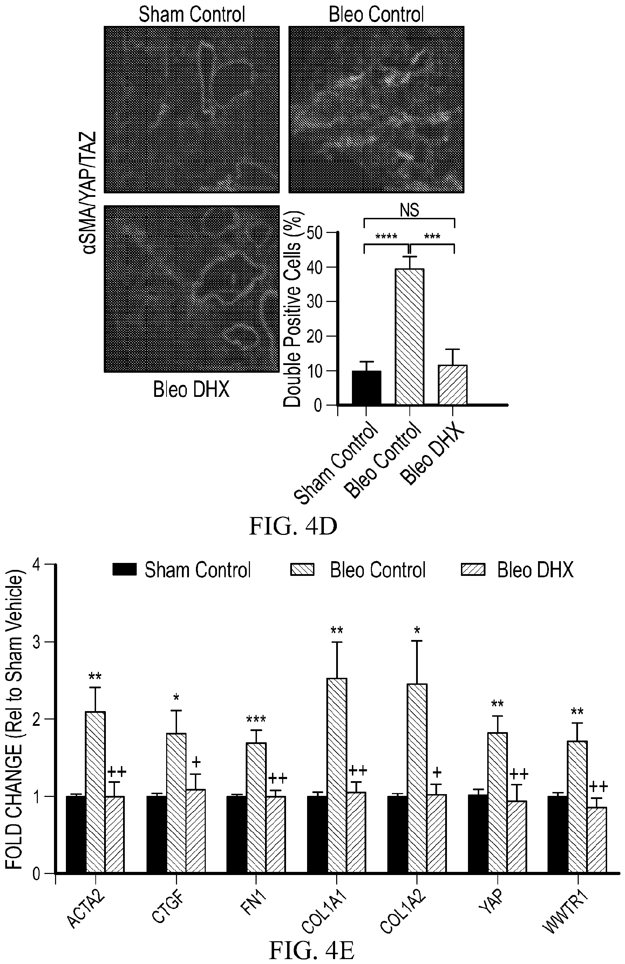

**FIG. 4** **DHX therapeutically reverses bleomycin-induced pulmonary fibrosis, a.** Weight change as a result of bleomycin induced lung injury and DHX therapeutic benefit. Female mice were intratracheally administered Bleomycin on Day 0; treatment was initiated on Day 10 (5 mg/kg DHX i.n., daily) and continued until day 24. **b.** H&E staining to visualize collagen and architectural changes. Paraffin embedded lung sections were stained and analyzed in a blinded fashion by a pulmonary pathologist and scored using the Ashcroft method, **c.** Hydroxyproline assay to measure collagen deposition in the lungs. Snap frozen lung tissue was biochemically analyzed for collagen abundance using the hydroxyproline assay. **d.** Immunofluorescence imaging of lung sections for αSMA and Yap/Taz. Lung sections were stained immune-probed for αSMA and Yap/Taz. Cells which were double positive for both αSMA and Yap/Taz were quantified using automated software **e.** Changes in pro-fibrotic gene expression, *Yap* and *Taz* (*Wwtr1*) in whole lung homogenates. Sham Control N=15, Bleo Control N=17, and Bleo DHX N=17. (**** *p <* 0.0001, *** *p* < 0.001, ** *p <* 0.01, * *p* < 0.05 vs. Sham Control) (++ *p* < 0.01, + p < 0.05 vs. the respective Bleo Control).

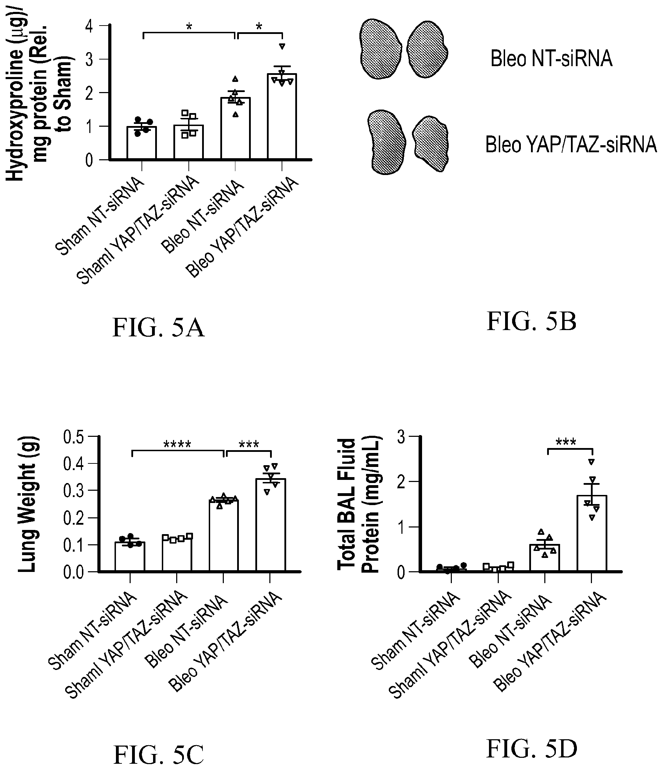

**FIG. 5** **Intratracheally administered YAP/TAZ siRNA worsens bleomycin induced lung injury and fibrosis.** Mice were administered bleomycin on Day 0 and then intratracheally administered siRNA for *Yap* and *Taz* on Day 14. On Day 21 BAL fluid was collected and lungs were harvested for analysis. *Yap*/*Taz* siRNA enhanced collagen deposition (a), vascular leak and lung injury **(b-d).** Sham treated mice N=4, Bleo treated mice N=6 (**** *p* < 0.0001, *** *p* < 0.001, ** *p* < 0.01, * *p* < 0.05 vs. Sham NT-siRNA).

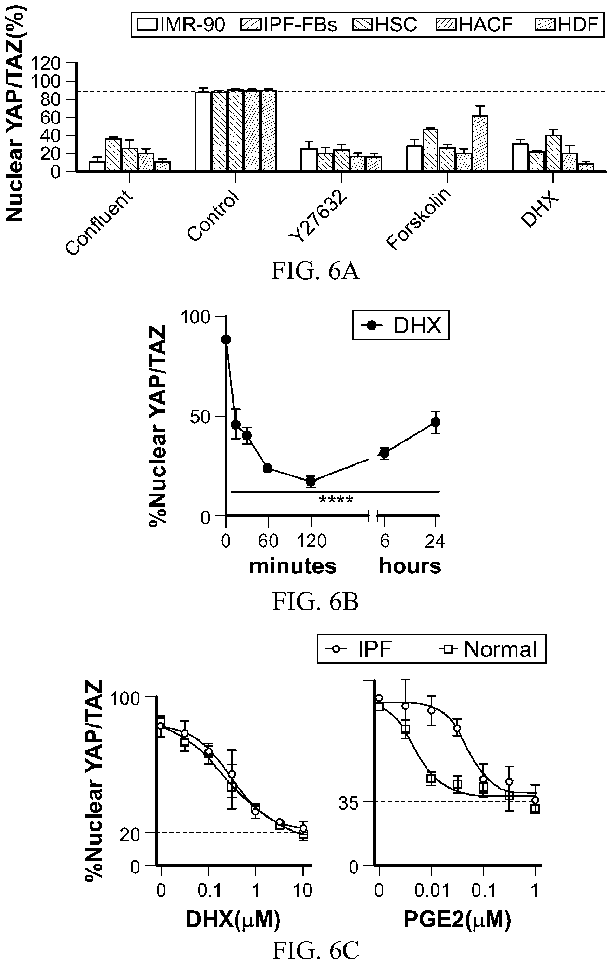

**FIG. 6** **DHX inhibits YAP/TAZ localization in fibroblasts from multiple tissues. a.** Mesenchymal cells derived from lung (IMR-90 and IPF-FBs) hepatic stellate cells (HSC), human adult cardiac fibroblasts, (HACF) and human dermal fibroblasts (HDF) were plated densely (Confluent) or sparsely (Control and all remaining conditions) onto plastic cell culture plates for 24 hours in media containing 0.1% FBS, treated for 2 hours with Rho kinase inhibitor Y27632, adenylate cyclase activator forskolin and DHX. N=4, Cells that were positive for nuclear YAP/TAZ were quantified by automated image analysis. **b.** Time course for DHX inhibition of YAP/TAZ nuclear localization. IMR-90 cells, N=3 (*** *p* < 0.001 vs. 0.1% DMSO vehicle control). **c.** DHX does not lose potency in IPF derived fibroblasts, unlike PGE2. N=3.

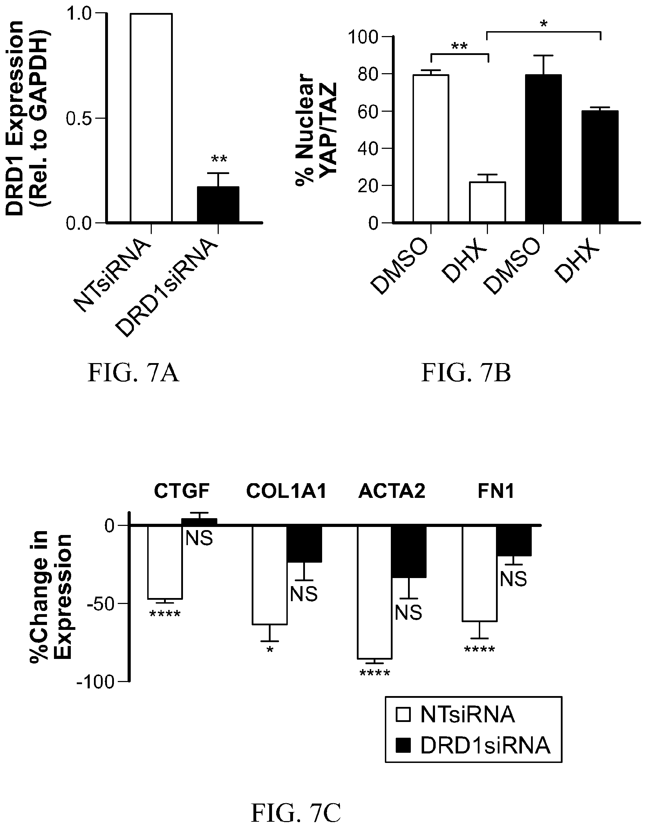

**FIG. 7** **DHX inhibits pro-fibrotic gene expression through DRD1 agonism. a.** siRNA treatment to knockdown *DRD1.* **b/c.** Reduced DHX-mediated inhibition of YAP/TAZ nuclear localization and pro-fibrotic gene expression in *DRD1*-siRNA treated cells. IMR-90 cells transfected with siRNA targeting *DRD1* or NTsiRNA for 72 hours prior to 2 hour (b) or 24 hour (c) treatment with DHX. N=2 for all (**** *p <* 0.0001, ** p < 0.01, * *p* < 0.05 vs. NTsiRNA).

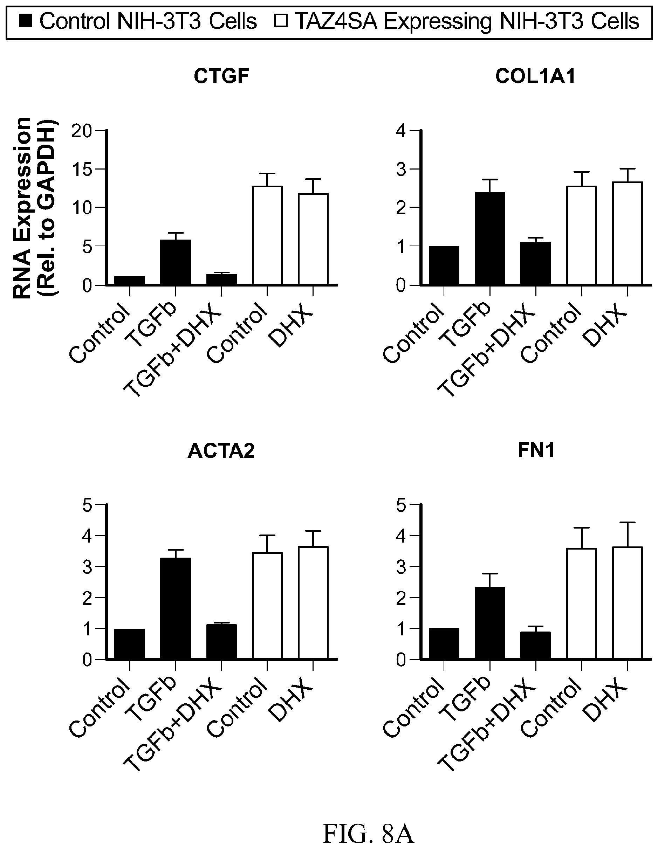

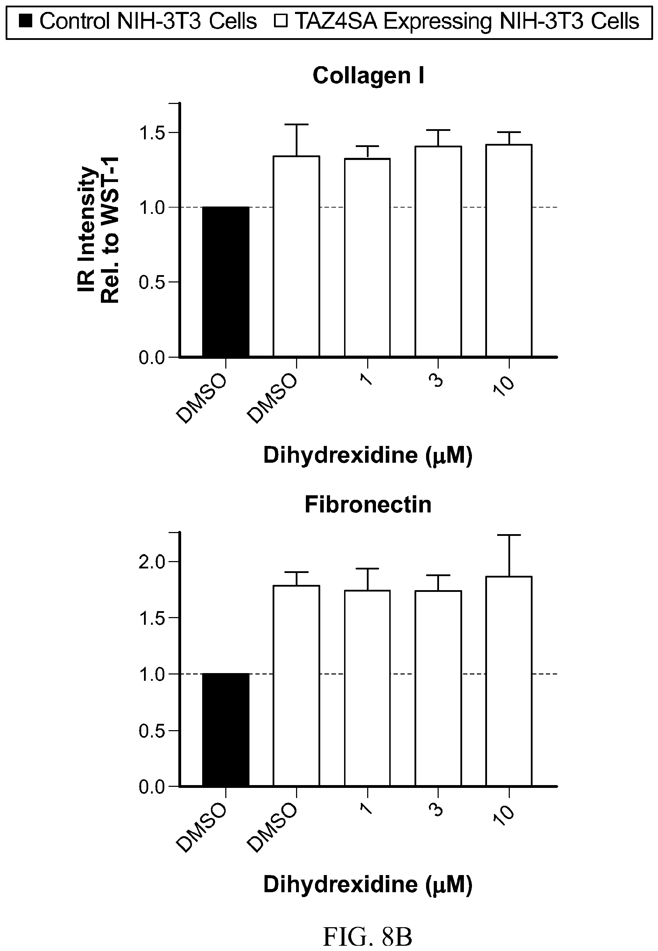

**FIG. 8** **DHX inhibition of pro-fibrotic gene expression and matrix deposition requires inhibition of YAP/TAZ.** DHX does not inhibit pro-fibrotic gene expression **(a)** and ECM deposition **(b)** when fibroblasts express a constitutively active mutant TAZ (TAZ4SA). TAZ4SA expression was induced with 100 ng/mL doxycycline for 72 hours prior to treatment with 10µM or the indicated concentration of dihydrexidine. For gene expression experiments efficacy of DHX in NIH-3T3 cells was validated with by DHX (10µM) effect on TGFβ (24 hour, 2ng/mL) induced gene expression. N=2 for all.

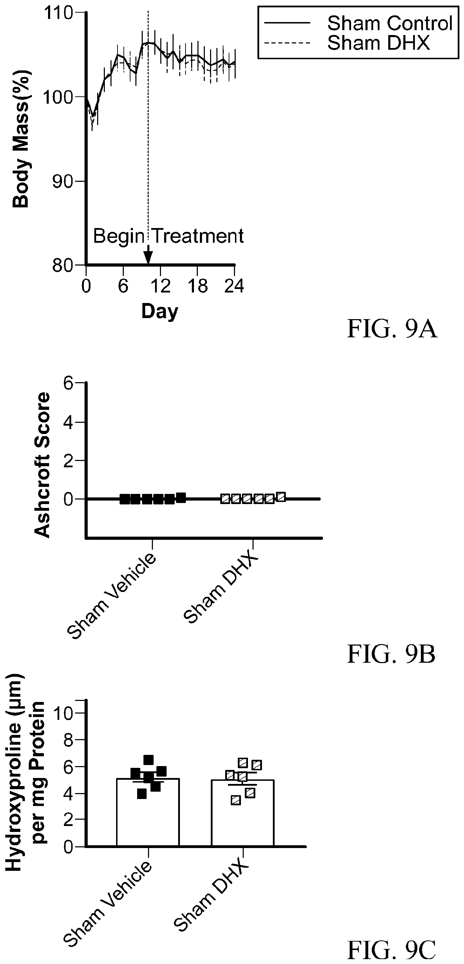

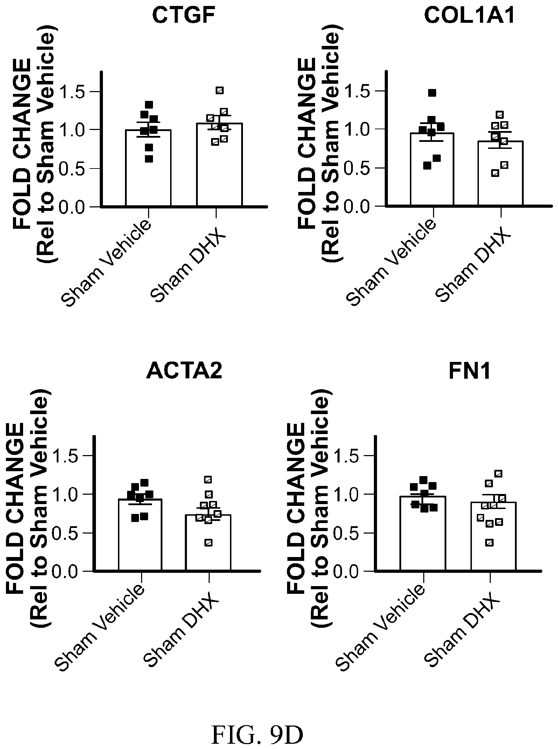

**FIG. 9** **DHX alone does not affect lung matrix content or profibrotic gene expression.** In normal healthy mice DHX did not alter body weight (a), lung histology (b), lung collagen deposition (c), or expression of *Ctgf, Col1a1, Acta2,* and *Fnl* (d). n = 6 mice per group.

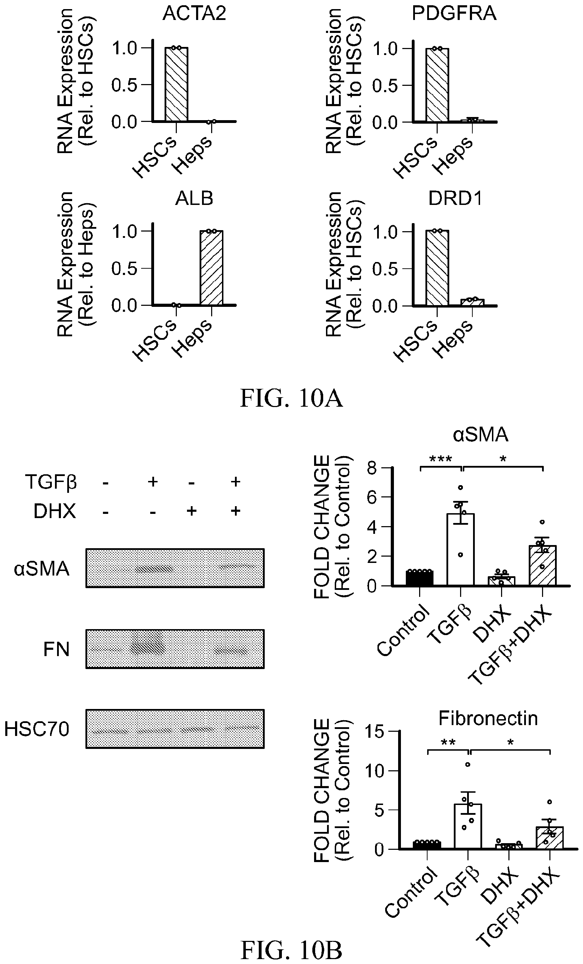

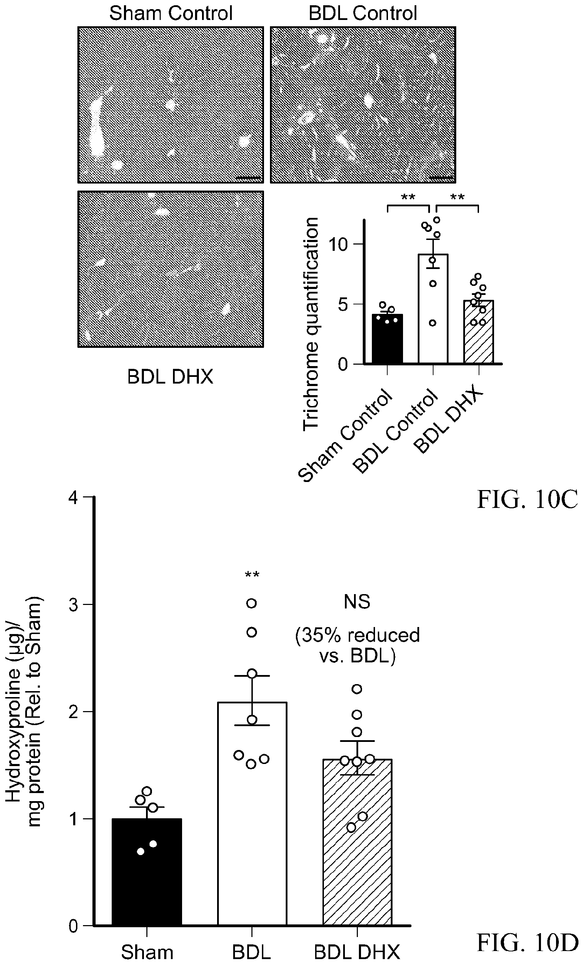

**FIG. 10** **DHX reverses hepatic stellate cell activation and in vivo hepatic fibrosis, a.** *DRD1* is preferentially expressed in cultured human hepatic stellate cells (HSCs): *ACTA2, PDGFRA* positive, relative to cultured human hepatocytes (Heps): *ALB* positive. N=1. **b.** DHX inhibits TGFβ-mediated hepatic stellate cell activation in vitro. HSCs were stimulated with TGFβ for 48 hours +/- DHX (10 µM) prior to total protein isolation and western blot analysis of αSMA and Fibronectin. N=3 (*** *p <* 0.001, ** *p* < 0.01, * *p* < 0.05 vs. Control + TGFβ). **c.** DHX reverses Bile duct ligation (BDL) mediated fibrosis in vivo measured by trichrome staining, and **d.** hydroxyproline. Sham Control N=5, BDL Control N=7, and BDL DHX N=8 (** *p <* 0.01 vs. BDL Control).

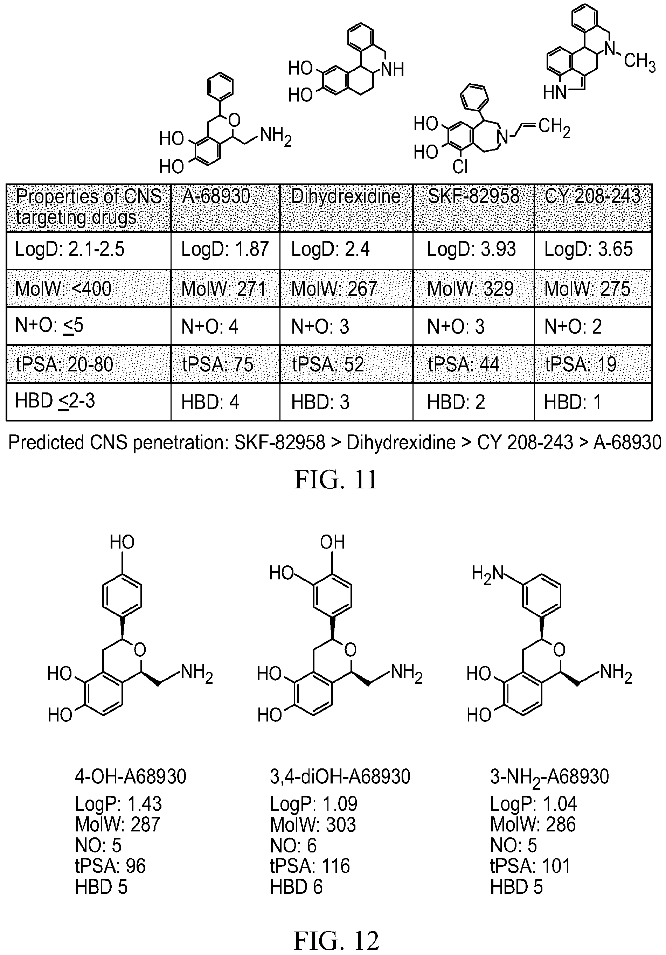

**FIG. 11** shows physical properties of selected D1 receptor agonists.
**FIG. 12** shows physical properties of selected analogs of A-68930.

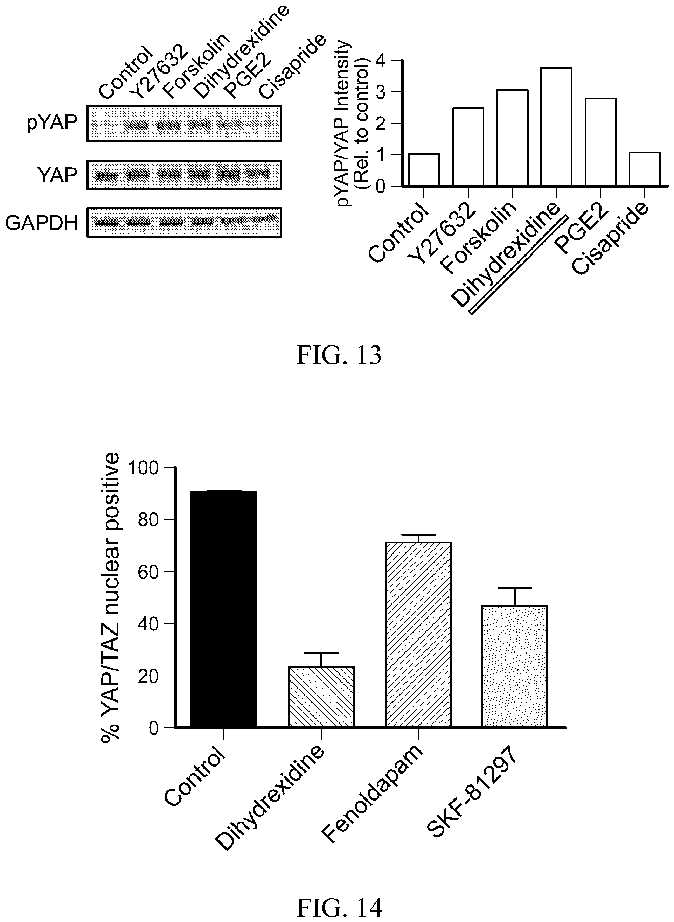

**FIG. 13** shows that YAP phosphorylation blocks nuclear localization of pYAP.
**FIG. 14** shows that D1 receptor agonists targeting the same receptor and having diverse structures produce similar effect.

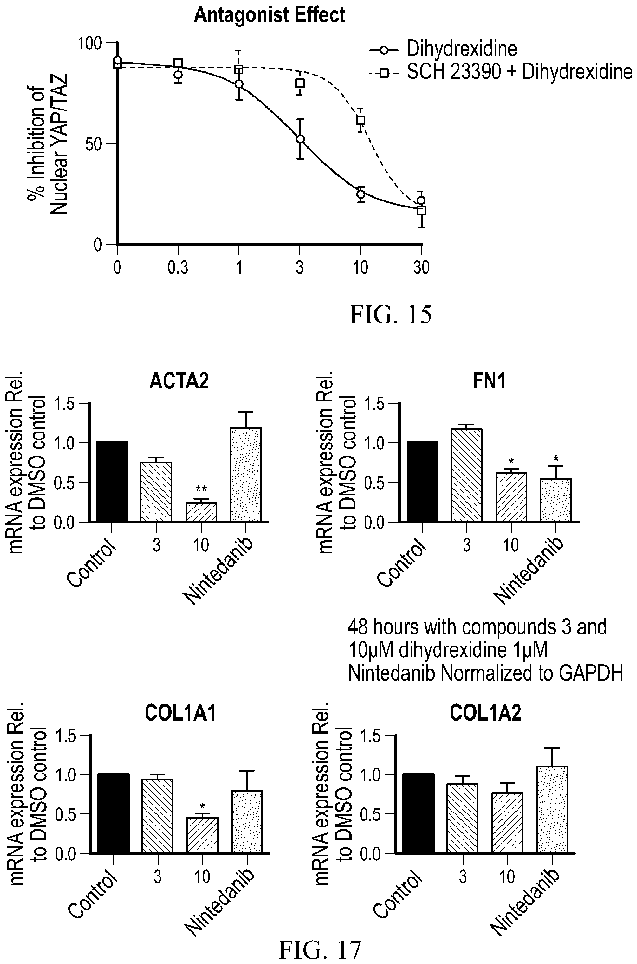

**FIG. 15** shows that YAP inactivation by DHX are based on D1 receptor activity.
**FIG. 17** shows that D1 receptor agonist reduces expression of multiple profibrotic genes in fibroblasts from patients with IPF.

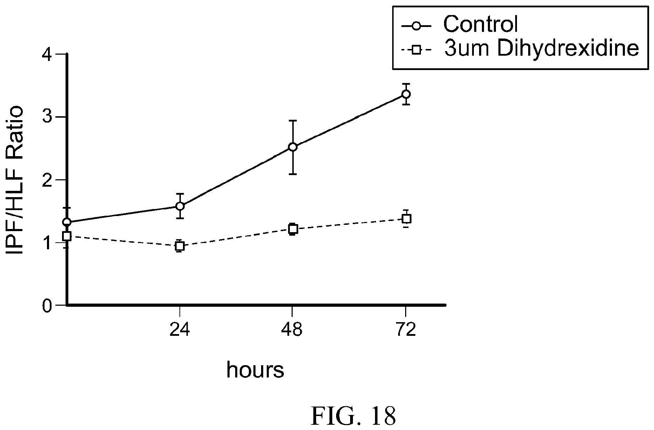

**FIG. 18** shows that D1 receptor agonist selectively slows proliferation in IPF fibroblasts. IPF fibroblast and Normal lung fibroblast co-culture proliferation. Cell are pre - labelled with fluorescent dyes (red and green) and then co - cultured at a Ratio of 1:1 in 96 well plates. Cell counts are determined every 24 hours and plotted as a ratio of IPF/HLF cells. In the control wells the IPF cells outgrow and take over the well. N=2.

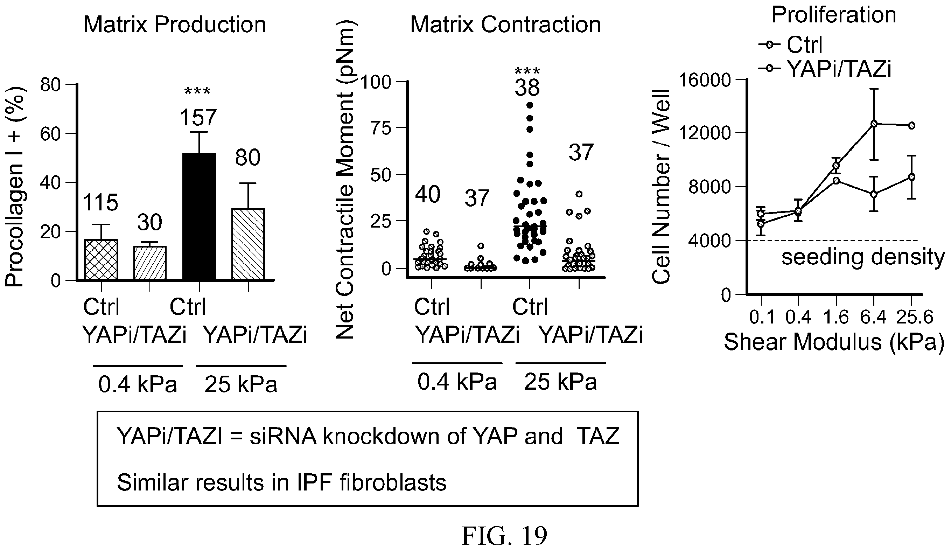

**FIG. 19** shows that YAP/TAZ are necessary for matrix stiffness-dependent fibroblast activation.

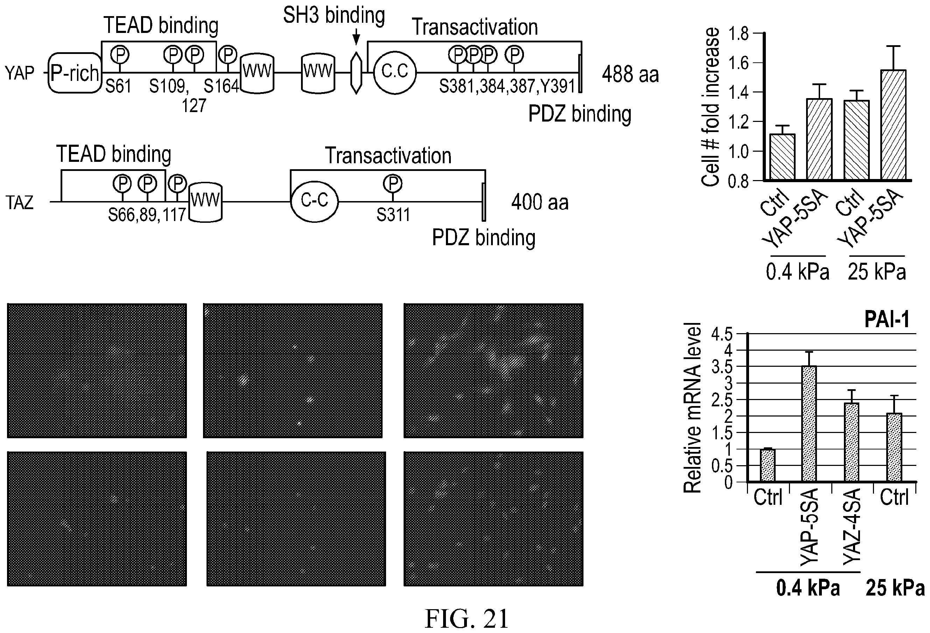

**FIG. 21** shows that mutant YAP/TAZ are active on soft matrices in NIH 3T3 fibroblasts.

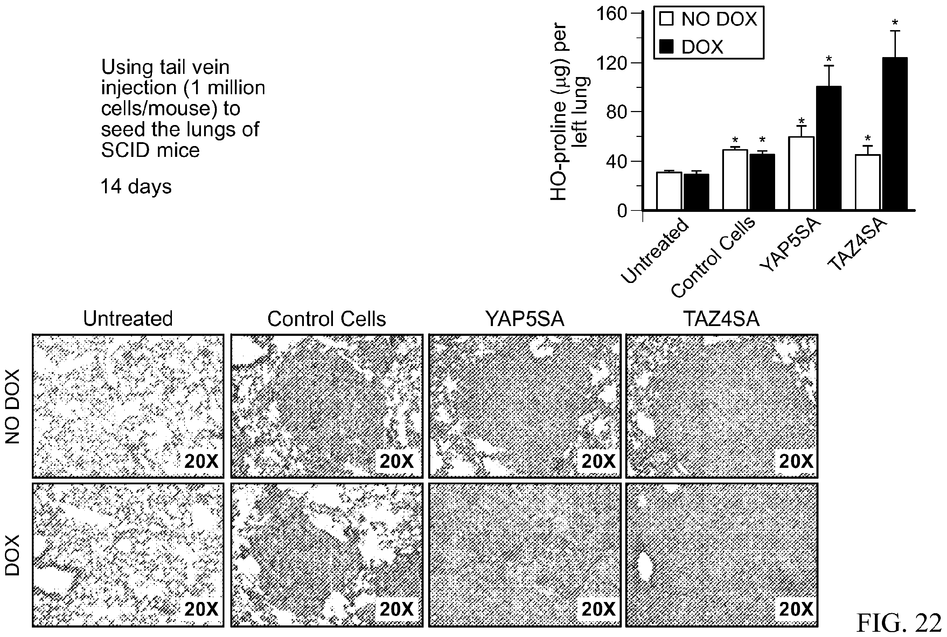

**FIG. 22** shows that YAP/TAZ confer fibrogenic potential in vivo.

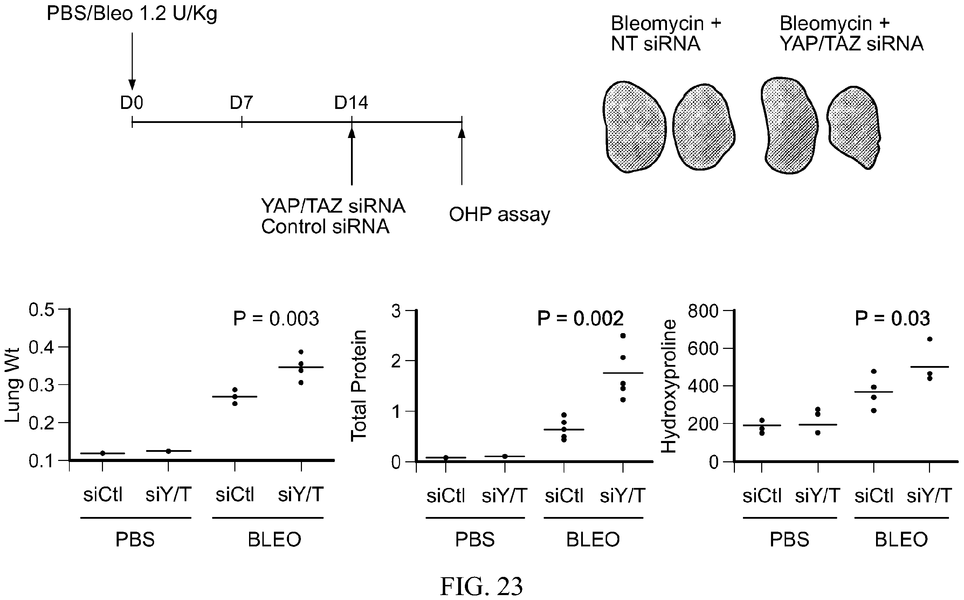

**FIG. 23** shows that global YAP/TAZ targeting is not viable.

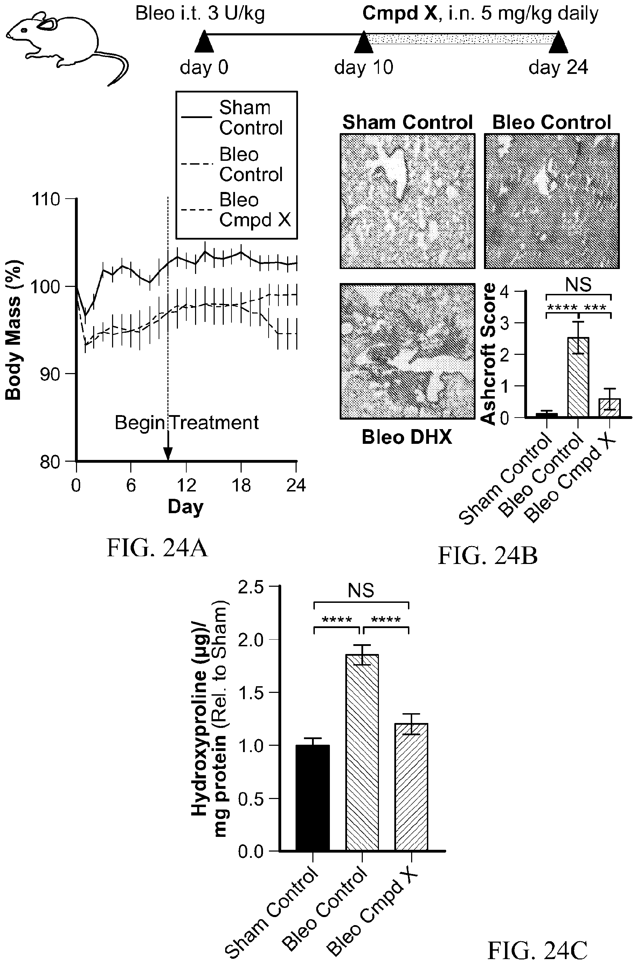

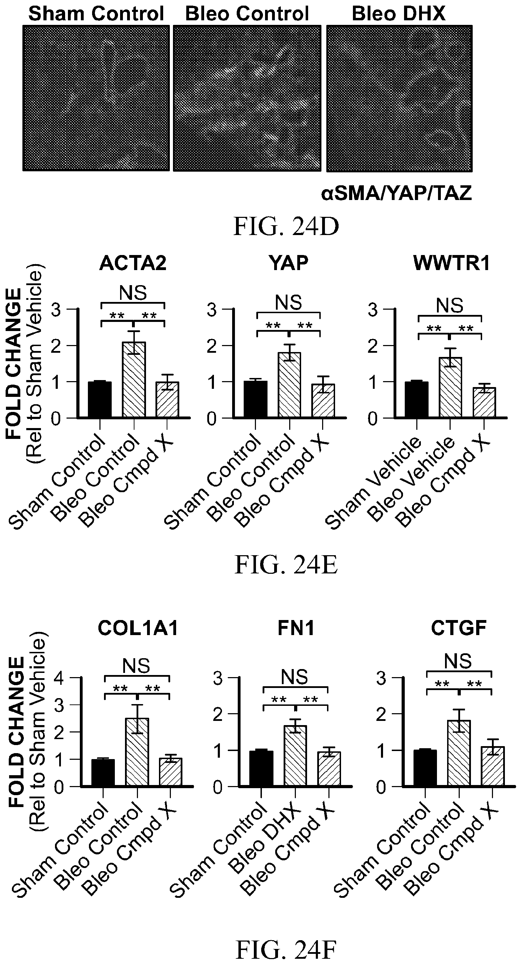

**FIG. 24** shows that Compound X (DHX) is antifibrotic in vivo.

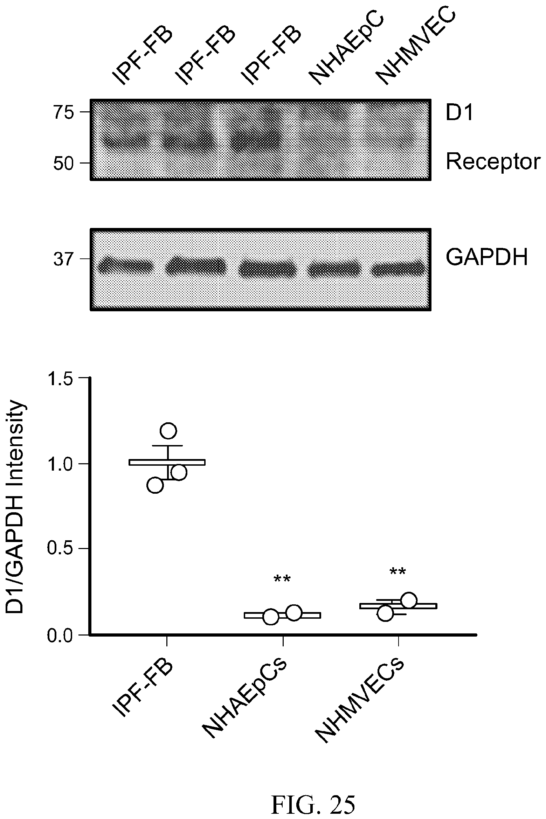

**FIG. 25** shows Western blot protein expression of the D1 dopamine receptor from IPF patient derived fibroblasts, normal human alveolar epithelial cells (NHAEpC), and normal human microvasculature endothelial cells. NHAEpC and NHMVEC, N=2. non-IPF FB and IPF FB, N=3 different donor lines.

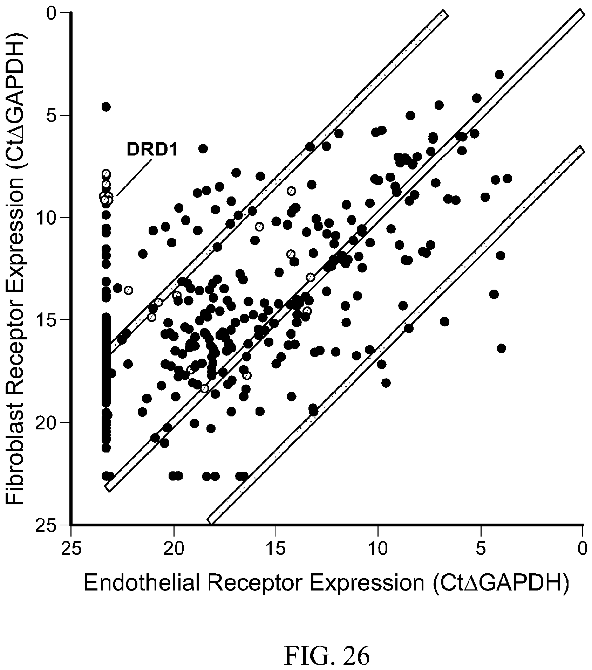

**FIG. 26** shows GPCR expression profiling of primary cultured human pulmonary microvascular endothelial cells and normal human pulmonary fibroblasts. Red points indicate GPCRs that selectively couple to Gαs. Blue lines indicate 100-fold preferential expression. Prostaglandin receptors PTGER2 and PTGDR (red points directly above DRD1) were also selectively expressed in fibroblasts vs. endothelial cells, however both of these receptors were highly expressed in epithelial cells.

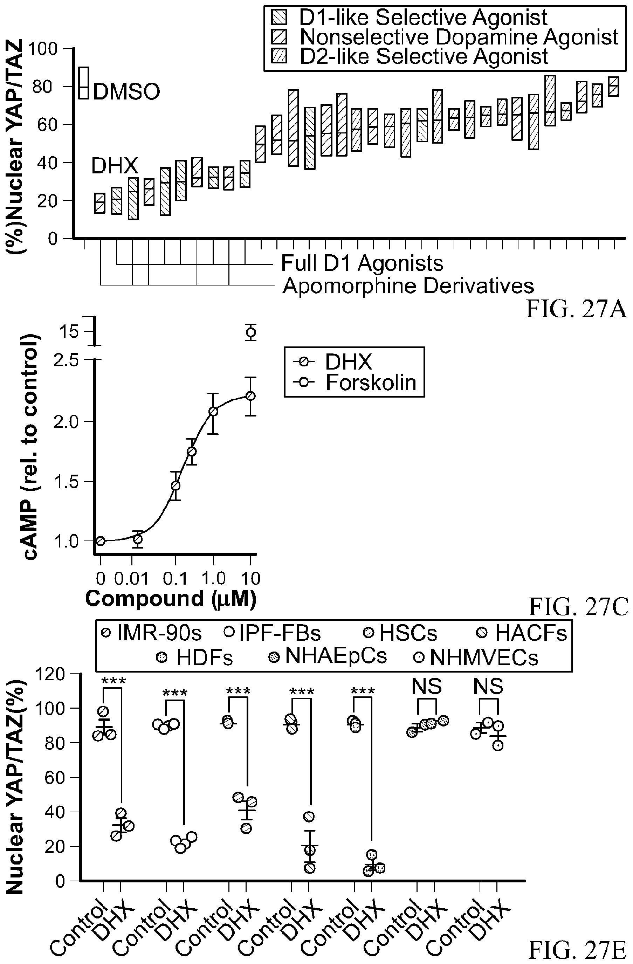

**FIG. 27** **Dopamine Receptor D1 agonism blocks YAP/TAZ nuclear localization. A.** D1 receptor selective agonists inhibit YAP/TAZ nuclear localization. IPF patient-derived lung fibroblasts cells treated 2 hours prior to fixation with diverse dopaminergic agonists (10 µM). N=4 different patient samples. %nuclear localization of YAP/TAZ was determined using automated imaging software. Scale bar represents 100µm. **C.** cAMP measured in IPF patient-derived fibroblasts treated for 20 minutes with DHX. N=3. **E.** DHX inhibits YAP/TAZ nuclear localization in fibroblasts from multiple organs: hepatic stellate cells (HSC), human adult cardiac fibroblasts (HACFs), and human dermal fibroblasts (HDFs) but not in lung alveolar epithelial (NHAEp) or endothelial (NHMVE) cells. N=3 (**** *p* < 0.0001 vs. 0.1% DMSO vehicle control).

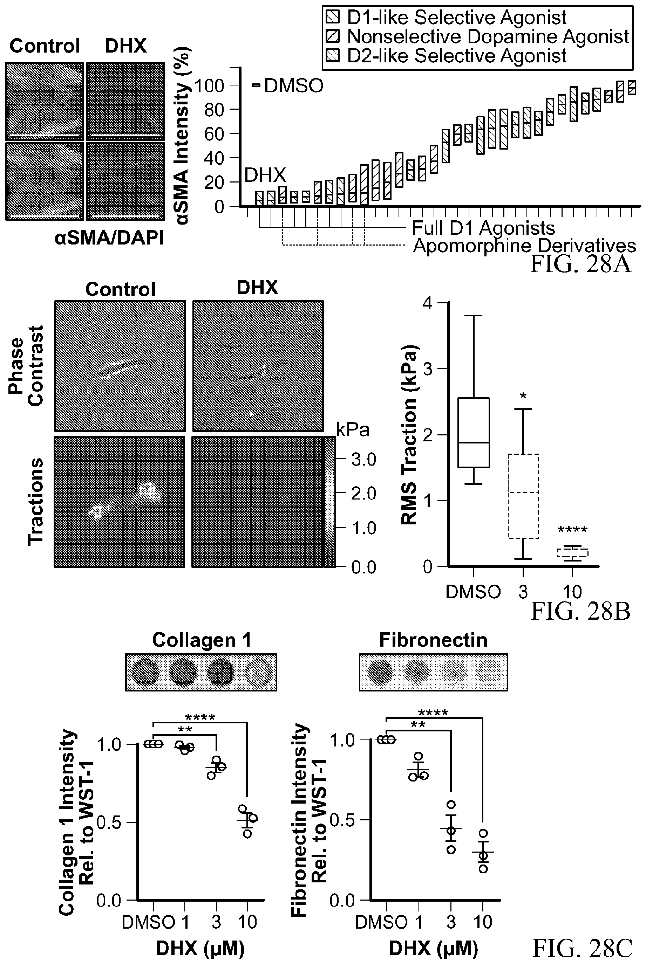

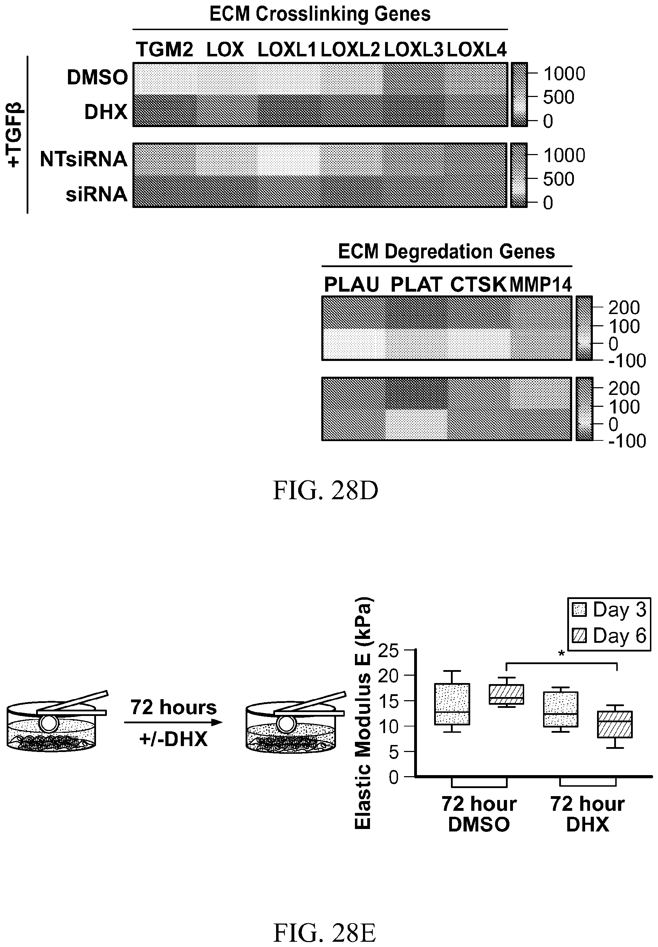

**FIG. 28** **DHX reverses fibroblast matrix deposition, contraction and stiffening. A.** D1 receptor selective agonists inhibit fibroblast activation (Representative image: 1µM dihydrexidine (DHX). IPF patient-derived lung fibroblasts cells treated for 72 hours prior to fixation with a library of diverse, mixed selectivity dopaminergic agonists (1µM) + TGFβ. N=4 different patient samples. αSMA intensity was determined using automated imaging software. Scale bar represents 100µm. **B.** DHX attenuates IPF fibroblast contractility measured by traction force microscopy. (**** *p* < 0.0001, * *p <* 0.05 vs. 0.1% DMSO vehicle control). **C.** DHX reverses extracellular matrix accumulation. IPF patient-derived fibroblasts pre-stimulated with 2ng/mL TGFβ for 48 hours, then treated with DHX +2ng/mL TGFβ for additional 24 hours. N=3 (**** *p* < 0.0001, *** *p* < 0.001, ** *p <* 0.01 vs. 0.1% DMSO vehicle control) **D.** DHX and YAP/TAZ siRNA modulate matrix crosslinking and degradation gene programs. IPF fibroblasts treated 24 hours with 2 ng/mL TGFβ +/- 10 µM DHX or YAP and TAZ siRNA (>90% knockdown). N=3. Heat map indicates % change relative to unstimulated controls. **E.** DHX reverses extracellular matrix stiffening. IPF patient derived fibroblasts and their cell-derived matrices were characterized by AFM microindentation using a spherical tip after 72 hours, then treated +/- 10 µM DHX in matrix deposition media for additional 72 hours and re-characterized. N=5 different patient samples. (* *p* < 0.05 vs. 0.1% DMSO vehicle control).

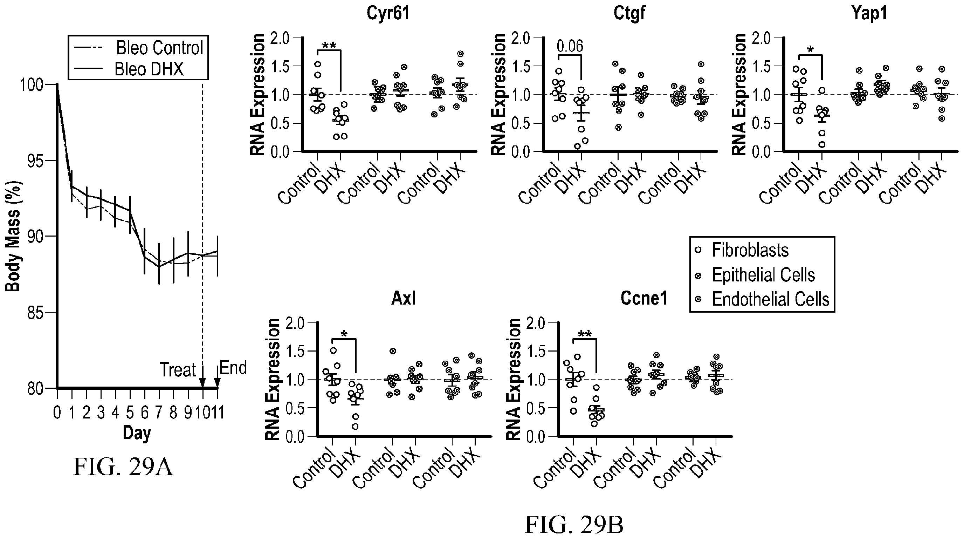

**FIG. 29** **DHX selectively blocks expression of YAP/TAZ target genes in lung fibroblasts in vivo. A.** Two groups of mice were injured intratracheally with bleomycin at day 0, on day 10 one group received two doses of DHX (2 and 24 hours prior to collecting lungs) and the other received vehicle control. On day 11 lungs were collected to flow sort fibroblasts, epithelial, and endothelial cells. **B.** Changes in RNA expression of YAP/TAZ target genes from freshly isolated cells.

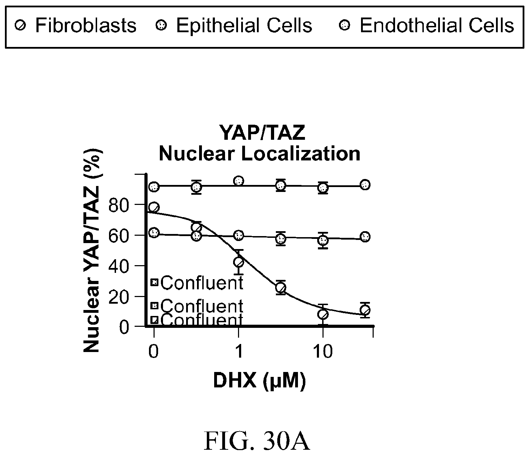

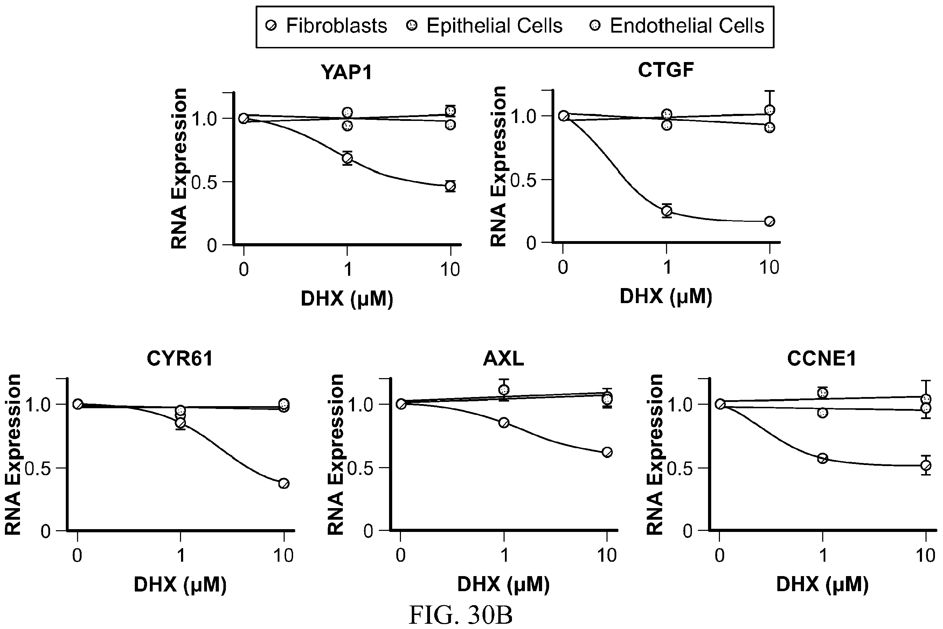

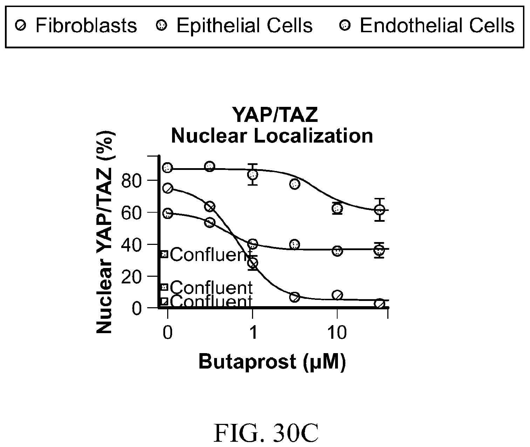

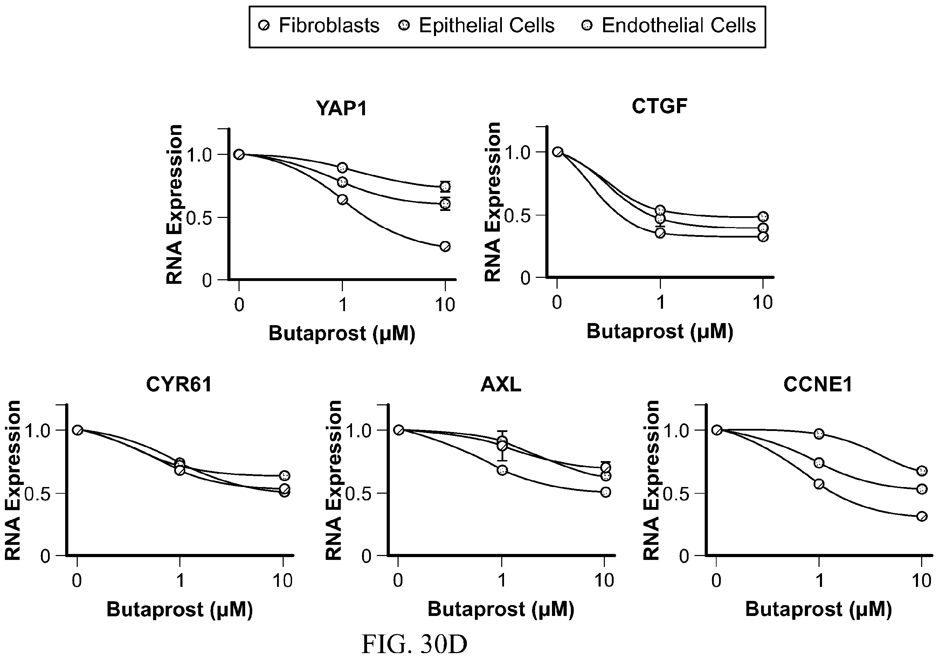

**FIG. 30** **DRD1 agonism selectively blocks localization and activity of YAP/TAZ in lung fibroblasts. A** and **C.** IPF derived lung fibroblasts, lung alveolar epithelial (NHAEp) and endothelial (NHMVE) cells were treated for 2 hours with DRD1 selective agonist (DHX, **A**) or with butaprost (EP2 receptor agonist, **C**)**.** DRD1 receptor is only expressed in fibroblasts while EP2 receptor, which also elevates cAMP, is expressed in all three cell types. **B** and **D.** IPF derived lung fibroblasts, lung alveolar epithelial (NHAEp) and endothelial (NHMVE) cells were treated for 24 hours with DRD1 selective agonist (DHX, **B**) or with butaprost (EP2 receptor agonist, **D**) prior to RNA isolation and measurement of YAP/TAZ target genes. N=2 technical replicates.

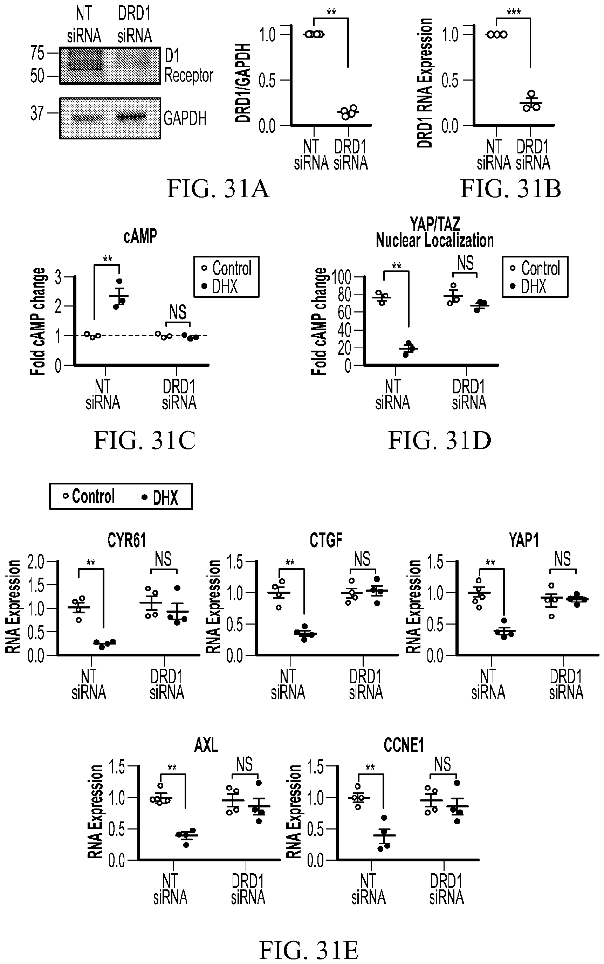

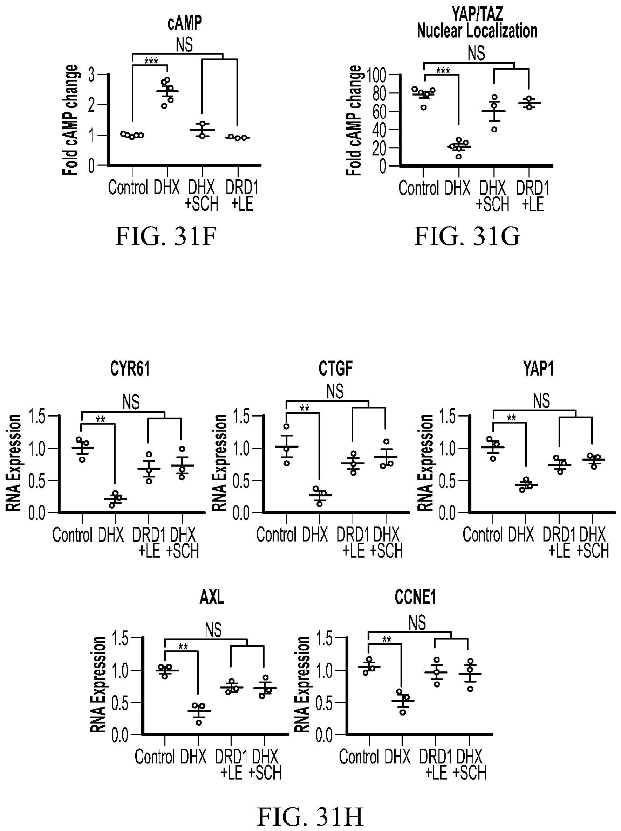

**FIG. 31** **DHX activity is dependent on DRD1 receptor. A-E.** DRD1 siRNA treatment blocks DHX's elevation of cAMP **(C),** inhibition of YAP/TAZ nuclear localization **(D),** and inhibition of YAP/TAZ target genes **(E). F-H.** Dopamine receptor D1 antagonists SCH-39166 and LE-300 block DHX's elevation of cAMP **(F),** inhibition of YAP/TAZ nuclear localization **(G),** and inhibition of YAP/TAZ target genes **(H). F-H.** N=3, IMR-90 lung fibroblasts.

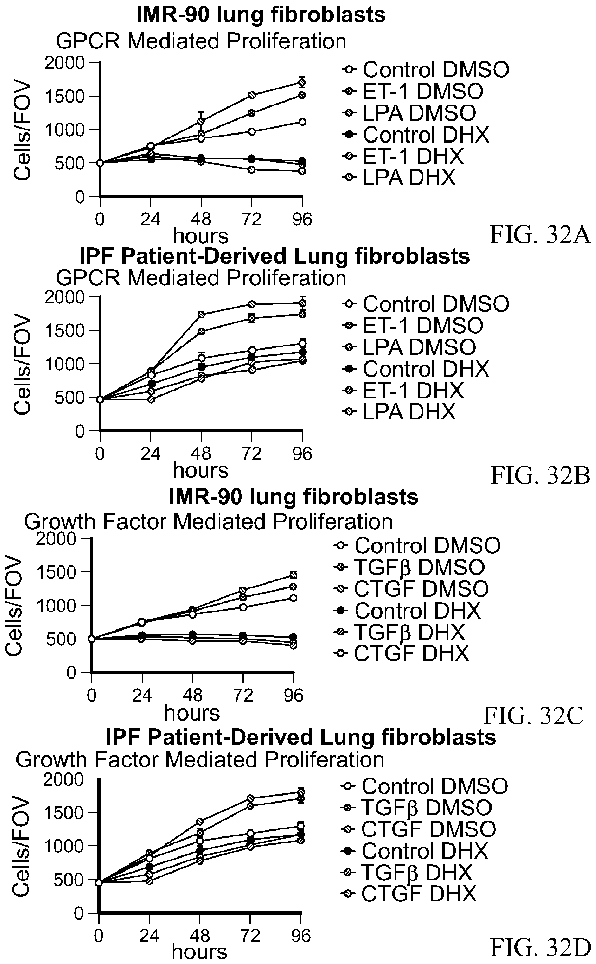

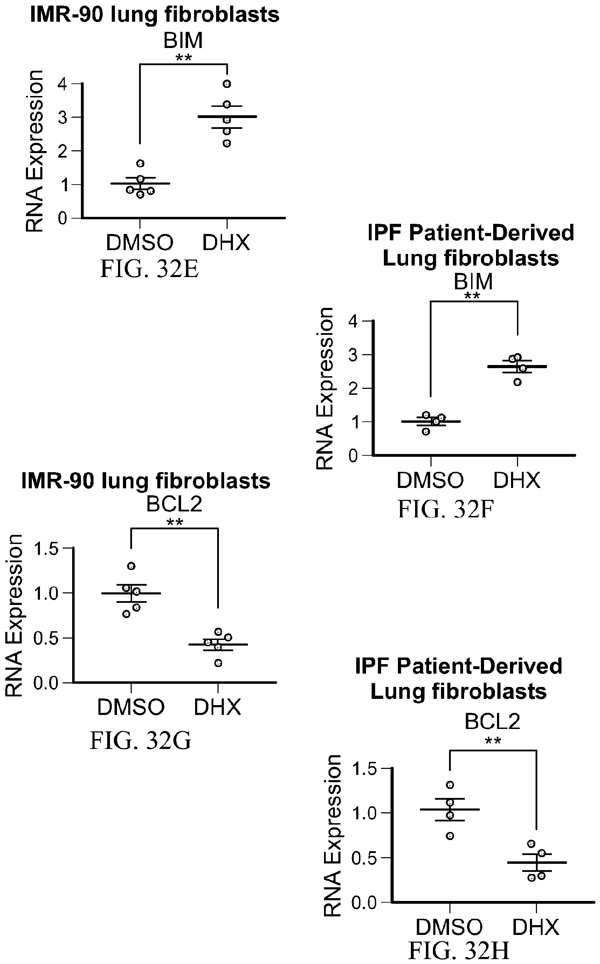

**FIG. 32** **DHX blocks proliferation and primes lung fibroblasts for apoptosis.** Proliferation of lung fibroblasts (IMR-90 cells and IPF Patient-Derived Lung fibroblasts) measured for 4 days in the presence of GPCR agonists ET-1 (100nM) and LPA (10µM) **(A,B)** or growth factors TGFβ (2ng/mL) and CTGF (100ng/mL) **(C,D)** +/- 10µM DHX. Cells were fixed and counted with DAPI at the end of each day. N= 3 technical triplicates. RNA Expression of pro-apoptotic factor *BIM* **(E,F)** and anti-apoptotic factor *BCL2* **(G,H)** was assessed following 24 treatment with DHX (10µM). N=4.

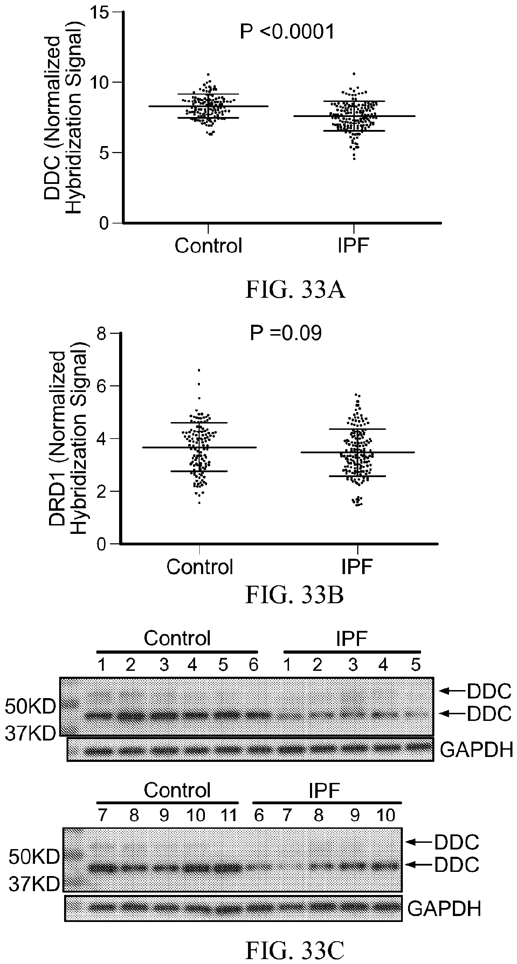

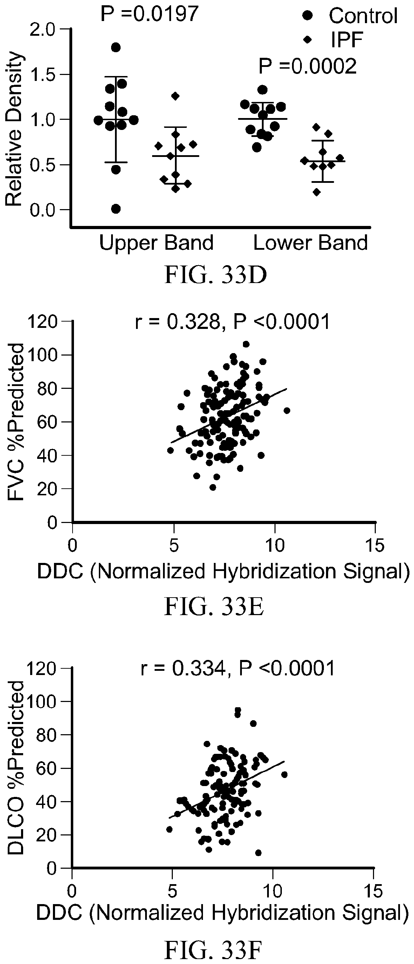

**FIG. 33** **DOPA decarboxylase is decreased in IPF, and correlates with worsening disease severity. A.** Expression levels for *DDC* and *DRD1* were queried from microarray analyses of IPF (n=134) and control (n=108) lungs. Each data point represents expression levels from an individual. Bars indicate mean and standard deviation. **B.** Western blotting was used to detect DDC protein expression in whole lung homogenates from IPF (n=10) and control (n=11) lungs. Bars indicate mean and standard deviation. **C.** Univariate analysis of the correlation of *DDC* expression with forced vital capacity (FVC) and diffusing capacity of the lung for carbon monoxide (DLCO) performed using the Pearson's correlation coefficient (r). Each data point represents expression levels and lung function (expressed a percent predicted based on age, sex and ideal body weight) from an individual. P values are as indicated for each figure panel.

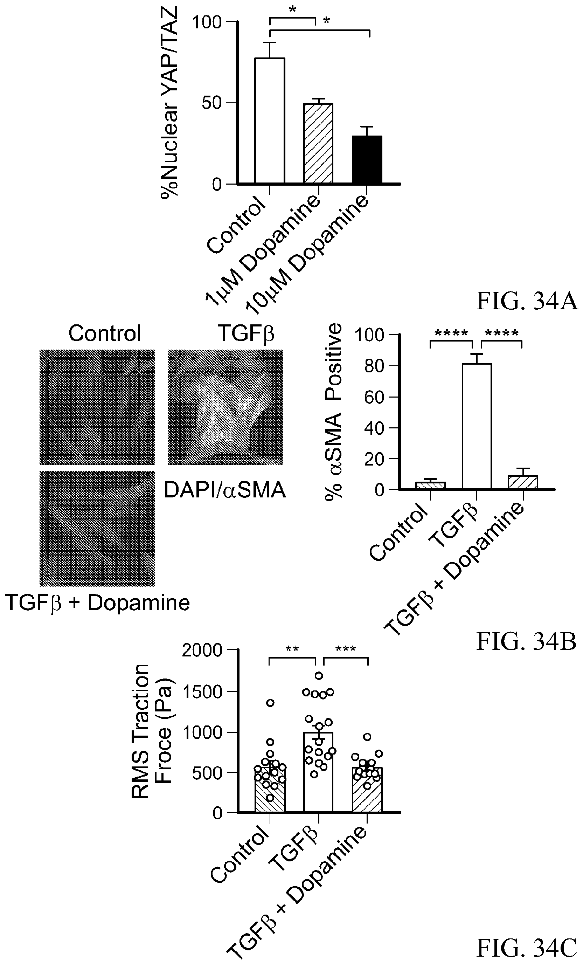

**FIG. 34** **Dopamine promotes anti-fibrotic effects. A.** Dopamine inhibits YAP/TAZ nuclear localization in low density IPF-patient derived fibroblasts plated onto tissue culture plastic. N=2. **B.** Dopamine reverses αSMA+ stress fiber formation. IPF-patient derived fibroblasts, pre-stimulated with 2 ng/mL TGFβ for 48 hours, then treated with dopamine (1 µM) + 2 ng/mL TGFβ for additional 24 hours. N=4. Scale bar represents 500 µm. **C.** Dopamine attenuates IPF fibroblast contractility measured by traction force microscopy. (**** *p <* 0.0001, *** *p <* 0.001, ** *p* < 0.01, * *p <* 0.05 vs. the indicated group).

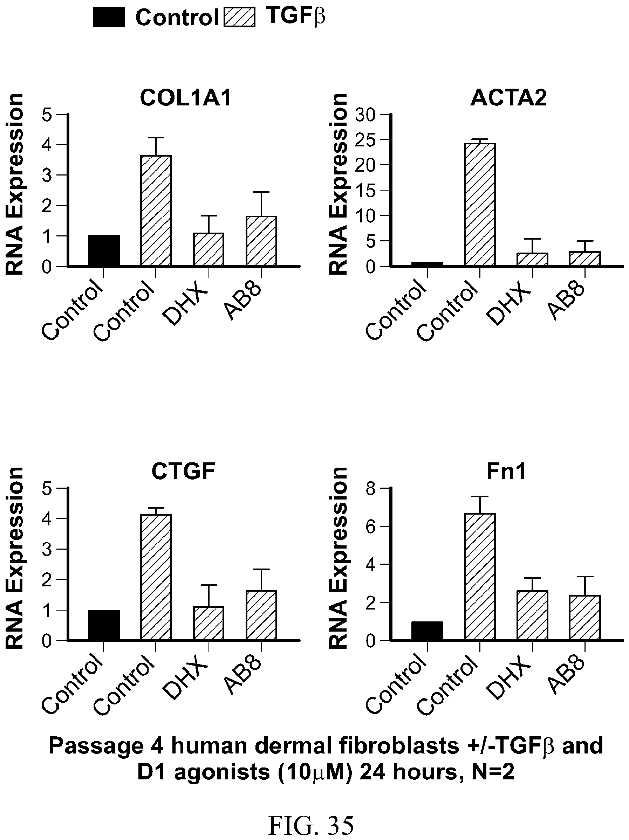

**FIG. 35** **DRD1 agonism blocks profibrotic gene expression in human dermal fibroblasts.** Human dermal fibroblasts treated for 24 hours +/- 2ng/mL TGFβ and D1 agonists: DHX and A68930 prior to RNA isolation. N=2.

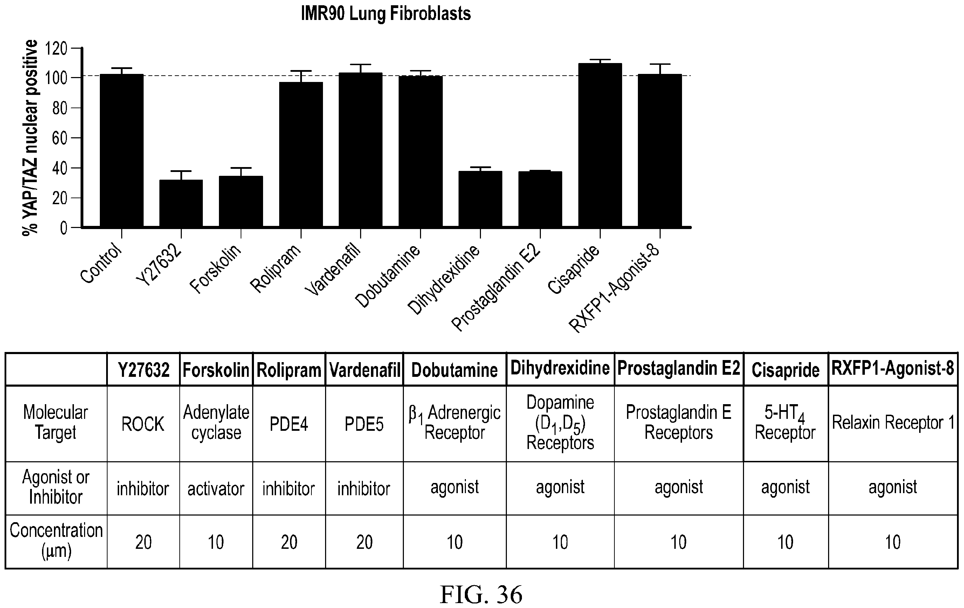

**FIG. 36** shows cAMP modulators and their effects on YAP/TAZ nuclear localization. IMR-90 cells were sparsely plated into 96-well cell culture plate and treated for 2 hours with the indicated compounds. Cells were then fixed and stained for YAP/TAZ. Nuclear localization was assessed using automated imaging software. Y27632 is used as a positive control to reduce YAP/TAZ nuclear localization. Remaining compounds were randomly selected based on their known involvement in elevating cAMP in other cell types. N=3.

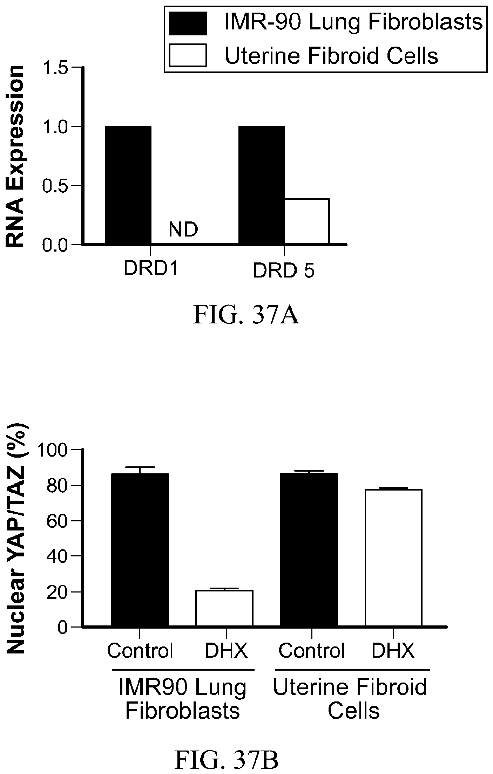

**FIG. 37** shows that dihydrexidine (DHX) efficacy depends on expression of D1 like dopamine receptors (DRD1, DRD5). IMR-90 lung fibroblasts express higher levels of DRD1 and DRD5 than mesenchymal cells derived from uterine fibroids (A) which results in marginal inhibition of YAP/TAZ nuclear localization by DHX in these cells (B). ND refers to the gene not being detected.

### DETAILED DESCRIPTION

Tissue fibrosis can occur in multiple vital organs including heart, lung, liver, and kidney. Fibrosis is a progressive process which, through multiple mechanisms, transforms a normal healthy organ into an architecturally and functionally compromised tissue. From a clinical standpoint they represent a serious problem as the therapeutic options remain minimal and the prognosis is generally very poor. Dopamine receptors, which are almost exclusively researched as part of the central nervous system, are actually highly expressed in the periphery as well in select tissues and cells in the body. These receptors signal through downstream pathways which play a major role in tissue fibrosis. The present work shows that a Gαₛ-coupled receptor, such as a dopamine receptor D1 (DRD1), as a viable therapeutic target for the treatment or prevention of tissue fibrosis in multiple organs.

YAP and TAZ are transcriptional co-activators and central effectors of the Hippo pathway⁸. Originally identified based on their roles in organ growth and size control during tissue morphogenesis, the Hippo pathway and YAP/TAZ in adult tissues regulate epithelial and endothelial homeostasis⁹⁻¹³, stem cell function¹⁴⁻¹⁶ and tissue regeneration^{9,17,18}. Roles for YAP and TAZ in mesenchymal cell activation and fibrosis in multiple organs¹⁹⁻²², including the lung and liver², was also shown. An array of mechanical and biochemical signals have been implicated as upstream regulators of YAP and TAZ, with multiple pro-fibrotic stimuli including matrix stiffness, TGFβ/SMAD, MRTF/SRF, and WNT^{5,23,24} signaling all potentially involved.

G protein coupled receptors are linked to effector proteins from four main classes of G-proteins (e.g., Gα_{12/13}, Gα_{q/11}, Gα_{i/o} or Gαₛ). In some instances, G protein coupled receptor stimulates YAP/TAZ nuclear translocation and transcriptional activity. In other instances, G protein coupled receptors inhibit YAP/TAZ nuclear localization and activity via elevation of cAMP (see, e.g., Fig. 1a).

Activation (agonism) of a G protein coupled receptor can result in YAP/TAZ hyper phosphorylation and inactivation under physiological conditions (e.g., agonism of the receptor prevents YAP/TAZ nuclear localization). This is in contrast to inactivation (antagonism) of G protein coupled receptor, which stimulates YAP/TAZ nuclear translocation and transcriptional activity, which results in expression of profibrotic genes, such as *Acta2* (αSMA), *Ctgf* (Connective tissue growth factor), *Fn1* (Fibronectin), *Col1a1* (Collagen I), and *Col1a2* (Collagen II).

In some embodiments, the present disclosure provides an *in vitro* method of agonizing a G protein coupled receptor in a cell, the method comprising contacting the cell with any one of compounds described above, or a pharmaceutically acceptable salt thereof. In some embodiments, the present disclosure provides an *in vitro* method of promoting YAP phosphorylation in a cell, the method comprising contacting the cell with any one of compounds described above, or a pharmaceutically acceptable salt thereof. In some embodiments, agonism of the G protein coupled receptor results in YAP/TAZ phosphorylation and subsequent degradation. In some embodiments, the present disclosure provides an *in vitro* method of inhibiting YAP/TAZ function in a cell (e.g., inhibiting expression of a profibrotic gene in a cell), the method comprising contacting the cell with any one of the compounds of the present disclosure, or a pharmaceutically acceptable salt thereof. In some embodiments, agonism of the G protein coupled receptor results in inhibition of YAP/TAZ function in a cell and subsequent inhibition of expression of profibrotic genes in the cell. In some embodiments, inhibiting YAP/TAZ function in a cell results in prevention of accumulation of extracellular matrix in a tissue. In some embodiments, agonism of G protein coupled receptor reverses fiber formation and extracellular matrix accumulation. In some embodiments, the fiber formation is induced by trauma or tissue injury. Normally cells generate just the right amount of tissue to replace old tissue or repair damage. Excessive connective tissue generation (e.g., in response to trauma or injury) results in pathological accumulation of fibrotic tissue (e.g., extracellular matrix proteins) leading to organ or tissue thickening and scarring.

In some embodiments, the present disclosure provides compounds for use in a method of treating or preventing a fibrotic pathology in a subject, the method comprising administering to the subject in need thereof a therapeutically effective amount of any one of the compounds described above, or a pharmaceutically acceptable salt thereof. In some embodiments, the subject in need of treatment of fibrotic pathology is diagnosed with fibrotic pathology by a treating physician.

In some embodiments, the fibrotic pathology is interstitial lung disease (ILD). In some embodiments, fibrotic pathology is lung tissue fibrosis, e.g., pulmonary fibrosis (PF) or idiopathic pulmonary fibrosis (IPF). Despite the name, cystic fibrosis is not considered an interstitial lung disease or predominantly a fibrotic pathology. Cystic fibrosis results from impaired ion transport, mucus dysfunction, and failure to effectively clear pathogens from the airways, which eventually results in scarring of the airways and lungs.

In some embodiments, fibrotic pathology is a liver tissue fibrosis, e.g., cirrhosis or biliary atresia. In some embodiments, fibrotic pathology is a heart tissue fibroses (cardiac fibrosis), e.g., atrial fibrosis, endomyocardial fibrosis, or post-myocardial infarction scarring. In some embodiments, fibrotic pathology is a brain tissue fibrosis, e.g., glial scar. In some embodiments, fibrotic pathology is arterial stiffness, arthrofibrosis (knee, shoulder, elbow, or other joints), chronic kidney disease and fibrosis, liver fibrosis, nonalcoholic fatty liver, nonalcoholic steatohepatitis, Crohn's disease (intestinal scarring), Dupuytren's contracture (scar tissue in hands or fingers), skin tissue fibrosis, e.g., keloid (a scar on the skin), mediastinal fibrosis (soft tissue of the mediastinum), Peyronie's disease (scar in a penial tissue), nephrogenic systemic fibrosis, progressive massive fibrosis, retroperitoneal fibrosis (scar on the soft tissue of the retroperitoneum) or adhesive capsulitis.

In some embodiments, the subject in need of prevention of fibrotic pathology is diagnosed with tissue trauma or injury by a treating physician. Suitable examples of tissue injury include injury caused by inhaled substances (e.g., silica or asbestos), drug-induced injury (injury caused by an antibiotic or an anticancer drug), tissue injury caused by autoimmune disease (e.g., rheumatoid arthritis, sclerosis, such as systemic sclerosis, lupus), injury caused by infection (e.g., tuberculosis, pneumonia, respiratory virus), or sarcoidosis.

### Exemplary therapeutic compounds

In some embodiments, a compound that can be used in any one of the methods described here is an agonist of a G protein coupled receptor. In such embodiments, the compound is a selective agonist of a Gαₛ receptor (e.g., the compound is 100-fold, 50-fold, or 10-fold selective to Gαₛ protein coupled receptor as compared to Gα_{12/13}, Gα_{q/11} or Gα_{i/o} protein coupled receptor, or any combination of the aforementioned).

In some embodiments, the G protein coupled receptor is expressed in a mesenchymal cell. In some embodiments, the mesenchymal cell is a fibroblast (e.g., pulmonary, cardiac, hepatic, renal or dermal fibroblast) or a stellate cell (e.g., pancreatic stellate cell, hepatic stellate cell, podocyte, or osteocyte). In some embodiments, the G protein coupled receptor is preferentially expressed in mesenchymal cells as compared to epithelial or endothelial cells of a tissue. In one example, the G protein coupled receptor is preferentially expressed in pulmonary fibroblasts over alveolar epithelial cells. In another example, the G protein coupled receptor is preferentially expressed in hepatic stellate cells over hepatocytes. In some embodiments, the agonist is specific for Gα_{S} receptor that is expressed preferentially in the mesenchymal cell. For example, dopamine D1 receptor and dopamine D5 receptor are both Gαₛ protein coupled receptors. Hence, an agonist of a Gαₛ protein coupled receptor may agonize both D1 and D5 receptors. In this example, a dopamine D1 receptor and dopamine D5 may both be expressed in mesenchymal cells and also in epithelial or endothelial cells of a tissue, but dopamine D1 receptor is preferentially expressed in mesenchymal cells while D5 is equally distributed between both cell types. In this example, the Gαₛ protein coupled receptor agonist is specific to D1 receptor over D5 receptor.

In some embodiments, the receptor agonist is hydrophilic. In such embodiments, the structure of the receptor agonist compound contains hydrogen bond donor (HBD) atoms that are capable of forming hydrogen bonds with molecules of water and with the amino acids within the active site of the G protein coupled receptor. In some embodiments, the molecule of the receptor agonist contains at least 2, 3, 4, 5, or 6 HBD atoms (e.g., heteroatoms such as O, N or S). In some embodiments, the molecule of the receptor agonist contains at least one hydroxyl group (e.g., 1, 2, 3, 4, 5, or 6 hydroxyl groups). In some embodiments, the molecule of the receptor agonist contains amino groups (e.g., 1, 2, 3, 4, 5, or 6 amino groups).

In some embodiments, the receptor agonist does not penetrate the blood brain barrier or only an insignificant amount of the receptor agonist penetrates the blood brain barrier after the receptor agonist is administered to a subject (e.g., not more than about 0.1 wt. %, about 1 wt. %, about 5 wt. %, about 10 wt. %, or about 20 wt.% of the amount of the compound administered to the subject penetrates the blood brain barrier).

In some embodiments, the receptor agonist is a small molecule, e.g., about 2000 daltons or less (e.g., from about 300 to about 1200, from about 300 to about 1000, from about 300 to about 800, and/or from about 300 to about 600 daltons). In some embodiments, the receptor agonist is a biomolecule. Typically, biomolecules are organic molecules having a molecular weight of 200 daltons or more produced by living organisms or cells, including large polymeric molecules such as polypeptides, proteins, glycoproteins, polysaccharides, polynucleotides and nucleic acids.

A Gαₛ protein coupled receptor can be a dopamine receptor (e.g., D1, D2, D3, D4, or D5 dopamine receptor). That is, the G protein coupled receptor agonist is a dopamine receptor agonist (e.g. agonist of D1, D2, D3, D4, or D5 dopamine receptor, or any combination thereof). In some embodiments the dopamine receptor is a dopamine receptor D1 (DRD1). In some embodiments, the agonist is selective with respect to the dopamine receptor. In some embodiments, the compound is a selective agonist of a dopamine receptor D1 (e.g., the compound is 100-fold, 50-fold, or 10-fold selective to D1 dopamine receptor as compared to D2, D3, D4, or D5 receptor, or any combination of the aforementioned). In some embodiments, the D1 receptor agonist is ineffective or only weakly effective in treating central nervous system (CNS) disorders.

In some embodiments, the dopamine receptor D1 agonist is selected from dihydrexidine (DHX), SKF-81297, SKF-82958, SKF-38393, fenoldopam, 6-Br-APB, A-68930, A-77636, CY-208,243, and pergolide, or a pharmaceutically acceptable salt thereof.

In some embodiments, the GαS protein coupled receptor agonist is selected from dihydrexidine (DHX), SKF-81297, SKF-82958, SKF-38393, fenoldopam, 6-Br-APB, A-68930, A-77636, CY-208-243, SKF-89145, pergolide, R(-)-2,10,11-trihydroxyaporphine, (R)-(-)-apomorphine, R(-)-propylnorapomorphine, R(+)-6-bromo-APB, R(-)-2,10,11-trihydroxy-N-propyl-noraporphine, 6,7-ADTN, mesulergine, N-methyldopamine, 4-hydroxyphenethylamine, cabergoline, 3-hydroxyphenethylamine, pramipexole, PD-168077, fenoldopam, (±)-PD 128-907, (±)-2-(N-phenylethyl-N-propyl)amino-5-hydroxytetralin, bromocriptine, ropinirole, LY-163-502, dipropyldopamine, B-HT 920, piribedil, (+)-UH 232, pergolide, (-)-quinpirole, and R(-)-2,11-dihydroxy-10-methoxyapomorphine, or a pharmaceutically acceptable salt thereof.

In some embodiments, the Gα_{S} protein coupled receptor agonist is selected from dihydrexidine (DHX), SKF-82958, A-68930, and CY-208-243, or a pharmaceutically acceptable salt thereof.

In some embodimentsm, the receptor agonist is dihydrexidine: 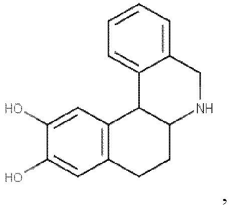 or a pharmaceutically acceptable salt thereof.

In some embodiments, the receptor agonist is A-68930: 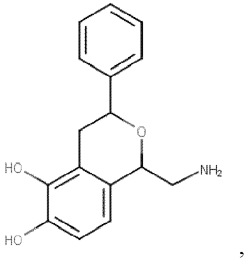 or a pharmaceutically acceptable salt thereof.

In some embodiments, the receptor agonist is SKF-82958: 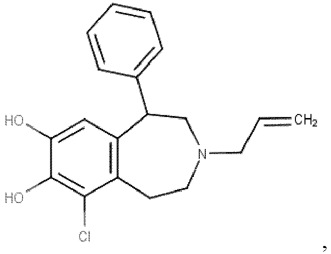 or a pharmaceutically acceptable salt thereof.

In some embodiments, the receptor agonst is CY 208-243: 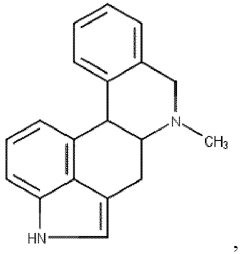 or a pharmaceutically acceptable salt thereof.

In some embodiments, the receptor agonist is SKF-38393: 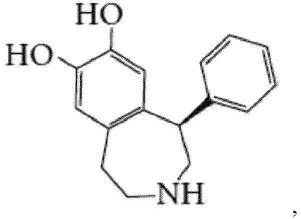 or a pharmaceutically acceptable salt thereof.

In some embodiments, the receptor agonist is A-77636: 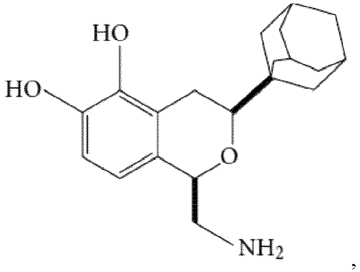 or a pharmaceutically acceptable salt thereof.

Dopamine receptor agonist is any one of the compounds described in Martin et al., International Journal of Medicinal Chemistry, 2011, Article ID 424535.

In some embodiments, the receptor agonist is a compound of Formula (I): 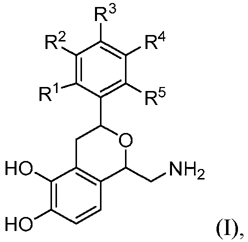 or a pharmaceutically acceptable salt thereof, wherein:
each of R¹, R², R³, R⁴ and R⁵ is independently selected from H, OH, C₁₋₆ alkoxy, C₁₋₆ haloalkoxy, HO-C₁₋₃ alkyl, NH₂-C₁₋₃ alkyl, HS-C₁₋₃ alkyl, SH, NH₂, C₁₋₆ alkylamino and di(C₁₋₆ alkyl)amino.

In some embodiments, the compound of Formula (I) is not any one of the following compounds:

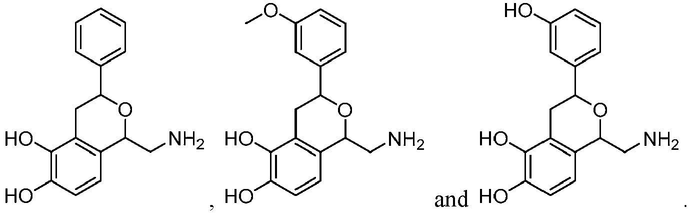

In some embodiments, the compound of Formula (I) is not any one of compounds described in J. Med. Chem., 1991, 34 (8), 2561-2569.

In some embodiments, R¹ is H. In some embodiments, R¹ is OH. In some embodiments, R¹ is methoxy, ethoxy, propoxy, isopropoxy, or trifluoromethoxy. In some embodiments, R¹ is NH₂. In some embodiments, R¹ is SH. In some embodiments, R¹ is methylamino, dimethylamino, or methylehtylamino.

In some embodiments, R² is H. In some embodiments, R² is OH. In some embodiments, R² is methoxy, ethoxy, propoxy, isopropoxy, or trifluoromethoxy. In some embodiments, R² is NH₂. In some embodiments, R² is SH. In some embodiments, R² is methylamino, dimethylamino, or methylehtylamino.

In some embodiments, R³ is H. In some embodiments, R³ is OH. In some embodiments, R³ is methoxy, ethoxy, propoxy, isopropoxy, or trifluoromethoxy. In some embodiments, R³ is NH₂. In some embodiments, R³ is SH. In some embodiments, R³ is methylamino, dimethylamino, or methylehtylamino.

In some embodiments, R⁴ is H. In some embodiments, R⁴ is OH. In some embodiments, R⁴ is methoxy, ethoxy, propoxy, isopropoxy, or trifluoromethoxy. In some embodiments, R⁴ is NH₂. In some embodiments, R⁴ is SH. In some embodiments, R⁴ is methylamino, dimethylamino, or methylehtylamino.

In some embodiments, R⁵ is H. In some embodiments, R⁵ is OH. In some embodiments, R⁵ is methoxy, ethoxy, propoxy, isopropoxy, or trifluoromethoxy. In some embodiments, R⁵ is NH₂. In some embodiments, R⁵ is SH. In some embodiments, R⁵ is methylamino, dimethylamino, or methylehtylamino.

In some embodiments, at least one of R¹, R², R³, R⁴ and R⁵ is OH. In some embodiments, at least two of R¹, R², R³, R⁴ and R⁵ are OH. In some embodiments, three of R¹, R², R³, R⁴ and R⁵ are OH, and the remaining groups are H. In some embodiments, at least one of R¹, R², R³, R⁴ and R⁵ is NH₂. In some embodiments, at least two of R¹, R², R³, R⁴ and R⁵ are NH₂.

In some embodiments, the compound of Formula (I) is selected from any one of the following compounds: 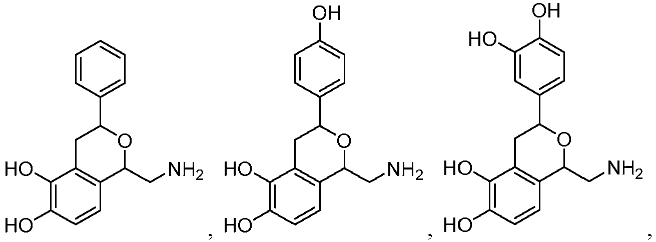 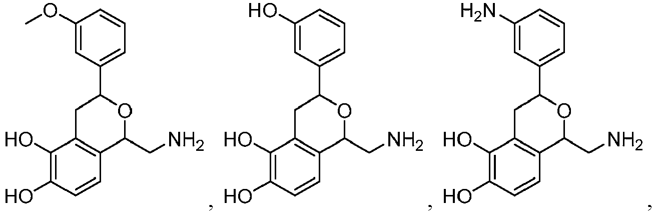 or a pharmaceutically acceptable salt thereof.

In some embodiments, the compound of Formula (I) is any one of compounds described in J. Med. Chem., 1991, 34 (8), 2561-2569.

In some embodiments, the compound of Formula (I) is any one of the following compounds: 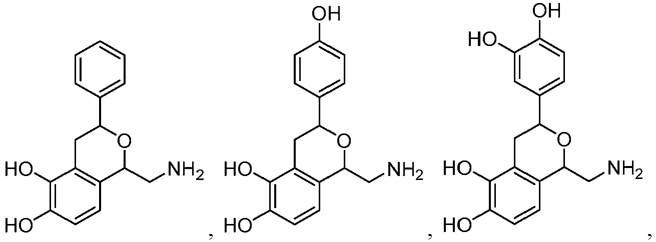 and 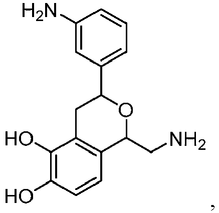 or a pharmaceutically acceptable salt thereof.

In some embodiments, the compound of Formula (I) is any one of the following compounds: 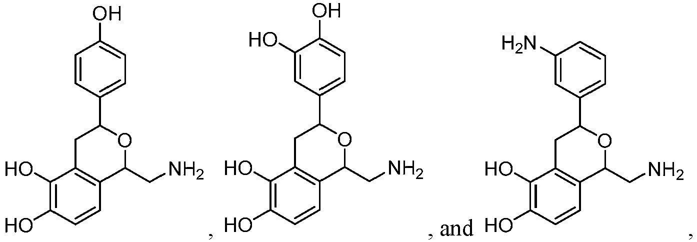 or a pharmaceutically acceptable salt thereof.

### Pharmaceutical compositions and formulations

The present application also provides pharmaceutical compositions comprising an effective amount of a compound of the present disclosure, or a pharmaceutically acceptable salt thereof, and a pharmaceutically acceptable carrier. The pharmaceutical composition may also comprise at least one of any one of the additional therapeutic agents described. In certain embodiments, the application also provides pharmaceutical compositions and dosage forms comprising any one the additional therapeutic agents described herein (e.g., in a kit). The carrier(s) are "acceptable" in the sense of being compatible with the other ingredients of the formulation and, in the case of a pharmaceutically acceptable carrier, not deleterious to the recipient thereof in an amount used in the medicament.

Pharmaceutically acceptable carriers, adjuvants and vehicles that may be used in the pharmaceutical compositions of the present application include ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol, and wool fat.

The compositions or dosage forms may contain any one of the compounds and therapeutic agents described herein in the range of 0.005% to 100% with the balance made up from the suitable pharmaceutically acceptable excipients. The contemplated compositions may contain 0.001%-100% of any one of the compounds and therapeutic agents provided herein, in one embodiment 0.1-95%, in another embodiment 75-85%, in a further embodiment 20-80%, wherein the balance may be made up of any pharmaceutically acceptable excipient described herein, or any combination of these excipients.

### Routes of administration and dosage forms

The pharmaceutical compositions of the present application include those suitable for any acceptable route of administration. Acceptable routes of administration include, buccal, cutaneous, endocervical, endosinusial, endotracheal, enteral, epidural, interstitial, intra-abdominal, intra-arterial, intrabronchial, intrabursal, intracerebral, intracisternal, intracoronary, intradermal, intraductal, intraduodenal, intradural, intraepidermal, intraesophageal, intragastric, intragingival, intraileal, intralymphatic, intramedullary, intrameningeal, intramuscular, intranasal, intraovarian, intraperitoneal, intraprostatic, intrapulmonary, intrasinal, intraspinal, intrasynovial, intratesticular, intrathecal, intratubular, intratumoral, intrauterine, intravascular, intravenous, nasal, nasogastric, oral, parenteral, percutaneous, peridural, rectal, respiratory (inhalation), subcutaneous, sublingual, submucosal, topical, transdermal, transmucosal, transtracheal, ureteral, urethral and vaginal.

Compositions and formulations described herein may conveniently be presented in a unit dosage form, e.g., tablets, capsules (e.g., hard or soft gelatin capsules), sustained release capsules, and in liposomes, and may be prepared by any methods well known in the art of pharmacy. See, for example, Remington: The Science and Practice of Pharmacy, Lippincott Williams & Wilkins, Baltimore, MD (20th ed. 2000). Such preparative methods include the step of bringing into association with the molecule to be administered ingredients such as the carrier that constitutes one or more accessory ingredients. In general, the compositions are prepared by uniformly and intimately bringing into association the active ingredients with liquid carriers, liposomes or finely divided solid carriers, or both, and then, if necessary, shaping the product.

In some embodiments, any one of the compounds and therapeutic agents disclosed herein are administered orally. Compositions of the present application suitable for oral administration may be presented as discrete units such as capsules, sachets, granules or tablets each containing a predetermined amount (e.g., effective amount) of the active ingredient; a powder or granules; a solution or a suspension in an aqueous liquid or a non-aqueous liquid; an oil-in-water liquid emulsion; a water-in-oil liquid emulsion; packed in liposomes; or as a bolus, etc. Soft gelatin capsules can be useful for containing such suspensions, which may beneficially increase the rate of compound absorption. In the case of tablets for oral use, carriers that are commonly used include lactose, sucrose, glucose, mannitol, and silicic acid and starches. Other acceptable excipients may include: a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. For oral administration in a capsule form, useful diluents include lactose and dried corn starch. When aqueous suspensions are administered orally, the active ingredient is combined with emulsifying and suspending agents. If desired, certain sweetening and/or flavoring and/or coloring agents may be added. Compositions suitable for oral administration include lozenges comprising the ingredients in a flavored basis, usually sucrose and acacia or tragacanth; and pastilles comprising the active ingredient in an inert basis such as gelatin and glycerin, or sucrose and acacia.

Compositions suitable for parenteral administration include aqueous and non-aqueous sterile injection solutions or infusion solutions which may contain antioxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example, sealed ampules and vials, and may be stored in a freeze dried (lyophilized) condition requiring only the addition of the sterile liquid carrier, for example water for injections, saline (e.g., 0.9% saline solution) or 5% dextrose solution, immediately prior to use. Extemporaneous injection solutions and suspensions may be prepared from sterile powders, granules and tablets. The injection solutions may be in the form, for example, of a sterile injectable aqueous or oleaginous suspension. This suspension may be formulated according to techniques known in the art using suitable dispersing or wetting agents and suspending agents. The sterile injectable preparation may also be a sterile injectable solution or suspension in a non-toxic parenterally-acceptable diluent or solvent, for example, as a solution in 1,3-butanediol. Among the acceptable vehicles and solvents that may be employed are mannitol, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant.

The pharmaceutical compositions of the present application may be administered in the form of suppositories for rectal administration. These compositions can be prepared by mixing a compound of the present application with a suitable non-irritating excipient which is solid at room temperature but liquid at the rectal temperature and therefore will melt in the rectum to release the active components. Such materials include cocoa butter, beeswax, and polyethylene glycols.

The pharmaceutical compositions of the present application may be administered by nasal aerosol or inhalation. Such compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art. See, for example, U.S. Patent No. 6,803,031. Additional formulations and methods for intranasal administration are found in Ilium, L., J Pharm Pharmacol, 56:3-17, 2004 and Ilium, L., Eur J Pharm Sci 11: 1-18, 2000.

The topical compositions of the present disclosure can be prepared and used in the form of an aerosol spray, cream, emulsion, solid, liquid, dispersion, foam, oil, gel, hydrogel, lotion, mousse, ointment, powder, patch, pomade, solution, pump spray, stick, towelette, soap, or other forms commonly employed in the art of topical administration and/or cosmetic and skin care formulation. The topical compositions can be in an emulsion form. Topical administration of the pharmaceutical compositions of the present application is especially useful when the desired treatment involves areas or organs readily accessible by topical application. In some embodiments, the topical composition comprises a combination of any one of the compounds and therapeutic agents disclosed herein, and one or more additional ingredients, carriers, excipients, or diluents including absorbents, anti-irritants, anti-acne agents, preservatives, antioxidants, coloring agents/pigments, emollients (moisturizers), emulsifiers, film-forming/holding agents, fragrances, leave-on exfoliants, prescription drugs, preservatives, scrub agents, silicones, skin-identical/repairing agents, slip agents, sunscreen actives, surfactants/detergent cleansing agents, penetration enhancers, and thickeners.

The compounds and therapeutic agents of the present application may be incorporated into compositions for coating an implantable medical device, such as prostheses, artificial valves, vascular grafts, stents, or catheters. Suitable coatings and the general preparation of coated implantable devices are known in the art and are exemplified in U.S. Patent Nos. 6,099,562; 5,886,026; and 5,304,121. The coatings are typically biocompatible polymeric materials such as a hydrogel polymer, polymethyldisiloxane, polycaprolactone, polyethylene glycol, polylactic acid, ethylene vinyl acetate, and mixtures thereof. The coatings may optionally be further covered by a suitable topcoat of fluorosilicone, polysaccharides, polyethylene glycol, phospholipids or combinations thereof to impart controlled release characteristics in the composition. Coatings for invasive devices are to be included within the definition of pharmaceutically acceptable carrier, adjuvant or vehicle, as those terms are used herein.

According to another embodiment, the present application provides an implantable drug release device impregnated with or containing a compound or a therapeutic agent, or a composition comprising a compound of the present application or a therapeutic agent, such that said compound or therapeutic agent is released from said device and is therapeutically active.

### Dosages and regimens

In the pharmaceutical compositions of the present application, a therapeutic compound is present in an effective amount (e.g., a therapeutically effective amount).

Effective doses may vary, depending on the diseases treated, the severity of the disease, the route of administration, the sex, age and general health condition of the subject, excipient usage, the possibility of co-usage with other therapeutic treatments such as use of other agents and the judgment of the treating physician.

In some embodiments, an effective amount of a therapeutic compound can range, for example, from about 0.001 mg/kg to about 500 mg/kg (e.g., from about 0.001 mg/kg to about 200 mg/kg; from about 0.01 mg/kg to about 200 mg/kg; from about 0.01 mg/kg to about 150 mg/kg; from about 0.01 mg/kg to about 100 mg/kg; from about 0.01 mg/kg to about 50 mg/kg; from about 0.01 mg/kg to about 10 mg/kg; from about 0.01 mg/kg to about 5 mg/kg; from about 0.01 mg/kg to about 1 mg/kg; from about 0.01 mg/kg to about 0.5 mg/kg; from about 0.01 mg/kg to about 0.1 mg/kg; from about 0. 1 mg/kg to about 200 mg/kg; from about 0. 1 mg/kg to about 150 mg/kg; from about 0. 1 mg/kg to about 100 mg/kg; from about 0.1 mg/kg to about 50 mg/kg; from about 0. 1 mg/kg to about 10 mg/kg; from about 0.1 mg/kg to about 5 mg/kg; from about 0.1 mg/kg to about 2 mg/kg; from about 0.1 mg/kg to about 1 mg/kg; or from about 0.1 mg/kg to about 0.5 mg/kg).

In some embodiments, an effective amount of a therapeutic compound is about 0.1 mg/kg, about 0.5 mg/kg, about 1 mg/kg, about 2 mg/kg, or about 5 mg/kg.

The foregoing dosages can be administered on a daily basis (e.g., as a single dose or as two or more divided doses, e.g., once daily, twice daily, thrice daily) or non-daily basis (e.g., every other day, every two days, every three days, once weekly, twice weekly, once every two weeks, once a month). The compounds and compositions described herein can be administered to the subject in any order. A first therapeutic agent, such as a compound of the present disclosure, can be administered prior to or subsequent to (e.g., 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before or after), or concomitantly with the administration of a second therapeutic agent, such as an anticancer therapy described herein, to a subject in need of treatment. Thus, the compound of the present disclosure, or a composition containing the compound, can be administered separately, sequentially or simultaneously with the second therapeutic agent, such as a chemotherapeutic agent described herein. When the compound of the present disclosure, or a pharmaceutically acceptable salt thereof, and a second or third therapeutic agent are administered to the subject simultaneously, the therapeutic agents may be administered in a single dosage form (e.g., tablet, capsule, or a solution for injection or infusion). In some embodiments, the second therapeutic agent is a drug that is useful in treating or preventing a fibrotic pathology. Suitable examples of such drugs include nintedanib, pirfenidone, or prednisone, or mmunosuppressants, such as cyclophosphamide, azathioprine, methotrexate, penicillamine, and cyclosporine. In some embodiments, the additional therapeutic agent is dopamine, or a pharmaceutically acceptable salt thereof.

### Kits

The present invention also includes pharmaceutical kits useful, for example, in the treatment of disorders, diseases and conditions referred to herein, which include one or more containers containing a pharmaceutical composition comprising a therapeutically effective amount of a compound of the present disclosure. Such kits can further include, if desired, one or more of various conventional pharmaceutical kit components, such as, for example, containers with one or more pharmaceutically acceptable carriers, additional containers, etc. Instructions, either as inserts or as labels, indicating quantities of the components to be administered, guidelines for administration, and/or guidelines for mixing the components, can also be included in the kit.

### Definitions

As used herein, the term "about" means "approximately" (e.g., plus or minus approximately 10% of the indicated value).

As used herein, the term "compound" as used herein is meant to include all stereoisomers, geometric isomers, tautomers, and isotopes of the structures named or depicted. Compounds herein identified by name or structure as one particular tautomeric form are intended to include other tautomeric forms unless otherwise specified.

The terms "pharmaceutical" and "pharmaceutically acceptable" are employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein, the term "cell" is meant to refer to a cell that is *in vitro, ex vivo* or *in vivo.* In some embodiments, an *ex vivo* cell can be part of a tissue sample excised from an organism such as a mammal. In some embodiments, an *in vitro* cell can be a cell in a cell culture. In some embodiments, an *in vivo* cell is a cell living in an organism such as a mammal. In some embodiments, the cell is a mesenchymal cell. In some embodiments, the cell is a fibroblast (e.g., cardiac, dermal or lung fibroblast). In some embodiments, the cell is a hepatic stellate cell.

As used herein the term "treating" or "treatment" refers to 1) inhibiting the disease; for example, inhibiting a disease, condition or disorder in an individual who is experiencing or displaying the pathology or symptomatology of the disease, condition or disorder (i.e., arresting further development of the pathology and/or symptomatology), or 2) ameliorating the disease; for example, ameliorating a disease, condition or disorder in an individual who is experiencing or displaying the pathology or symptomatology of the disease, condition or disorder (i.e., reversing the pathology and/or symptomatology).

As used herein, the term "preventing" or "prevention" of a disease, condition or disorder refers to decreasing the risk of occurrence of the disease, condition or disorder in a subject or group of subjects (e.g., a subject or group of subjects predisposed to or susceptible to the disease, condition or disorder). In some embodiments, preventing a disease, condition or disorder refers to decreasing the possibility of acquiring the disease, condition or disorder and/or its associated symptoms. In some embodiments, preventing a disease, condition or disorder refers to completely or almost completely stopping the disease, condition or disorder from occurring.

As used herein, the term "pharmaceutically acceptable salt" refers to a salt that is formed between an acid and a basic group of the compound, such as an amino functional group, or a base and an acidic group of the compound, such as a carboxyl functional group. In some embodiments, the compound is a pharmaceutically acceptable acid addition salt. In some embodiments, acids commonly employed to form pharmaceutically acceptable salts of the therapeutic compounds described herein include inorganic acids such as hydrogen bisulfide, hydrochloric acid, hydrobromic acid, hydroiodic acid, sulfuric acid and phosphoric acid, as well as organic acids such as para-toluenesulfonic acid, salicylic acid, tartaric acid, bitartaric acid, ascorbic acid, maleic acid, besylic acid, fumaric acid, gluconic acid, glucuronic acid, formic acid, glutamic acid, methanesulfonic acid, ethanesulfonic acid, benzenesulfonic acid, lactic acid, oxalic acid, para-bromophenylsulfonic acid, carbonic acid, succinic acid, citric acid, benzoic acid and acetic acid, as well as related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylene sulfonate, phenylacetate, phenylpropionate, phenylbutyrate, citrate, lactate, β-hydroxybutyrate, glycolate, maleate, tartrate, methanesulfonate, propanesulfonate, naphthalene-1-sulfonate, naphthalene-2- sulfonate, mandelate and other salts. In one embodiment, pharmaceutically acceptable acid addition salts include those formed with mineral acids such as hydrochloric acid and hydrobromic acid, and especially those formed with organic acids such as maleic acid.

In some embodiments, bases commonly employed to form pharmaceutically acceptable salts of the therapeutic compounds described herein include hydroxides of alkali metals, including sodium, potassium, and lithium; hydroxides of alkaline earth metals such as calcium and magnesium; hydroxides of other metals, such as aluminum and zinc; ammonia, organic amines such as unsubstituted or hydroxyl-substituted mono-, di-, or tri-alkylamines, dicyclohexylamine; tributyl amine; pyridine; N-methyl, N-ethylamine; diethylamine; triethylamine; mono-, bis-, or tris-(2-OH-(C1-C6)-alkylamine), such as N,N-dimethyl-N-(2-hydroxyethyl)amine or tri-(2-hydroxyethyl)amine; N-methyl-D-glucamine; morpholine; thiomorpholine; piperidine; pyrrolidine; and amino acids such as arginine, lysine, and the like.

As used in the present application, the term "Cₙ₋ₘ alkoxy", employed alone or in combination with other terms, refers to a group of formula -O-Cₙ₋ₘ alkyl, win the present application the alkyl group contains n to m carbon atoms. Suitable examplary alkoxy groups include methoxy, ethoxy, propoxy (for example, n-propoxy and isopropoxy), butoxy (for example, n-butoxy and tert-butoxy), and the like. In some embodiments, the alkoxy group has 1 to 6, 1 to 4, or 1 to 3 carbon atoms.

As used in the present application, "Cₙ₋ₘ haloalkoxy" refers to a group of formula -O-haloalkyl having n to m carbon atoms. An example haloalkoxy group is OCF₃. In some embodiments, the haloalkoxy group is fluorinated only. In some embodiments, the alkyl group has 1 to 6, 1 to 4, or 1 to 3 carbon atoms.

As used herein, the term "Cₙ₋ₘ alkylamino" refers to a group of formula -NH(alkyl), wherein the alkyl group has n to m carbon atoms. In some embodiments, the alkyl group has 1 to 6, 1 to 4, or 1 to 3 carbon atoms. Suitable examples of alkylamino groups include N-methylamino, N-ethylamino, N-propylamino (e.g., N-(*n*-propyl)amino and N-isopropylamino), N-butylamino (e.g., N-(n-butyl)amino and N-(*tert*-butyl)amino), and the like.

As used herein, the term "di Cₙ₋ₘ alkylamino" refers to a group of formula -N(alkyl)₂, wherein each alkyl group independently has n to m carbon atoms. In some embodiments, each alkyl group has 1 to 6, 1 to 4, or 1 to 3 carbon atoms. Suitable examples of dialkylamino groups include N,N-methylehtylamino, N,N-diethylamino, N,N-propylethylamino, N,N-butylisopropylamino, and the like.

### EXAMPLES

### Materials and methods

*Cell culture:* Cells were all maintained in EMEM (ATCC) containing 10% FBS, unless otherwise noted. IMR-90 embryonic lung fibroblasts and NIH-3T3 mouse fibroblasts were purchased from ATCC. Doxycycline-inducible Tet-On NIH3T3 expressing TAZ4SA or control empty vector were described previously. Normal Human Alveolar Epithelial Cells (NHAEpCs), Normal Human Microvascular Endothelial Cells (NHMVECs), Normal Human Lung Fibroblasts (NHLFs), and Human Dermal Fibroblasts (HDFs) were purchased from Lonza and were cultured in the proprietary media per Lonza's recommendation. Human Adult Cardiac Fibroblasts (HACFs) and Hepatic Stellate Cells (HSCs) were purchased from ScienCell and were cultured in the proprietary media per ScienCell's recommendation. Hepatocytes were purchased from Samsara and were cultured in the proprietary media per Samsara's recommendation. All additional experiments with pulmonary fibroblasts used primary human lung fibroblasts isolated by explant culture from the lungs of subjects diagnosed with IPF who underwent lung transplantation, or donors whose organs were rejected for transplantation (non-IPF), generously provided by Peter Bitterman and Craig Henke at the University of Minnesota under a protocol approved by the University of Minnesota Institutional Review Board. All primary cell culture experiments were performed with cells at passage six or less.

*Chemicals and Reagents:* Dimethyl sulfoxide (DMSO), Y-27632, endothelin 1 (ET-1), and ascorbic acid were purchased from Sigma-Aldrich. Dihydrexidine, SKF-81297, Fenoldopam, Forskolin, and Prostaglandin E2 were purchased from Tocris Bioscience. Lysophosphatidic Acid (LPA), and Serotonin (5-HT) were purchased from Cayman Chemical. SCH 39166 was purchased from Santa Cruz Biotechnology. TGFβ1 was purchased from eBioscience.

*GPCRome Profiling and qPCR:* GPCRome profiling was performed according to the manufacturer's suggestions (Qiagen). Cells were grown in their recommended growth media for 24 hours prior to RNA isolation using RNeasy Plus Mini Kit (Qiagen) according to manufacturer's instructions. Isolated RNA (1000 ng) was then used to synthesize cDNA using the RT² First Strand Kit (Qiagen) and the G Protein Coupled Receptors 384HT PCR Array was analyzed using a LightCycler 480 (Roche). Data are shown as 1/Ct (Fig. 1b), GAPDH for fibroblast and epithelial cell datasets were nearly identical (17.39 and 17.41 respectively). Raw Ct values for all receptors are available (Table 1).

**Table 1**

| Receptors marked (*) were identified to exclusively couple to Gα_{S}²¹ | | | | | |
|---|---|---|---|---|---|
| **Uni Gene** | **Gen Bank** | **Symbol** | **Description** | **Fibro blast cT** | **Aveolar Epithelial cT** |
| Hs.37 7783 | NM_001 118 | ADCY AP1R1 | Adenylate cyclase activating polypeptide 1 (pituitary) receptor type I | 34.98 | 34.41 |
| Hs.77 867 | NM_000 674 | ADOR A1 | Adenosine A1 receptor | 29.7 | 27.95 |
| Hs. 19 7029 | NM_000 675 | ADOR A2A | Adenosine A2a receptor | 32.49 | 30.97 |
| Hs. 16 7046 | NM_000 676 | ADOR A2B | Adenosine A2b receptor | 26.82 | 26.08 |
| Hs.28 1342 | NM_000 677 | ADOR A3 | Adenosine A3 receptor | 37.78 | 35.78 |
| Hs.70 9175 | NM_033 303 | ADRA 1A | Adrenergic, alpha-1A-, receptor | 34.69 | 33.45 |
| Hs.36 8632 | NM_000 679 | ADRA 1B | Adrenergic, alpha-1B-, receptor | 32.9 | 29.79 |
| Hs.55 7 | NM_000 678 | ADRA 1D | Adrenergic, alpha-1D-, receptor | 29.16 | 32.89 |
| Hs.24 9159 | NM_000 681 | ADRA 2A | Adrenergic, alpha-2A-, receptor | 33.65 | 34.51 |
| Hs.24 7686 | NM_000 682 | ADRA 2B | Adrenergic, alpha-2B-, receptor | 36.07 | 40 |
| Hs. 12 3022 | NM_000 683 | ADRA 2C | Adrenergic, alpha-2C-, receptor | 33.79 | 33.89 |
| Hs.99 913 | NM_000 684 | ADRB1 | Adrenergic, beta-1-, receptor | 36.08 | 30.93 |
| Hs.59 1251 | NM_000 024 | ADRB2 | Adrenergic, beta-2-, receptor, surface | 24.66 | 22.13 |
| Hs.25 49 | NM_000 025 | ADRB3 | Adrenergic, beta-3-, receptor | 33.1 | 34.93 |
| Hs.72 8754 | NM_031 850 | AGTR1 | Angiotensin II receptor, type 1 | 26.71 | 31.48 |
| Hs.40 5348 | NM_000 686 | AGTR2 | Angiotensin II receptor, type 2 | 35.14 | 40 |
| Hs.43 8311 | NM_005 161 | APLNR | Apelin receptor | 36.26 | 36.72 |
| Hs.21 31 | NM_000 706 | AVPR1 A | Arginine vasopressin receptor 1A | 33.8 | 32.59 |

| **Uni Gene** | **Gen Bank** | **Symbol** | **Description** | **Fibro blast cT** | **Aveolar Epithelial cT** |
|---|---|---|---|---|---|
| Hs. 13 72 | NM_000 707 | AVPR1 B | Arginine vasopressin receptor 1B | 30.85 | 27.69 |
| Hs.56 7240 | NM_000 054 | AVPR2 | Arginine vasopressin receptor 2 (*) | 35.1 | 39 |
| Hs. 19 4654 | NM_001 702 | BAI1 | Brain-specific angiogenesis inhibitor 1 | 35.5 | 31.24 |
| Hs.52 4138 | NM_001 703 | BAI2 | Brain-specific angiogenesis inhibitor 2 | 28.05 | 31.49 |
| Hs. 13 261 | NM_001 704 | BAI3 | Brain-specific angiogenesis inhibitor 3 | 34.63 | 35.72 |
| Hs.52 5572 | NM_000 710 | BDKR B1 | Bradykinin receptor B 1 | 25.52 | 31.86 |
| Hs.65 4542 | NM_000 623 | BDKR B2 | Bradykinin receptor B2 | 29.25 | 32.77 |
| Hs. 12 1484 | NM_001 727 | BRS3 | Bombesin-like receptor 3 | 34.59 | 34.82 |
| Hs.59 1148 | NM_004 054 | C3AR1 | Complement component 3a receptor 1 | 31.75 | 31.58 |
| Hs.21 61 | NM_001 736 | C5AR1 | Complement component 5a receptor 1 | 30.59 | 30.75 |
| Hs.48 9127 | NM_001 742 | CALC R | CALCITONIN RECEPTOR | 38.19 | 37.02 |
| Hs.47 0882 | NM_005 795 | CALC RL | Calcitonin receptor-like | 31.19 | 28.81 |
| Hs.43 5615 | NM_000 388 | CASR | Calcium-sensing receptor | 31.32 | 31.84 |
| Hs. 14 6346 | NM_001 296 | CCBP2 | Chemokine binding protein 2 | 27.74 | 27.65 |
| Hs. 12 9 | NM_000 730 | CCKA R | Cholecystokinin A receptor | 32.43 | 36.62 |
| Hs.20 3 | NM_176 875 | CCKB R | Cholecystokinin B receptor | 34.99 | 39.34 |
| Hs.30 1921 | NM_001 295 | CCR1 | Chemokine (C-C motif) receptor 1 | 30.53 | 30.92 |
| Hs.27 8446 | NM_016 602 | CCR10 | Chemokine (C-C motif) receptor 10 | 29.82 | 28.25 |
| Hs.51 1794 | NM_001 123396 | CCR2 | Chemokine (C-C motif) receptor 2 | 34.75 | 33.51 |
| Hs.50 6190 | NM_001 837 | CCR3 | Chemokine (C-C motif) receptor 3 | 33.35 | 36.14 |
| Hs.18 4926 | NM_005 508 | CCR4 | Chemokine (C-C motif) receptor 4 | 32.99 | 34.95 |
| Hs.45 0802 | NM_000 579 | CCR5 | Chemokine (C-C motif) receptor 5 | 32.86 | 32.49 |
| Hs.46 468 | NM_004 367 | CCR6 | Chemokine (C-C motif) receptor 6 | 33.77 | 30.9 |
| Hs.37 0036 | NM_001 838 | CCR7 | Chemokine (C-C motif) receptor 7 | 30.45 | 32.7 |
| Hs.11 3222 | NM_005 201 | CCR8 | Chemokine (C-C motif) receptor 8 | 36.9 | 40 |
| Hs.22 5946 | NM_006 641 | CCR9 | Chemokine (C-C motif) receptor 9 | 33.71 | 35.12 |
| Hs.72 9361 | NM_016 557 | CCRL1 | Chemokine (C-C motif) receptor-like 1 | 24.68 | 29.02 |
| Hs.53 5713 | NM_003 965 | CCRL2 | Chemokine (C-C motif) receptor-like 2 | 32.82 | 26.46 |
| Hs.46 6039 | NM_001 784 | CD97 | CD97 molecule | 23.15 | 23.23 |
| Hs.25 2387 | NM_014 246 | CELSR 1 | Cadherin, EGF LAG seven-pass G-type receptor 1 | 29.57 | 27.06 |
| Hs.57 652 | NM_001 408 | CELSR 2 | Cadherin, EGF LAG seven-pass G-type receptor 2 | 32.54 | 31.57 |
| Hs.63 1926 | NM_001 407 | CELSR 3 | Cadherin, EGF LAG seven-pass G-type receptor 3 | 32.97 | 31.12 |
| Hs.63 2119 | NM_000 738 | CHRM 1 | Cholinergic receptor, muscarinic 1 | 40 | 35.56 |
| Hs.53 5891 | NM_000 739 | CHRM 2 | Cholinergic receptor, muscarinic 2 | 26.19 | 31.13 |
| Hs.71 38 | NM_000 740 | CHRM 3 | Cholinergic receptor, muscarinic 3 | 36.75 | 36.74 |
| Hs.24 8100 | NM_000 741 | CHRM 4 | Cholinergic receptor, muscarinic 4 | 30.1 | 29.43 |
| Hs.58 4747 | NM_012 125 | CHRM 5 | Cholinergic receptor, muscarinic 5 | 31.48 | 31.18 |
| Hs.19 7143 | NM_004 072 | CMKL R1 | CHEMOKINE-LIKE RECEPTOR 1 | 34.55 | 34.77 |
| Hs.75 110 | NM_016 083 | CNR 1 | Cannabinoid receptor 1 (brain) | 36.13 | 33.43 |
| Hs.73 037 | NM_001 841 | CNR2 | Cannabinoid receptor 2 (macrophage) | 27.88 | 26.15 |
| Hs.30 0684 | NM_014 478 | CRCP | CGRP receptor component | 24.2 | 23.45 |
| Hs.41 7628 | NM_004 382 | CRHR1 | Corticotropin releasing hormone receptor 1 | 33 | 32.61 |
| Hs.72 9970 | NM_001 883 | CRHR2 | Corticotropin releasing hormone receptor 2 | 35.59 | 35.16 |
| Hs.78 913 | NM_001 337 | CX3CR 1 | Chemokine (C-X3-C motif) receptor 1 | 35.81 | 36.67 |
| Hs.19 4778 | NM_000 634 | CXCR1 | Chemokine (C-X-C motif) receptor 1 | 38.63 | 34.23 |
| Hs.84 6 | NM_001 557 | CXCR2 | Chemokine (C-X-C motif) receptor 2 | 26.1 | 25.7 |
| Hs. 19 8252 | NM_001 504 | CXCR3 | Chemokine (C-X-C motif) receptor 3 | 31.54 | 30.31 |
| Hs.59 3413 | NM_003 467 | CXCR4 | Chemokine (C-X-C motif) receptor 4 | 33.79 | 27.46 |
| Hs.11 3916 | NM_001 716 | CXCR5 | Chemokine (C-X-C motif) receptor 5 | 34.73 | 31.79 |
| Hs.34 526 | NM_006 564 | CXCR6 | Chemokine (C-X-C motif) receptor 6 | 29.43 | 28.22 |
| Hs.47 1751 | NM_020 311 | CXCR7 | Chemokine (C-X-C motif) receptor 7 | 29.5 | 29.02 |
| Hs.20 1300 | NM_006 639 | CYSLT R1 | Cysteinyl leukotriene receptor 1 | 34.63 | 35.83 |
| Hs.25 3706 | NM_020 377 | CYSLT R2 | Cysteinyl leukotriene receptor 2 | 36.85 | 35.32 |
| Hs.15 3381 | NM_002 036 | DARC | Duffy blood group, chemokine receptor | 40 | 37.48 |
| Hs.26 24 | NM_000 794 | DRD1 | Dopamine receptor D1 (*) | 26.47 | 40 |
| Hs.73 893 | NM_000 795 | DRD2 | Dopamine receptor D2 | 32.97 | 30.29 |
| Hs. 12 1478 | NM_000 796 | DRD3 | Dopamine receptor D3 | 32.27 | 33.02 |
| Hs.99 922 | NM_000 797 | DRD4 | Dopamine receptor D4 | 33.5 | 33.22 |
| Hs.38 0681 | NM_000 798 | DRD5 | Dopamine receptor D5 (*) | 35.72 | 35 |
| Hs. 18 3713 | NM_001 957 | EDNR A | Endothelin receptor type A | 24.05 | 28.15 |
| Hs.82 002 | NM_000 115 | EDNR B | Endothelin receptor type B | 27.72 | 27.09 |
| Hs. 13 2314 | NM_022 159 | ELTD1 | EGF, latrophilin and seven transmembrane domain containing 1 | 25.5 | 27.01 |
| Hs.23 75 | NM_001 974 | EMR1 | Egf-like module containing, mucin-like, hormone receptor-like 1 | 31.03 | 32.47 |
| Hs.48 2562 | NM_001 992 | F2R | Coagulation factor II (thrombin) receptor | 20.46 | 23.5 |
| Hs.15 4299 | NM_005 242 | F2RL1 | Coagulation factor II (thrombin) receptor-like 1 | 23.32 | 20.47 |
| Hs.42 502 | NM_004 101 | F2RL2 | Coagulation factor II (thrombin) receptor-like 2 | 23.34 | 29.22 |
| Hs.13 7574 | NM_003 950 | F2RL3 | Coagulation factor II (thrombin) receptor-like 3 | 32.52 | 30.04 |
| Hs.24 8127 | NM_005 303 | FFAR1 | Free fatty acid receptor 1 | 34.82 | 33.99 |
| Hs.24 8056 | NM_005 306 | FFAR2 | Free fatty acid receptor 2 | 40 | 36.9 |
| Hs.24 8055 | NM_005 304 | FFAR3 | Free fatty acid receptor 3 | 35.52 | 35.47 |
| Hs.75 3 | NM_002 029 | FPR1 | Formyl peptide receptor 1 | 35.8 | 40 |
| Hs.99 855 | NM_001 462 | FPR2 | Formyl peptide receptor 2 | 33.2 | 34.11 |
| Hs.44 5466 | NM_002 030 | FPR3 | Formyl peptide receptor 3 | 33.45 | 33.8 |
| Hs. 14 28 | NM_181 446 | FSHR | Follicle stimulating hormone receptor | 35.87 | 35.7 |
| Hs.94 234 | NM_003 505 | FZD1 | Frizzled family receptor 1 | 23.26 | 25.3 |
| Hs.31 664 | NM_007 197 | FZD 10 | Frizzled family receptor 10 | 37.64 | 31.86 |
| Hs. 14 2912 | NM_001 466 | FZD2 | Frizzled family receptor 2 | 26.96 | 25.31 |
| Hs.40 735 | NM_017 412 | FZD3 | Frizzled family receptor 3 (*) | 29.18 | 25.21 |
| Hs. 19 545 | NM_012 193 | FZD4 | Frizzled family receptor 4 | 25.59 | 27.44 |
| Hs. 17 631 | NM_003 468 | FZD5 | Frizzled family receptor 5 | 29.48 | 25.43 |
| Hs.59 1863 | NM_003 506 | FZD6 | Frizzled family receptor 6 | 23.58 | 22.69 |
| Hs.17 3859 | NM_003 507 | FZD7 | Frizzled family receptor 7 | 25.43 | 28.28 |
| Hs.30 2634 | NM_031 866 | FZD8 | Frizzled family receptor 8 | 28.64 | 27.28 |
| Hs.64 7029 | NM_003 508 | FZD9 | Frizzled family receptor 9 | 33.01 | 33.15 |
| Hs. 16 7017 | NM_001 470 | GABB R1 | Gamma-aminobutyric acid (GABA) B receptor, 1 | 29.81 | 30.5 |
| Hs.19 8612 | NM_005 458 | GABB R2 | Gamma-aminobutyric acid (GABA) B receptor, 2 | 23.5 | 29.12 |
| Hs.27 2191 | NM_001 480 | GALR1 | Galanin receptor 1 | 37.82 | 35.83 |
| Hs.66 6366 | NM_003 857 | GALR2 | GALANIN RECEPTOR 2 | 34.97 | 33.03 |
| Hs.15 8353 | NM_003 614 | GALR3 | Galanin receptor 3 | 35.06 | 40 |
| Hs.20 8 | NM_000 160 | GCGR | Glucagon receptor (*) | 40 | 33.78 |
| Hs.76 7 | NM_000 823 | GHRH R | Growth hormone releasing hormone receptor (*) | 32.27 | 32.5 |
| Hs.24 8115 | NM_004 122 | GHSR | Growth hormone secretagogue receptor | 35.58 | 33.62 |
| Hs.65 8534 | NM_000 164 | GIPR | Gastric inhibitory polypeptide receptor (*) | 31.28 | 29.57 |
| Hs.38 9103 | NM_002 062 | GLP1R | Glucagon-like peptide 1 receptor | 40 | 37.36 |
| Hs.24 8202 | NM_004 246 | GLP2R | Glucagon-like peptide 2 receptor | 35.47 | 37.79 |
| Hs.40 7587 | NM_000 406 | GNRH R | Gonadotropin-releasing hormone receptor | 33.11 | 32.34 |
| Hs. 16 0954 | NM_170 699 | GPBA R1 | G protein-coupled bile acid receptor 1 (*) | 32.49 | 31.71 |
| Hs.20 961 | NM_001 505 | GPER | G protein-coupled estrogen receptor 1 | 28.52 | 28.89 |
| Hs.18 4907 | NM_005 279 | GPR1 | G protein-coupled receptor 1 | 26.65 | 26.31 |
| Hs.35 0569 | NM_054 021 | GPR10 1 | G protein-coupled receptor 101 (*) | 34.32 | 35.07 |
| Hs.25 6897 | NM_153 840 | GPR11 0 | G protein-coupled receptor 110 | 33.06 | 26.43 |
| Hs.71 5357 | NM_153 839 | GPR11 1 | G protein-coupled receptor 111 | 33.75 | 29.59 |
| Hs.38 1354 | NM_153 834 | GPR11 2 | G protein-coupled receptor 112 | 40 | 40 |
| Hs.63 1878 | NM_153 835 | GPR11 3 | G protein-coupled receptor 113 | 34.24 | 31.19 |
| Hs. 18 7884 | NM_153 837 | GPR11 4 | G protein-coupled receptor 114 | 31.86 | 30.84 |
| Hs.71 0050 | NM_153 838 | GPR11 5 | G protein-coupled receptor 115 | 40 | 23.51 |
| Hs.36 2806 | NM_015 234 | GPR11 6 | G protein-coupled receptor 116 | 27.99 | 21.72 |
| Hs.49 6762 | NM_178 471 | GPR11 9 | G protein-coupled receptor 119 (*) | 35.63 | 35.98 |
| Hs.12 3034 | NM_005 288 | GPR12 | G protein-coupled receptor 12 | 33.54 | 34.47 |
| Hs.43 5183 | NM_001 083909 | GPR12 3 | G protein-coupled receptor 123 | 34.4 | 36.7 |
| Hs.70 8086 | NM_032 777 | GPR12 4 | G protein-coupled receptor 124 | 27.98 | 34.7 |
| Hs.99 195 | NM_145 290 | GPR12 5 | G protein-coupled receptor 125 | 24.46 | 24.44 |
| Hs.71 5560 | NM_020 455 | GPR12 6 | G protein-coupled receptor 126 | 22.45 | 20.94 |
| Hs.33 4511 | NM_032 787 | GPR12 8 | G protein-coupled receptor 128 | 36.17 | 35.28 |
| Hs.53 2504 | NM_013 345 | GPR13 2 | G protein-coupled receptor 132 | 28.86 | 27.48 |
| Hs.65 6751 | NM_198 827 | GPR13 3 | G protein-coupled receptor 133 | 27.94 | 29.87 |
| Hs.64 7573 | NM_022 571 | GPR13 5 | G protein-coupled receptor 135 | 28.69 | 30.03 |
| Hs.44 6875 | NM_001 002911 | GPR13 9 | G protein-coupled receptor 139 | 30.45 | 29.85 |
| Hs.68 8230 | NM_181 791 | GPR14 1 | G protein-coupled receptor 141 | 37.06 | 39.01 |
| Hs.57 4368 | NM_181 790 | GPR14 2 | G protein-coupled receptor 142 | 35.8 | 34.46 |
| Hs.74 124 | NM_000 273 | GPR14 3 | G protein-coupled receptor 143 | 34.13 | 27.12 |
| Hs.45 4099 | NM_001 161808 | GPR14 4 | G protein-coupled receptor 144 | 33.57 | 34.42 |
| Hs.72 9332 | NM_138 445 | GPR14 6 | G protein-coupled receptor 146 | 31.74 | 30.75 |
| Hs.45 2574 | NM_207 364 | GPR14 8 | G protein-coupled receptor 148 | 35.2 | 35.27 |
| Hs.68 8231 | NM_001 038705 | GPR14 9 | G protein-coupled receptor 149 | 31.07 | 30.7 |
| Hs.56 3128 | NM_005 290 | GPR15 | G protein-coupled receptor 15 | 36.47 | 34.66 |
| Hs. 14 3315 | NM_199 243 | GPR15 0 | G protein-coupled receptor 150 | 32.57 | 33.87 |
| Hs.48 3732 | NM_194 251 | GPR15 1 | G protein-coupled receptor 151 | 33.16 | 32 |
| Hs.56 7997 | NM_206 997 | GPR15 2 | G protein-coupled receptor 152 | 30.54 | 30.27 |
| Hs.53 1581 | NM_207 370 | GPR15 3 | G protein-coupled receptor 153 | 27.19 | 27.2 |
| Hs.33 3358 | NM_153 002 | GPR15 6 | G protein-coupled receptor 156 | 32.07 | 31.58 |
| Hs.63 2367 | NM_024 980 | GPR15 7 | G protein-coupled receptor 157 | 27.57 | 25.28 |
| Hs.49 9108 | NM_020 752 | GPR15 8 | G protein-coupled receptor 158 | 33.68 | 31.79 |
| Hs.23 1320 | NM_014 373 | GPR16 0 | G protein-coupled receptor 160 | 29.28 | 24 |
| Hs.27 1809 | NM_153 832 | GPR16 1 | G protein-coupled receptor 161 | 25.87 | 24.7 |
| Hs.63 1654 | NM_014 449 | GPR16 2 | G protein-coupled receptor 162 | 26.71 | 28.97 |
| Hs.46 453 | NM_005 291 | GPR17 | G protein-coupled receptor 17 | 31.26 | 30.45 |
| Hs.54 9152 | NM_013 308 | GPR17 1 | G protein-coupled receptor 171 | 33.11 | 32.54 |
| Hs.66 1815 | NM_018 969 | GPR17 3 | G protein-coupled receptor 173 | 28.15 | 30.92 |
| Hs.32 6713 | NM_032 553 | GPR17 4 | G protein-coupled receptor 174 | 38.4 | 38.98 |
| Hs.37 196 | NM_007 223 | GPR 17 6 | G protein-coupled receptor 176 | 21.97 | 23.55 |
| Hs.46 2915 | NM_001 004334 | GPR 17 9 | G protein-coupled receptor 179 | 33.88 | 32.21 |
| Hs.63 1765 | NM_005 292 | GPR 18 | G protein-coupled receptor 18 | 29.78 | 29.19 |
| Hs.48 3909 | NM_007 264 | GPR18 2 | G protein-coupled receptor 182 | 32.29 | 31.2 |
| Hs.78 4 | NM_004 951 | GPR18 3 | G protein-coupled receptor 183 | 27.5 | 27.52 |
| Hs.65 7862 | NM_006 143 | GPR 19 | G protein-coupled receptor 19 | 31.65 | 32.27 |
| Hs.18 8859 | NM_005 293 | GPR20 | G protein-coupled receptor 20 | 34.57 | 34.59 |
| Hs.72 8941 | NM_005 294 | GPR21 | G protein-coupled receptor 21 | 31.02 | 29.77 |
| Hs.65 7277 | NM_005 295 | GPR22 | G protein-coupled receptor 22 | 31.47 | 29.99 |
| Hs.53 4316 | NM_005 298 | GPR25 | G protein-coupled receptor 25 | 37.46 | 38.18 |
| Hs. 12 751 | NM_153 442 | GPR26 | G protein-coupled receptor 26 (*) | 34.83 | 34.88 |
| Hs.59 1653 | NM_018 971 | GPR27 | G protein-coupled receptor 27 | 28.07 | 29.67 |
| Hs.66 542 | NM_005 281 | GPR3 | G protein-coupled receptor 3 (*) | 26.17 | 26.56 |
| Hs.24 8124 | NM_005 299 | GPR31 | G protein-coupled receptor 31 | 31 | 30.4 |
| Hs.51 5555 | NM_001 506 | GPR32 | G protein-coupled receptor 32 | 36.07 | 33.68 |
| Hs.49 5989 | NM_005 300 | GPR34 | G protein-coupled receptor 34 | 31.49 | 30.5 |
| Hs.23 9891 | NM_005 301 | GPR35 | G protein-coupled receptor 35 | 34.2 | 33.59 |
| Hs.40 6094 | NM_005 302 | GPR37 | G protein-coupled receptor 37 | 24.85 | 26.61 |
| Hs.13 2049 | NM_004 767 | GPR37 L1 | G protein-coupled receptor 37 like 1 | 32.8 | 30.17 |
| Hs.43 2395 | NM_001 508 | GPR39 | G protein-coupled receptor 39 | 28.53 | 25.46 |
| Hs.17 170 | NM_005 282 | GPR4 | G protein-coupled receptor 4 | 29.13 | 28.77 |
| Hs.29 9567 | NM_004 778 | PTGDR 2 | Prostaglandin D2 receptor 2 | 37.67 | 37.96 |
| Hs.59 0903 | NM_007 227 | GPR45 | G protein-coupled receptor 45 | 33.14 | 35.04 |
| Hs.56 7390 | NM_004 224 | GPR50 | G protein-coupled receptor 50 | 34.9 | 34.67 |
| Hs.67 3850 | NM_005 684 | GPR52 | G protein-coupled receptor 52 | 31.7 | 31.63 |
| Hs.11 4545 | NM_005 683 | GPR55 | G protein-coupled receptor 55 | 33.55 | 33.22 |
| Hs.51 3633 | NM_005 682 | GPR56 | G protein-coupled receptor 56 | 26.56 | 24.79 |
| Hs.46 332 | NM_005 284 | GPR6 | G protein-coupled receptor 6 | 36.18 | 37.9 |
| Hs.70 9782 | NM_031 936 | GPR61 | G protein-coupled receptor 61 (*) | 37.71 | 35.49 |
| Hs.23 2213 | NM_080 865 | GPR62 | G protein-coupled receptor 62 | 35.19 | 35.44 |
| Hs.63 2612 | NM_030 784 | GPR63 | G protein-coupled receptor 63 | 28.62 | 28.31 |
| Hs.14 6978 | NM_005 756 | GPR64 | G protein-coupled receptor 64 | 30.16 | 29.06 |
| Hs.51 3440 | NM_003 608 | GPR65 | G protein-coupled receptor 65 (*) | 32.03 | 32.56 |
| Hs.88 82 | NM_003 485 | GPR68 | G protein-coupled receptor 68 | 25.81 | 30.14 |
| Hs.69 6596 | NM_006 794 | GPR75 | G protein-coupled receptor 75 | 25.52 | 26.2 |
| Hs.53 4412 | NM_018 485 | GPR77 | G protein-coupled receptor 77 | 36.11 | 34.31 |
| Hs.35 0588 | NM_080 819 | GPR78 | G protein-coupled receptor 78 (*) | 32.78 | 32.56 |
| Hs.66 4795 | NM_080 817 | GPR82 | G protein-coupled receptor 82 | 31.72 | 30.15 |
| Hs.27 2385 | NM_016 540 | GPR83 | G protein-coupled receptor 83 | 30.91 | 30.45 |
| Hs.30 6199 | NM_020 370 | GPR84 | G protein-coupled receptor 84 | 35.03 | 33.96 |
| Hs. 15 2009 | NM_018 970 | GPR85 | G protein-coupled receptor 85 | 27.77 | 31.55 |
| Hs.59 1292 | NM_023 915 | GPR87 | G protein-coupled receptor 87 | 32.93 | 25.64 |
| Hs. 17 0053 | NM_022 049 | GPR88 | G protein-coupled receptor 88 | 30.94 | 30.86 |
| Hs.38 3403 | NM_170 776 | GPR97 | G protein-coupled receptor 97 | 32.72 | 32 |
| Hs.59 1777 | NM_032 119 | GPR98 | G protein-coupled receptor 98 | 35.7 | 28.62 |
| Hs.63 1733 | NM_003 979 | GPRC5 A | G protein-coupled receptor, family C, group 5, member A | 23.58 | 17.06 |
| Hs. 14 8685 | NM_016 235 | GPRC5 B | G protein-coupled receptor, family C, group 5, member B | 29.27 | 22.56 |
| Hs.44 6438 | NM_018 653 | GPRC5 C | G protein-coupled receptor, family C, group 5, member C | 33.92 | 25.51 |
| Hs.64 4599 | NM_018 654 | GPRC5 D | G protein-coupled receptor, family C, group 5, member D | 29.49 | 25.92 |
| Hs.26 6745 | NM_148 963 | GPRC6 A | G protein-coupled receptor, family C, group 6, member A | 34.22 | 39.42 |
| Hs. 12 8848 | NM_000 831 | GRIK3 | Glutamate receptor, ionotropic, kainate 3 | 40 | 40 |
| Hs.32 945 | NM_000 838 | GRM1 | Glutamate receptor, metabotropic 1 | 36.87 | 34.53 |
| Hs. 12 1510 | NM_000 839 | GRM2 | Glutamate receptor, metabotropic 2 | 32.26 | 32.67 |
| Hs.59 0575 | NM_000 840 | GRM3 | Glutamate receptor, metabotropic 3 | 36.87 | 32.92 |
| Hs.65 4847 | NM_000 841 | GRM4 | Glutamate receptor, metabotropic 4 | 33.12 | 32.9 |
| Hs. 14 7361 | NM_000 842 | GRM5 | Glutamate receptor, metabotropic 5 | 31.84 | 30.8 |
| Hs.24 8131 | NM_000 843 | GRM6 | Glutamate receptor, metabotropic 6 | 35.33 | 33.24 |
| Hs.60 6393 | NM_000 844 | GRM7 | Glutamate receptor, metabotropic 7 | 33.68 | 32.69 |
| Hs.44 9625 | NM_000 845 | GRM8 | Glutamate receptor, metabotropic 8 | 34.05 | 40 |
| Hs.56 7282 | NM_005 314 | GRPR | Gastrin-releasing peptide receptor | 28.1 | 30.68 |
| Hs.61 0873 | NM_032 554 | HCAR1 | Hydroxycarboxylic acid receptor 1 | 31.72 | 30.02 |
| Hs.52 4812 | NM_177 551 | HCAR2 | Hydroxycarboxylic acid receptor 2 | 33.92 | 32.17 |
| Hs.38 8226 | NM_001 525 | HCRT R1 | Hypocretin (orexin) receptor 1 | 33.89 | 33.48 |
| Hs.15 1624 | NM_001 526 | HCRT R2 | Hypocretin (orexin) receptor 2 | 37.99 | 35.2 |
| Hs. 15 70 | NM_000 861 | HRH1 | Histamine receptor H1 | 25.73 | 28.32 |
| Hs.24 7885 | NM_022 304 | HRH2 | Histamine receptor H2 | 32.13 | 31.68 |
| Hs.25 1399 | NM_007 232 | HRH3 | Histamine receptor H3 | 31.47 | 31.07 |
| Hs.28 7388 | NM_021 624 | HRH4 | Histamine receptor H4 | 33.14 | 31.81 |
| Hs.24 7940 | NM_000 524 | HTR1A | 5-hydroxytryptamine (serotonin) receptor 1A | 35.62 | 40 |
| Hs. 12 3016 | NM_000 863 | HTR1B | 5-hydroxytryptamine (serotonin) receptor 1B | 29.48 | 28.24 |
| Hs. 12 1482 | NM_000 864 | HTR1D | 5-hydroxytryptamine (serotonin) receptor 1D | 33.86 | 30.01 |
| Hs. 16 11 | NM_000 865 | HTR1E | 5-hydroxytryptamine (serotonin) receptor 1E | 40 | 37.33 |
| Hs.24 8136 | NM_000 866 | HTR1F | 5-hydroxytryptamine (serotonin) receptor 1F | 33.26 | 35.72 |
| Hs.65 4586 | NM_000 621 | HTR2A | 5-hydroxytryptamine (serotonin) receptor 2A | 32.74 | 33.32 |
| Hs.42 1649 | NM_000 867 | HTR2B | 5-hydroxytryptamine (serotonin) receptor 2B | 29.09 | 28.81 |
| Hs. 14 9037 | NM_000 868 | HTR2C | 5-hydroxytryptamine (serotonin) receptor 2C | 36.23 | 40 |
| Hs.41 3899 | NM_000 869 | HTR3A | 5-hydroxytryptamine (serotonin) receptor 3A | 33.43 | 31.46 |
| Hs.24 1377 | NM_006 028 | HTR3B | 5-hydroxytryptamine (serotonin) receptor 3B | 33.91 | 33.52 |
| Hs.48 3773 | NM_000 870 | HTR4 | 5-hydroxytryptamine (serotonin) receptor 4 | 34.83 | 34.95 |
| Hs.65 791 | NM_024 012 | HTR5A | 5-hydroxytryptamine (serotonin) receptor 5A | 35.84 | 34.13 |
| Hs.22 180 | NM_000 871 | HTR6 | 5-hydroxytryptamine (serotonin) receptor 6 | 35.5 | 36.22 |
| Hs.73 739 | NM_000 872 | HTR7 | 5-hydroxytryptamine (serotonin) receptor 7 (adenylate cyclase-coupled) (*) | 27.89 | 30.19 |
| Hs.20 8229 | NM_032 551 | KISS1 R | KISS1 receptor | 34.04 | 32.88 |
| Hs.70 5413 | NM_002 303 | LEPR | Leptin receptor | 26.57 | 24.8 |
| Hs.50 2176 | NM_018 490 | LGR4 | Leucine-rich repeat containing G protein-coupled receptor 4 | 24.47 | 28.09 |
| Hs.65 8889 | NM_003 667 | LGR5 | Leucine-rich repeat containing G protein-coupled receptor 5 | 33.8 | 33.2 |
| Hs.46 8490 | NM_000 233 | LHCG R | Luteinizing hormone/choriogonadotropin receptor | 35.45 | 34.77 |
| Hs.12 6667 | NM_057 159 | LPAR1 | Lysophosphatidic acid receptor 1 | 22.02 | 23.12 |
| Hs.12 2575 | NM_004 720 | LPAR2 | Lysophosphatidic acid receptor 2 | 29.18 | 25.33 |
| Hs.67 4915 | NM_012 152 | LPAR3 | Lysophosphatidic acid receptor 3 | 27.34 | 29.24 |
| Hs.52 2701 | NM_005 296 | LPAR4 | Lysophosphatidic acid receptor 4 | 31.29 | 35.52 |
| Hs.15 5538 | NM_020 400 | LPAR5 | Lysophosphatidic acid receptor 5 | 33.23 | 31.2 |
| Hs.12 3464 | NM_005 767 | LPAR6 | Lysophosphatidic acid receptor 6 | 28.73 | 26.09 |
| Hs.65 4658 | NM_014 921 | LPHN 1 | Latrophilin 1 | 33.57 | 29.74 |
| Hs.24 212 | NM_012 302 | LPHN2 | Latrophilin 2 | 23.44 | 22.42 |
| Hs.28 391 | NM_015 236 | LPHN3 | Latrophilin 3 | 34.52 | 28.43 |
| Hs.65 5431 | NM_181 657 | LTB4R | Leukotriene B4 receptor | 32.26 | 31.3 |
| Hs. 13 0685 | NM_019 839 | LTB4R 2 | Leukotriene B4 receptor 2 | 31.26 | 29.47 |
| Hs.99 900 | NM_002 377 | MAS1 | MAS1 oncogene | 36 | 38.64 |
| Hs.51 3829 | NM_002 386 | MC1R | Melanocortin 1 receptor (alpha melanocyte stimulating hormone receptor) (*) | 30.35 | 29.34 |
| Hs.24 8144 | NM_000 529 | MC2R | Melanocortin 2 receptor (adrenocorticotropic hormone) (*) | 40 | 40 |
| Hs.24 8018 | NM_019 888 | MC3R | Melanocortin 3 receptor (*) | 30.98 | 34.42 |
| Hs.53 2833 | NM_005 912 | MC4R | Melanocortin 4 receptor (*) | 35.44 | 34.64 |
| Hs.24 8145 | NM_005 913 | MC5R | Melanocortin 5 receptor (*) | 31.57 | 31.76 |
| Hs.24 8122 | NM_005 297 | MCHR 1 | Melanin-concentrating hormone receptor 1 | 34 | 32.94 |
| Hs.59 1342 | NM_032 503 | MCHR 2 | Melanin-concentrating hormone receptor 2 | 37.38 | 36.01 |
| Hs.52 7802 | NM_198 923 | MRGP RD | MAS-related GPR, member D | 31.31 | 30.07 |
| Hs.70 6565 | NM_001 039165 | MRGP RE | MAS-related GPR, member E | 31.61 | 31.18 |
| Hs.11 8513 | NM_145 015 | MRGP RF | MAS-related GPR, member F | 25.45 | 31.62 |
| Hs.73 0306 | NM_001 164377 | MRGP RG | MAS-related GPR, member G | 27.3 | 27.83 |
| Hs.71 1459 | NM_147 199 | MRGP RX1 | MAS-related GPR, member X1 | 37.05 | 38.5 |
| Hs.35 0566 | NM_054 030 | MRGP RX2 | MAS-related GPR, member X2 | 25.94 | 30.83 |
| Hs.38 0177 | NM_054 031 | MRGP RX3 | MAS-related GPR, member X3 | 34.05 | 40 |
| Hs.63 2138 | NM_054 032 | MRGP RX4 | MAS-related GPR, member X4 | 35.65 | 35.67 |
| Hs.24 3467 | NM_005 958 | MTNR 1A | Melatonin receptor 1A | 34.82 | 33.03 |
| Hs.56 9039 | NM_005 959 | MTNR 1B | Melatonin receptor 1B | 30.7 | 30.61 |
| Hs.65 4478 | NM_002 511 | NMBR | Neuromedin B receptor | 32.99 | 34.44 |
| Hs.47 1619 | NM_006 056 | NMUR 1 | Neuromedin U receptor 1 | 33.93 | 32.54 |
| Hs.28 3093 | NM_020 167 | NMUR 2 | Neuromedin U receptor 2 | 34.63 | 34.07 |
| Hs.24 8117 | NM_005 285 | NPBW R1 | Neuropeptides B/W receptor 1 | 36.11 | 34.44 |
| Hs.24 8118 | NM_005 286 | NPBW R2 | Neuropeptides B/W receptor 2 | 37.46 | 36.74 |
| Hs.30 2026 | NM_022 146 | NPFFR 1 | Neuropeptide FF receptor 1 | 34.41 | 34.73 |
| Hs.99 231 | NM_053 036 | NPFFR 2 | Neuropeptide FF receptor 2 | 40 | 29.59 |
| Hs.49 0330 | NM_000 906 | NPR1 | Natriuretic peptide receptor A | 28.76 | 28.04 |
| Hs.78 518 | NM_003 995 | NPR2 | Natriuretic peptide receptor B | 25.45 | 25.75 |
| Hs.23 7028 | NM_000 908 | NPR3 | Natriuretic peptide receptor B | 26.24 | 27.09 |
| Hs.65 2373 | NM_207 172 | NPSR1 | Neuropeptide S receptor 1 | 34.05 | 34.78 |
| Hs.51 9057 | NM_000 909 | NPY1R | Neuropeptide Y receptor Y1 | 32.34 | 30.81 |
| Hs.37 125 | NM_000 910 | NPY2R | Neuropeptide Y receptor Y2 | 30.61 | 29.84 |
| Hs.59 8503 | NM_006 174 | NPY5R | Neuropeptide Y receptor Y5 | 38.24 | 35.21 |
| Hs.59 0869 | NM_002 531 | NTSR1 | Neurotensin receptor 1 (high affinity) | 27.66 | 32.7 |
| Hs. 13 1138 | NM_012 344 | NTSR2 | Neurotensin receptor 2 | 34.14 | 33.3 |
| Hs.67 7835 | NM_181 745 | O3FAR 1 | Omega-3 fatty acid receptor 1 | 35.31 | 32.92 |
| Hs.67 896 | NM_007 346 | OGFR | Opioid growth factor receptor | 26.18 | 25.76 |
| Hs.65 6404 | NM_001 708 | OPN1S W | Opsin 1 (cone pigments), short-wave-sensitive | 24.49 | 24.73 |
| Hs.53 4399 | NM_014 322 | OPN3 | Opsin 3 | 26.3 | 24.9 |
| Hs.28 3922 | NM_033 282 | OPN4 | Opsin 4 | 33.61 | 33.56 |
| Hs.21 3717 | NM_181 744 | OPN5 | Opsin 5 | 40 | 32.7 |
| Hs.37 2 | NM_000 911 | OPRD1 | Opioid receptor, delta 1 | 30.86 | 29.97 |
| Hs.10 6795 | NM_000 912 | OPRK1 | Opioid receptor, kappa 1 | 34.64 | 34.46 |
| Hs.28 59 | NM_000 913 | OPRL1 | Opiate receptor-like 1 | 33.94 | 31.44 |
| Hs.23 53 | NM_000 914 | OPRM 1 | Opioid receptor, mu 1 | 35.48 | 37.77 |
| Hs.35 2218 | NM_080 818 | OXGR 1 | Oxoglutarate (alpha-ketoglutarate) receptor 1 | 40 | 37.8 |
| Hs.28 20 | NM_000 916 | OXTR | Oxytocin receptor | 25.26 | 25.31 |
| Hs.65 4526 | NM_002 563 | P2RY1 | Purinergic receptor P2Y, G-protein coupled, 1 | 29.28 | 29.49 |
| Hs.29 6433 | NM_198 333 | P2RY1 0 | Purinergic receptor P2Y, G-protein coupled, 10 | 40 | 40 |
| Hs. 16 6168 | NM_002 566 | P2RY1 1 | Purinergic receptor P2Y, G-protein coupled, 11 | 31.61 | 31.58 |
| Hs.59 1281 | NM_022 788 | P2RY1 2 | Purinergic receptor P2Y, G-protein coupled, 12 | 32.42 | 32.59 |
| Hs.54 6396 | NM_176 894 | P2RY1 3 | Purinergic receptor P2Y, G-protein coupled, 13 | 33.65 | 33.98 |
| Hs.24 65 | NM_014 879 | P2RY1 4 | Purinergic receptor P2Y, G-protein coupled, 14 | 34.63 | 34.32 |
| Hs.33 9 | NM_002 564 | P2RY2 | Purinergic receptor P2Y, G-protein coupled, 2 | 35.47 | 27.48 |
| Hs.67 3854 | NM_002 565 | P2RY4 | Pyrimidinergic receptor P2Y, G-protein coupled, 4 | 34.14 | 32.57 |
| Hs.16 362 | NM_004 154 | P2RY6 | Pyrimidinergic receptor P2Y, G-protein coupled, 6 | 34.56 | 31.31 |
| Hs.11 1377 | NM_178 129 | P2RY8 | Purinergic receptor P2Y, G-protein coupled, 8 | 36.9 | 40 |
| Hs.50 9067 | NM_002 609 | PDGFR B | Platelet-derived growth factor receptor, beta polypeptide | 27.58 | 32.28 |
| Hs.45 8573 | NM_006 207 | PDGFR L | Platelet-derived growth factor receptor-like | 26.52 | 27.21 |
| Hs.52 4719 | NM_005 972 | PPYR1 | Pancreatic polypeptide receptor 1 | 29 | 28.22 |
| Hs.24 8119 | NM_004 248 | PRLHR | Prolactin releasing hormone receptor | 32.86 | 32.31 |
| Hs.68 3430 | NM_138 964 | PROK R1 | Prokineticin receptor 1 | 40 | 35.42 |
| Hs.37 5029 | NM_144 773 | PROK R2 | Prokineticin receptor 2 | 35.49 | 34.73 |
| Hs.70 9174 | NM_000 952 | PTAFR | Platelet-activating factor receptor | 31.98 | 29.55 |
| Hs.30 6831 | NM_000 953 | PTGDR | Prostaglandin D2 receptor (DP) (*) | 25.3 | 24.34 |
| Hs.15 9360 | NM_000 955 | PTGER 1 | Prostaglandin E receptor 1 (subtype EP1), 42kDa | 32.17 | 29.07 |
| Hs.20 90 | NM_000 956 | PTGER 2 | Prostaglandin E receptor 2 (subtype EP2), 53kDa (*) | 25.8 | 25.86 |
| Hs.44 5000 | NM_198 715 | PTGER 3 | Prostaglandin E receptor 3 (subtype EP3) | 26.95 | 29.88 |
| Hs. 19 9248 | NM_000 958 | PTGER 4 | Prostaglandin E receptor 4 (subtype EP4) | 26.62 | 25.16 |
| Hs.65 4365 | NM_000 959 | PTGFR | Prostaglandin F receptor (FP) | 27.13 | 33.28 |
| Hs.45 8324 | NM_000 960 | PTGIR | Prostaglandin 12 (prostacyclin) receptor (IP) | 28.32 | 30.43 |
| Hs.10 19 | NM_000 316 | PTH1R | Parathyroid hormone 1 receptor | 33.51 | 32.47 |
| Hs.57 0296 | NM_005 048 | PTH2R | Parathyroid hormone 2 receptor | 36.11 | 36.96 |
| Hs.36 8977 | NM_198 179 | QRFPR | Pyroglutamylated RFamide peptide receptor | 34.89 | 40 |
| Hs. 15 44 | NM_002 921 | RGR | Retinal G protein coupled receptor | 33.43 | 33.89 |
| Hs.24 7565 | NM_000 539 | RHO | Rhodopsin | 31.99 | 32.3 |
| Hs.65 8310 | NM_006 583 | RRH | Retinal pigment epithelium-derived rhodopsin homolog | 33.04 | 32.26 |
| Hs.59 1686 | NM_021 634 | RXFP1 | Relaxin/insulin-like family peptide receptor 1 | 33.24 | 36.53 |
| Hs.68 0763 | NM_130 806 | RXFP2 | Relaxin/insulin-like family peptide receptor 2 | 33.27 | 32.82 |
| Hs. 17 0146 | NM_016 568 | RXFP3 | Relaxin/insulin-like family peptide receptor 3 | 33.21 | 33.03 |
| Hs.44 9914 | NM_181 885 | RXFP4 | Relaxin/insulin-like family peptide receptor 4 | 34 | 32.75 |
| Hs.15 4210 | NM_001 400 | S1PR1 | Sphingosine-1-phosphate receptor 1 | 26.42 | 28.45 |
| Hs.65 5405 | NM_004 230 | S1PR2 | Sphingosine-1-phosphate receptor 2 | 23.97 | 25.2 |
| Hs.58 5118 | NM_005 226 | S1PR3 | Sphingosine-1-phosphate receptor 3 | 25.59 | 26.58 |
| Hs.66 2006 | NM_003 775 | S1PR4 | Sphingosine-1-phosphate receptor 4 | 33.52 | 30.76 |
| Hs.50 1561 | NM_030 760 | S1PR5 | Sphingosine-1-phosphate receptor 5 | 37.46 | 36.16 |
| Hs.42 091 | NM_002 980 | SCTR | Secretin receptor | 40 | 40 |
| Hs.52 2087 | NM_005 866 | SIGMA R1 | Sigma non-opioid intracellular receptor 1 | 21.57 | 22.06 |
| Hs.43 7846 | NM_005 631 | SMO | Smoothened, frizzled family receptor | 27.8 | 30.67 |
| Hs.59 1915 | NM_052 918 | SORCS 1 | Sortilin-related VPS10 domain containing receptor 1 | 32.14 | 33.05 |
| Hs.47 9099 | NM_020 777 | SORCS 2 | Sortilin-related VPS10 domain containing receptor 2 | 32.76 | 32.61 |
| Hs.67 1950 | NM_014 978 | SORCS 3 | Sortilin-related VPS10 domain containing receptor 3 | 40 | 30.11 |
| Hs.24 8160 | NM_001 049 | SSTR1 | Somatostatin receptor 1 | 23.91 | 29.84 |
| Hs.51 4451 | NM_001 050 | SSTR2 | Somatostatin receptor 2 | 32.93 | 32.08 |
| Hs.22 5995 | NM_001 051 | SSTR3 | Somatostatin receptor 3 | 33.57 | 33.23 |
| Hs.67 3846 | NM_001 052 | SSTR4 | Somatostatin receptor 4 | 31.45 | 31.61 |
| Hs.44 9840 | NM_001 053 | SSTR5 | Somatostatin receptor 5 | 40 | 37.35 |
| Hs.27 9575 | NM_033 050 | SUCN R1 | Succinate receptor 1 | 34.48 | 34.88 |
| Hs.37 5030 | NM_138 327 | TAAR1 | Trace amine associated receptor 1 | 35.07 | 34.03 |
| Hs.27 2382 | NM_014 626 | TAAR2 | Trace amine associated receptor 2 | 36.98 | 40 |
| Hs.24 8198 | NM_003 967 | TAAR5 | Trace amine associated receptor 5 | 27.07 | 30.8 |
| Hs.43 4196 | NM_175 067 | TAAR6 | Trace amine associated receptor 6 | 33.81 | 33.85 |
| Hs.43 4116 | NM_175 057 | TAAR9 | Trace amine associated receptor 9 (gene/pseudogene) | 35.81 | 40 |
| Hs.63 3301 | NM_001 058 | TACR1 | Tachykinin receptor 1 | 28.18 | 31.06 |
| Hs.88 372 | NM_001 057 | TACR2 | Tachykinin receptor 2 | 28.21 | 29.21 |
| Hs.94 2 | NM_001 059 | TACR3 | Tachykinin receptor 3 | 31.34 | 31.62 |
| Hs.44 2530 | NM_001 060 | TBXA2 R | Thromboxane A2 receptor | 29.62 | 31.6 |
| Hs.65 6790 | NM_032 027 | TM2D1 | TM2 domain containing 1 | 24.13 | 23.97 |
| Hs.30 22 | NM_003 301 | TRHR | Thyrotropin-releasing hormone receptor | 32.54 | 32.8 |
| Hs. 16 0411 | NM_000 369 | TSHR | Thyroid stimulating hormone receptor | 34.52 | 35.82 |
| Hs. 19 2720 | NM_018 949 | UTS2R | Urotensin 2 receptor | 32.55 | 31.77 |
| Hs.34 8500 | NM_004 624 | VIPR1 | Vasoactive intestinal peptide receptor 1 | 34.58 | 26.91 |
| Hs.58 5052 | NM_003 382 | VIPR2 | Vasoactive intestinal peptide receptor 2 (*) | 35.05 | 33.28 |
| Hs.24 8116 | NM_005 283 | XCR1 | Chemokine (C motif) receptor 1 | 35.03 | 32.98 |
| Hs.22 7656 | NM_004 736 | XPR1 | Xenotropic and polytropic retrovirus receptor 1 | 23.47 | 22.49 |
| Hs.59 2355 | NM_002 046 | GAPD H | Glyceraldehyde-3-phosphate dehydrogenase | 17.39 | 17.41 |

For all other in vitro experiments, cells were treated as indicated prior to RNA isolation using RNeasy Plus Mini Kit (Qiagen) according to manufacturer's instructions. Isolated RNA (250 ng) was then used to synthesize cDNA using SuperScript VILO (Invitrogen). Quantitative PCR was performed using FastStart Essential DNA Green Master (Roche) and analyzed using a LightCycler 96 (Roche). Data are expressed as a fold change by ΔΔCt relative to GAPDH. For in vivo experiments, tissue was immediately frozen, and stored at -80 °C. RNA isolation, cDNA synthesis, and qPCR analysis were performed as above. Primers used for qPCR are shown in Table 2.

**Table 2**

| **Human Primer** | **Sequence** | |
|---|---|---|
| *DRD1* | F: CCCAGCCCTATCAGTCATATTG, R: AGGATTCATCTGCGAGTTCAG | SEQ ID NOs:1 and 2 |
| *CTGF* | F: GTCCAGCACGAGGCTCA, R: TCGCCTTCGTGGTCCTC | SEQ ID NOs:3 and 4 |
| *COL1A1* | F: AAGGGACACAGAGGTTTCAGTGG, R: CAGCACCAGTAGCACCATCATTTC | SEQ ID NOs:5 and 6 |
| *ACTA2* | F: GTGAAGAAGAGGACAGCACTG, R: CCCATTCCCACCATC ACC | SEQ ID NOs:7 and 8 |
| *FN1* | F: TGTCAGTCAAAGCAAGCCCG, R: TTAGGACGCTCATAAGTGTCACCC | SEQ ID NOs:9 and 10 |
| *TGM2* | F: TCAGCTACAATGGGATCTTGG, R: AAGGCAGTCACGGTATTTCTC | SEQ ID NOs:11 and 12 |
| *LOX* | F: ACATTCGCTACACAGGACATC, R: TTCCCACTTCAGAACACCAG | SEQ ID NOs:13 and 14 |
| *LOXL1* | F: TGCCAGTGGATCGACATAAC, R: GAAACGTAGCGACCTGTGTAG | SEQ ID NOs:15 and 16 |
| *LOXL2* | F: GTGCAGCGACAAAAGGATTC, R: GCGGTAGGTTGAGAGGATG | SEQ ID NOs:17 and 18 |
| *LOXL3* | F: AGCGAAAAGAGGGTCAACG, R: TGTCATTGGCACGATAGAACTC | SEQ ID NOs:19 and 20 |
| *LOXL4* | F: GTGGCAGAGTCAGATTTCTCC, R: TTGTTCCTGAGACGCTGTTC | SEQ ID NOs:21 and 22 |
| *PLAU* | F: GGGAGATGAAGTTTGAGGTGG, R: AGATGGTCTGTATAGTCCGGG | SEQ ID NOs:23 and 24 |
| *PLAT* | F: AAACCCAGATCGAGACTCAAAG, R: ACCCATTCCCAAAGTAGCAG | SEQ ID NOs:25 and 26 |
| *CTSK* | F: CTCCTTCCAGTTTTACAGCAAAG, R: TTTCCCCAGTTTTCTCCCC | SEQ ID NOs:27 and 28 |
| *MMP14* | F: TGCCTACCGACAAGATTGATG, R: ATCCCTTCCCAGACTTTGATG | SEQ ID NOs:29 and 30 |

| Mouse Primer | Sequence | |
|---|---|---|
| *Drd1* | F: CCCGTAGCCATTATGATCGTC, R: AGAGCATTCGACAGGGTTTC | SEQ ID NOs:31 and 32 |
| *Pdgfra* | F: TCCTTCTACCACCTCAGCGAG, R: CCGGATGGTCACTCT TTAGGAAG | SEQ ID NOs:33 and 34 |
| *Epcam* | F: TTGCTCCAAACTGGCGTCTA, R: ACGTGATCTCCGTGTCCTTGT | SEQ ID NOs:35 and 36 |
| *Pecam1* | F: CTGCCAGTCCGAAAATGGAAC, R: CTTCATCCACTGGGGCTATC | SEQ ID NOs:37 and 38 |
| *Ptprc* | F: GACAGAGTTAGTGAATGGAGACC, R: AAAAGTTCGGAGAGTGTAGGC | SEQ ID NOs:39 and 40 |
| *Acta2* | F: GAGAAGCCCAGCCAGTCG, R: CTCTTGCTCTGGGCTTCA | SEQ ID NOs:41 and 42 |
| *Ctgf* | F: CCTGCGACCCACACAAG, R: GACCCACCGAAGACACAG | SEQ ID NOs:43 and 44 |
| *Fn1* | F: CCAGCAGCATGATCAAAACAC, R: GGTGGCTACATGTTAGAGTGTC | SEQ ID NOs:45 and 46 |
| *Col1a1* | F: ATCATAGCCATAGGACATCTGG, R: CTGGACAGCCTGGACTTC | SEQ ID NOs:47 and 48 |
| *Yap1* | F: CTCTGAGTGATCCTCTGGTTC, R: CCATAAGAACAAGACCACATCCT | SEQ ID NOs:49 and 50 |
| *WWtr1* | F: CTTGCTGGTGTTGGTGATTC, R: ATCAGCCTCTGAATCATGTGAA | SEQ ID NOs:51 and 52 |
| *Alb* | F: TGCTTTTCCAGGGTGTGTT, R: TTACTTCCTGCACTAATTTGGCA | SEQ ID NOs:53 and 54 |

*Cell Sorting:* FACS: PBS perfused mouse lungs were finely minced with a razor blade in a 100 mm petri dish in 1 mL of cold DMEM medium containing 0.2 mg ml⁻¹ Liberase DL (Roche) and 100 U ml⁻¹ DNase I (Roche). The mixture was transferred to 15 ml tubes and incubated at 37 °C for 30 min in a water bath. Enzymatic digestion was inactivated by adding DMEM medium containing 10% fetal bovine serum. The cell suspension was passed once through a 40 µm cell strainer (Fisher) to remove multicellular debris. Cells were then centrifuged at 1,300 r.p.m. at 4 °C for 10 min, washed once in PBS and resuspended in 0.2 ml of FACS buffer (1% BSA, 0.5 µM EDTA pH 7.4 in PBS). The single cell suspension was then incubated with anti-CD45-PerCp-Cy5.5, anti-CD31-PE, anti-PDGFRα-APC and anti-EpCAM-BV421 antibodies (1:200) (Biolegend) for 20 min on ice. After incubation, cells were washed with ice-cold FACS buffer and resuspended in 1 ml of FACS buffer. FACS sorting was conducted using a BD FACS Aria II (BD Biosciences). FACS-sorted epithelial cells, endothelial cells and fibroblasts were collected in 1.5 ml Eppendorf tubes containing RLT lysis buffer (Qiagen), which were subjected to mRNA extraction, complementary DNA synthesis and RT-PCR analysis. MACS: PBS perfused mouse lungs were finely minced with a razor blade in a 100 mm petri dish in 1 ml of MACs dissociation solution described in the MACS mouse lung dissociation kit. The mixture was transferred to 15 ml tubes and incubated at 37 °C for 30 min in a water bath. Enzymatic digestion was inactivated by adding DMEM containing 10% fetal bovine serum. The cell suspension was passed once through a 40 µm cell strainer (Fisher) to remove multicellular debris. Cells were then centrifuged at 1,350 r.p.m. at 4 °C for 10 min and supernatant aspirated. The samples were resuspended in 0.1ml 15% BSA-autoMACS rinsing solution. The single cell suspension was than incubated with mouse anti-CD45 MicroBeads (1:10) for 15 minutes at 4-8 °C. Cells were then magnetically filtered using LS column (Miltenyi Biotec). Positively selected cells were pelleted at 1350 r.p.m at 4 °C and resuspended in RLT lysis buffer (Qiagen). Samples were then subjected to mRNA extraction, complementary DNA synthesis and RT-PCR analysis.

*Immunofluorescence Microscopy:* Cells were plated into 96 well plates (Corning 3603) in their specific growth media and allowed to attach (8 hours). Media was then exchanged for the indicated conditions for each experiment. Cells were fixed in 3.7% formalin (Sigma-Aldrich), permeabilized in 0.25% Triton X-100 (Sigma-Aldrich) and then blocked with 1% BSA for 1 h. Cells or tissue sections were incubated overnight with mouse monoclonal antibody against αSMA (Sigma-Aldrich F3777), and/or a rabbit monoclonal antibody against YAP/TAZ (Cell Signaling D24E4) diluted 1:200 in PBS with 1% BSA. Cells were then washed and exposed to flourescence-conjugated secondary antibodies (Invitrogen) diluted 1: 1000 and DAPI (Thermo Fisher Scientific). Images were taken with a Cytation5 (BioTek) microscope. For scoring αSMA positive cells (Fig.3b), an observer blinded to the treatment conditions counted αSMA-positive cells using a visual threshold for bright fibrous staining; a minimum of 200 cells was counted for each condition. YAP/TAZ localization was quantified (Fig. 2, 6, 8) using Gen5 (Biotek) software. Images were taken at 4X magnification of both DAPI and YAP/TAZ staining. Objects were identified using the DAPI channel and a subpopulation of YAP/TAZ nuclear positive cells was counted based on nuclei where the average pixel intensity of the YAP/TAZ channel was greater than 85% of the average pixel intensity of all the nuclei in the control treated cells. Quantification of double positive (YAP/TAZ and αSMA) cells from lung tissue sections (Fig. 4d) was performed similarly; however separate thresholds were established for both YAP/TAZ and αSMA. A minimum of 4000 cells was quantified for each mouse.

*Traction Force Microscopy:* Traction analysis was conducted as previously described. Briefly, polyacrylamide substrates with shear moduli of 6.4 kPa were prepared, and fluorescent sulfate-modified latex microspheres (0.2 µm, 505/515 ex/em) (FluoSpheres, Life Technologies) were conjugated to the gel surfaces after treatment with 1 mg ml⁻¹ of dopamine hydrochloride (Sigma-Aldrich) in 50 mM HEPES solution (pH 8.5). IPF patient derived fibroblasts were plated on the gels overnight and treated as indicated before traction force measurements. Images of gel surface-conjugated fluorescent beads were acquired for each cell before and after trypsinization using a Nikon ECLIPSE Ti microscope at × 10 magnification. Traction forces were estimated by measuring bead displacement fields and computing corresponding traction fields using TractionsForAll (freely distributed program that calculates 2-D tractions exerted by an adherent cell on its substrate).

*cAMP Assay:* IPF patient derived fibroblasts were plated in EMEM containing 10% FBS overnight. Media was exchanged with EMEM containing 0.1% FBS for 24 hours. cAMP was measured using the cAMP-GloTM Assay (Promega) according to manufacturer's suggestions. 20 minutes prior to cell lysis media was removed and cells were treated with "induction buffer" containing nonselective phosphodiesterase inhibitors and the indicated concentration of compound(s). Luminescence was measured on a Flexstation 3 (Molecular Devices) plate reader.

*Western Blotting:* Cells were plated in EMEM containing 10% FBS overnight. Media was exchanged with EMEM containing 0.1% FBS for 24 hours. Prior to protein isolation cells were treated with the indicated concentration of compounds for the indicated time. Total protein was isolated using RIPA buffer (pH 8.0) containing Pierce Phosphatase Inhibitor (Thermo) and Halt Protease Inhibitor Cocktail (Thermo). Lysate total protein concentration was determined using Pierce BCA Protein Assay Kit (Thermo) and samples were run on a 10% polyacrylamide gel. Blots were incubated overnight with primary antibodies: pYAP (Ser 127, Cell Signaling D9W21), YAP/TAZ (Cell Signaling D24E4), GAPDH (Cell Signaling 14C10), HSC70 (Santa Cruz sc-7298), αSMA (Abcam ab5694), and fibronectin (Santa Cruz sc-9068) diluted 1:1000 in Li-Cor Odyssey Blocking Buffer. Blots were washed with TBS-Tween before 60 minute incubation with IR-dye-conjugated secondary antibodies (Li-Cor) diluted 1:10,000. Plates were imaged via a Li-Cor OdysseyXL system with quantification performed via densitometry.

*Immuno-ECM:* Adapting from previously published methods, IPF patient-derived fibroblasts were plated to confluence in clear-bottom 96-well plates. After cells attached, the medium was swapped for EMEM containing 0.1% FBS ± 2 ng/mL TGF-β. After 48 hours the indicated concentration of DHX or DMSO control was added to each well and incubated for 24 hours. WST-1 viability reagent was added to each well (Sigma-Aldrich) and measured on a Flexstation 3 (Molecular Devices) plate reader. Cells were then fixed in 3.7% formalin (Sigma-Aldrich), and permeabilized in 0.25% Triton X-100 (Sigma-Aldrich). Wells were washed with tris-buffered saline (TBS) and blocked with Li-Cor Odyssey Blocking Buffer for 60 minutes before overnight incubation in a polyclonal rabbit antibody for fibronectin (Sigma sc-9068) or collagen I (Novus NB600-408) diluted 1:200 in blocking buffer. Wells were washed with TBS-Tween before 45 minute incubation with IR-dye-conjugated secondary antibody (Li-Cor #926-32211) diluted 1:400. Plates were imaged via a Li-Cor OdysseyXL system with quantification performed via densitometry. Data are expressed as IR intensity relative to WST-1 signal absorbance in order to account for any potential compound toxicity.

*Matrix Remodeling Measured by Atomic Force Microscopy:* NIH-3T3 cells were plated to confluence onto gelatin coated (Cell Biologics) AFM compatible tissue culture dishes (Willco) in DMEM containing 10% FBS. After cells attached overnight media was replaced with DMEM containing 2% FBS, 2 ng/mL TGFβ, and 20 µg/mL ascorbic acid to promote matrix deposition. After 72 hours, measurements were made using a BioScope Catalyst AFM (Bruker, MA, USA). Microindentations were performed using a 2.5 µm radius sphere-tipped probe (Novascan, IA, USA) with a spring constant determined at -100 pN nm⁻¹ by thermal fluctuation method. For each dish, 3 different areas were analyzed. Force curves were acquired with MIRO 2.0 (NanoScope 9.1; Bruker) at an indentation rate of 20 µm s⁻¹ and a ramp size of 10 µm on different points. 75 force curves were performed per cell dish (25 per area). The Young's modulus E was determined by the fitting of force curve by Hertz model using NanoScope Analysis software (Bruker) and considering Poisson's ratio of 0.5. Media was exchanged for fresh DMEM containing 2% FBS, 2 ng/mL TGFβ, and 20 µg/mL ascorbic acid with the indicated concentration of DHX or 0.1% DMSO (vehicle control). After another 72 hours AFM measurements were made as before. The resulting cell-derived matrix was then decellularized with Phosphate-buffered saline (Gibco) containing: 0.5% (v/v) Triton X-100 (Sigma-Aldrich) and 20 mM NH₄OH (LabChem Inc.). Matrices were washed 3X with PBS and then plated with low passage (P3), NHLFs for 24 hours prior to RNA isolation.

*RNA Interference:* Cells were transfected using Lipofectamine RNAiMAX (Life Technologies) with 25nM siGENOME siRNA SMARTpool (Dharmacon) targeting DRD1 (L-005477-00-0005) or a nontargeting SMARTpool (D-001810-10-05). Cells were cultured for 72 hours before collecting RNA. For the YAP/TAZ localization experiments, the cells were transfected in their 6-well plates for 48 hours prior to re-plating into 96-well plates for the immunofluorescence assays. See below for in vivo siRNA methodology.

*Bleomycin Mouse Study:* In the initial in vivo siRNA study (Fig. 5), adult male age-matched C57BL/6N mice at 6-8 weeks of age were purchased from the National Cancer Institute (NCI)-Frederick Mouse Repository (Frederick, MD, USA). All experiments were performed in accordance with National Institute of Health guidelines and protocols approved by the Massachusetts General Hospital Subcommittee on Research Animal Care, and maintained all mice in a specific pathogen-free (SPF) environment certified by the American Association for Accreditation of Laboratory Animal Care (AAALAC). 6-8 weeks old mice were anesthetized with ketamine and xylazine before exposure of the trachea. Lung fibrosis was induced by intratracheal injection of bleomycin (50 µl at 1.2 U/kg) or phosphate buffered saline (PBS; as control) on day 0. After 14 days Small interfering RNA (siRNA) duplexes targeting mouse Yap (L-046247-01-0005) or Taz (L-058248-01-0005) mRNA (Dharmacon) or nontargeting control siRNA were administered in vivo by intratracheal instillation at a single dose of 25 µg (each siRNA) per mouse. On day 21 Mice were sacrificed and lungs harvested for collagen determination and biochemical analyses. To obtain BAL samples for total protein concentration determination, lungs were lavaged with six 0.5-ml aliquots of PBS. BAL samples were centrifuged at 3,000g for 20 min at 4 °C and transferred the supernatants to siliconized low-binding Eppendorf tubes (PGC Scientifics) for subsequent analysis. Total protein concentration of the BAL fluid was determined by BCA Protein Assay Kit (Pierce). In the dihydrexidine treatment studies (Fig. 4), 8 week old female C57/BL6 mice were purchased from Charles River Laboratories. Mouse lung fibrosis was induced with bleomycin (BLEO; Fresenius Kabi) delivered intratracheally (3 U/kg) to the lungs using MicroSprayer^{®} Aerosolizer (Penn-Century). The Sham mice received sterile 0.9% saline instead using identical methods. Mice were weighed every 24 hrs, and both groups were then randomized at day 10 into DHX and Control treatment groups, matching for the degree of weight change. DHX (5 mg/kg) was administered everyday intranasally (i.n.) dissolved in surfactant (infasurf) which has previously shown to aid in spreading to pulmonary aveoli for 14 days. The control groups of mice received the equivalent vehicle dose of surfactant. Following the final DHX treatment, mice were sacrificed and the right lungs were inflated with 4% paraformaldehyde (PFA) and further incubated in 4% PFA for 24 hours prior processing for paraffin embedding. The left lobe of the lung was snap frozen in liquid nitrogen for RNA isolation and hydroxyproline assay. Experimental procedures were approved by the Mayo Clinic Institutional Animal Care and Use Committee and the animals were handled in accordance with their guidelines.

*Bile Duct Ligation:* BDL was performed as previously described. Briefly, 8-10 weak old female C57BL/6N underwent either BDL or sham surgery. Mice were anesthetized on Day 0 following IACUC protocol, and the bile duct was ligated using sterile 3/0 silk ligatures. Sham surgery was performed by passing a silk ligature under the bile duct. Starting on Day 7, DHX (5 mg/kg) or vehicle control was administered everyday intraperitoneally (i.p.) for 14 days. Following the final DHX treatment, mice were sacrificed and the livers harvested for analysis of fibrosis.

*Histological Scoring:* Five µm thick sections were cut from Paraffin embedded lung tissues, and the sections were stained either with hematoxylin and eosin (H&E) or with Masson's Trichrome stain kit (Abcam). All H&E-stained slides and trichrome-stained slides were reviewed in a blinded fashion by a thoracic pathologist. The severity of interstitial and peribronchiolar lung immature and mature fibrosis was estimated on a numerical scale according to Ashcroft et al. For scoring purposes, all H&E stained slides were systematically scanned at 100x magnification and successive 100x fields were scored. Scoring was based on the following scale: 0 (no fibrosis), 1 (minimal interstitial and/or peribronchiolar thickening due to fibrosis), 3 (moderate thickening without obvious architectural distortion), 5 (increased fibrosis with formation of fibrous bands and/or small masses), 7 (severe architectural distortion with large areas of fibrosis and areas of honeycomb changes), and 8 (total fibrous obliteration of the field). The predominant score for each field was recorded. The mean of all scores was calculated for each case. Liver trichrome stained sections were computationally measured using Image J software. After converting each image in an RGB stack, the threshold was adjusted and kept at the same level for all the images.

*Hydroxyproline:* Hydroxyproline content was measured using a hydroxyproline assay kit (Biovision) according to the manufacture's instruction with slight modification. The lung tissues were weighed, homogenized in sterile water (10 mg of tissue per 100 µl H₂O) and hydrolyzed in 12N HCl in a pressure-tight, teflon capped vial at 120 °C for 3 hours followed by filtration through a 45 µm Spin-X^{®} Centrifuge Tube filter (Corning). Ten µl of the hydrolyzed samples was dried in a Speed-Vac for 2hours, followed by incubation with 100 µl of Chloramine T reagent for 5 minutes at room temperature and 100 µl of 4-(Dimethylamino) benzaldehyde (DMAB) for 90 minutes at 60 °C. The absorbance of oxidized hydroxyproline was determined at 560 nm. Hyrdroxyproline concentrations were calculated from a standard curve generated using known concentrations of trans-4-hydroxyl-L-proline. The total amount of protein isolated from the weighed tissues was determined by using a protein assay kit (Bio-Rad, absorbance at 595 nm). The amount of collagen was expressed in µg/mg total protein.

*Statistics:* Groups were compared by one-way ANOVA with Tukey's multiple comparison's test. All statistical tests were carried out using GraphPad Prism 6 with statistical significance defined as p < 0.05. Results are expressed throughout as the mean ± standard error of the mean (SEM).

### Example 1 - Selective YAP and TAZ targeting by agonizing Gαₛ receptors

To test whether nonselective YAP and TAZ targeting may be effective in a model of pulmonary fibrosis, YAP and TAZ siRNA were administered intratracheally to mice following bleomycin injury, a standard model of pulmonary fibrosis (Figure 5). Non-selective targeting of YAP/TAZ in this context amplified fibrosis (measured by hydroxyproline assay), while also increasing lung injury and vascular leakage. Contrastingly, fibroblast selective genetic deletion of YAP and TAZ has shown promise in a kidney fibrosis model²⁵.

G protein-coupled receptors (GPCRs) make up the largest family of membrane receptors in the human genome, and have been prolific therapeutic targets, with their ligands account for >30% of all clinically approved drugs²⁶. GPCRs are linked to effector proteins from four main classes of G-proteins. Activation of receptors which couple to Gα_{12/13}, Gα_{q/11} and Gα_{i/o} stimulates YAP/TAZ nuclear translocation and transcriptional activity. In contrast, receptors which couple to Gαₛ inhibit YAP/TAZ nuclear localization and activity via elevation of cAMP²⁷⁻³⁰ (Fig. 1a).

GPCR expression varies across organs and even within adjacent cell types in the same tissue³¹. Therefore, RNA expression of the GPCRome was profiled in both primary adult human pulmonary fibroblasts and alveolar epithelial cells (Fig. 1b), searching for receptors which exclusively couple to Gαₛ³² (highlighted in red, Fig. 1b) and are expressed selectively in fibroblasts. Of the 28 Gαₛ coupled receptors, expression of the Dopamine Receptor D1 (*DRD1*) exhibited relatively high expression and pronounced enrichment in fibroblasts compared to alveolar epithelial cells (Fig. 1b). Abundant transcripts for *DRD1* in cultured normal human lung fibroblasts and fibroblasts derived from patients with idiopathic pulmonary fibrosis was determined by qPCR, and undetectable transcript levels of *DRD1* in both primary human alveolar epithelial and microvasculature endothelial cells (Fig. 1c). To further validate our findings in freshly isolated lung cell populations, we sorted mouse lung tissue into mesenchymal, epithelial, endothelial, and leukocyte enriched fractions (Fig. 1d). As in cultured human cells we observed robust expression of *DRD1* in the freshly isolated mesenchymal cells, but undetectable levels in other lung cell populations.

### Example 2 - DRD1 agonists selectively inhibit YAP and TAZ localization in mesenchymal cells

Three selective DRD1 agonists (Dihydrexidine, SKF-81297, Fenoldopam) were tested for their ability to inhibit YAP/TAZ nuclear localization (Fig. 2a, 6). Fibroblasts plated on stiff matrix (plastic) and lacking cell contact inhibition have abundant nuclear localization of YAP/TAZ². All three compounds reduced nuclear localization of YAP/TAZ, and their efficacy was consistent with previously described intrinsic activity of these ligands³³.

The inhibition of YAP/TAZ nuclear localization by dihydrexidine (DHX) could be attenuated using a DRD1 selective antagonist (Fig. 2a) or by treating the cells with *DRD1*-siRNA (Fig. 7), confirming the receptor-specific effects of DHX. Consistent with the previously defined mechanism whereby YAP/TAZ nuclear localization is controlled by cAMP-dependent phosphorylation of serine residues, promoting cytoplasmic retention or degradation, DHX elevated cAMP and promoted YAP serine 127 phosphorylation²⁷⁻³⁰ (Fig. 2d,e). DHX was effective at inhibiting YAP/TAZ nuclear localization across a panel of mesenchymal cell types, including cardiac and dermal fibroblasts and hepatic stellate cells (Fig. 6), suggesting potentially broad relevance of this ligand for mesenchymal cell targeting of YAP and TAZ. DHX-mediated inhibition of YAP/TAZ nuclear localization was relatively sustained, and equally potent in normal lung fibroblasts and those derived from a patient with IPF, unlike the reduced potency of another GPCR ligand (PGE₂) with known anti-fibrotic effects in the lung (Fig. 6)³⁴. In contrast to these observations, DHX had no effect on YAP/TAZ localization in pulmonary epithelial and endothelial cells (Fig. 2b), consistent with the absence of detectable transcripts for *DRD1* in these cell types. Multiple GPCR ligands which are known to promote fibrosis couple to Gαᵢ, Gα_{q}, and Gα₁₂ which would in turn be expected to enhance YAP/TAZ nuclear localization in fibroblasts^{28,33}. It was confirmed in confluent fibroblasts, which otherwise exhibit reduced nuclear localization of YAP/TAZ, showing that endothelin-1, lysophosphatidic acid and serotonin, all GPCR ligands implicated in promotion of fibrosis³⁵, enhance YAP/TAZ nuclear localization. DHX blocked nuclear localization of YAP/TAZ in responses to all three of these ligands, demonstrating the broad effects of GPCR ligands on YAP/TAZ in fibroblasts, and identifying DHX and stimulation of Gαₛ/cAMP as an effective strategy for inhibiting both mechanical (stiff matrix) and biochemical regulation of YAP/TAZ nuclear localization (Fig. 2c). The ability of selected dopamine receptor agonists to inhibit YAP/TAZ nuclear localization is shown in Table 3.

**Table 3**

| | Nuclear localization (% of total cells) | | | |
|---|---|---|---|---|
| **Compound** | **IPF-1** | **IPF-2** | **IPF-3** | **IPF-4** |
| DMSO | 89.96719 | 77.08034 | 76.3871 | 73.26695 |
| R(-)-2,10,11-Trihydroxyaporphine HBr | 13.70637 | 22.14634 | 24.15385 | 17.75309 |
| Dihydrexidine | 13.22484 | 21.79444 | 27.63449 | 21.08058 |
| A 68930·HCl | 33.12535 | 30.59259 | 26.82353 | 10.25995 |
| (R)-(-)-Apomorphine·HCl | 29.20735 | 31.96825 | 27.47368 | 17.86425 |
| (±)-SKF-82958·HBr | 29.15385 | 38 | 38 | 12.28571 |
| CY 208-243 | 20.51748 | 39.79487 | 41.17073 | 19.81818 |
| R(-)-Propylnorapomorphine·HCl | 28.34884 | 26.70968 | 42.73684 | 29.03004 |
| R(+)-6-BROMO-APB'HBr | 28.36697 | 38 | 36 | 25.64706 |
| R(-)-2,10,11-Trihydroxy-N-propyl-noraporphine·HBr | 32.86874 | 35.25275 | 37.69697 | 25.35849 |
| A-77636·HCl·H₂O | 26.46154 | 30.88136 | 41.33333 | 37.78903 |
| Dopamine·HCl | 39.84783 | 59.28571 | 49.66667 | 49.50943 |
| 6,7-ADTN·HBr | 43.88865 | 44 | 65.5 | 52.64912 |
| Mesulergine·HCl | 37.81413 | 45.14286 | 78.625 | 45.14286 |
| SKF 38393·HBr | 69.5873 | 47.375 | 61.33333 | 35.79116 |
| N-Methyldopamine·HCl | 43.15829 | 46.73016 | 60.10526 | 70.44275 |
| 4-Hydroxyphenethylamine·HCl | 56.51852 | 45.69231 | 76.57143 | 43.3719 |
| Cabergoline | 57.01515 | 57.41176 | 45.95181 | 68.69498 |
| 3-Hydroxyphenethylamine·HCl | 48.95506 | 56.91892 | 69.25 | 58.37466 |
| Pramipexole Dihydrochloride Monohydrate | 64.92308 | 47.64912 | 65.5 | 58.96774 |
| PD 168077 maleate | 67.07869 | 42.44444 | 63.67568 | 68.4 |
| Fenoldopam·HCl | 68.09828 | 50.31884 | 66.63636 | 63 |
| (±)-PD 128,907·HCl | 60.13115 | 49.53846 | 78.47619 | 60.66187 |
| (±)-2-(N-phenylethyl-N-propyl)amino-5-hydroxytetralin·HCl | 68.85 | 60.85714 | 68 | 56.552 |
| Bromocriptine mesylate | 73.02879 | 52.81481 | 64.08696 | 64.59574 |
| Ropinirole HCl | 69.6036 | 58.68421 | 64.66099 | 65.69517 |
| LY-163,502·2HCl | 73.37074 | 60.97297 | 59.42857 | 66.48606 |
| Dipropyldopamine·HBr | 69.17914 | 74.36364 | 65.5 | 51.82253 |
| B-HT 920·2HCl | 75.67606 | 46.90411 | 64.78571 | 76.23529 |
| Piribedil·2HCl | 61.58491 | 85.76119 | 59.05263 | 59.63793 |
| (+)-UH 232 maleate | 61.9521 | 68 | 71.84615 | 67.26608 |
| Pergolide mesylate | 82.48669 | 69.37255 | 70.66667 | 65.57576 |
| (-)-Quinpirole·HCl | 80.11538 | 73.14851 | 80.53731 | 68.54475 |
| R(-)-2,11-dihydroxy-10-methoxyapomorphine·HCl | 85.18283 | 74.66667 | 80.72727 | 80.45283 |

The ability of selected dopamine receptor agonists to inhibit expression of αSMA is shown in Table 4.

**Table 4**

| | αSMA intensity (% of DMSO control) | | | |
|---|---|---|---|---|
| **Compound** | **IPF-1** | **IPF-2** | **IPF-3** | **IPF-4** |
| DMSO | 100 | 100 | 100 | 100 |
| Dihydrexidine·HCl | 4.984838 | 0.04693 | 0.513048 | 12.03436 |
| A-77636·HCl·H₂O | 5.808214 | 0.71885 | 0.067655 | 12.42941 |
| R(-)-2,10,11-Trihydroxyaporphine HBr | 2.887936 | 1.698043 | 7.646467 | 15.9942 |
| (±)-SKF-82958·HBr | 11.77987 | 4.966244 | 2.427766 | 9.32639 |
| A 68930·HCl | 12.40274 | 8.110719 | 3.24542 | 7.350053 |
| R(-)-Propylnorapomorphine·HCl | 5.081187 | 1.878811 | 5.560538 | 19.95755 |
| CY 208-243 | 21.01121 | 2.279688 | 2.610795 | 9.642782 |
| R(+)-6-bromo-APB·HBr | 11.25082 | 2.507086 | 0.850571 | 22.81622 |
| R(-)-2,10,11-Trihydroxy-N-propyl-noraporphine·HBr | 4.894616 | 8.436081 | 2.988195 | 25.60956 |
| (R)-(-)-Apomorphine·HCl | 7.067375 | 1.172584 | 0.961841 | 33.67351 |
| 6,7-ADTN·HBr | 37.14071 | 4.182238 | 9.908606 | 4.887935 |
| Dopamine·HCl | 35.24978 | 8.085496 | 26.56503 | 5.368141 |
| N-Methyldopamine·HCl | 43.19963 | 17.66949 | 28.99207 | 14.65662 |
| SKF 38393·HBr | 37.06277 | 26.81876 | 31.24847 | 20.7973 |
| Mesulergine·HCl | 35.50719 | 23.27498 | 40.01858 | 19.75155 |
| Dipropyldopamine·HBr | 48.72246 | 28.11431 | 40.33584 | 25.85713 |
| Bromocriptine mesylate | 54.0785 | 53.62163 | 61.14226 | 34.0979 |
| Pergolide mesylate | 65.01308 | 51.5948 | 63.61159 | 48.1321 |
| (±)-2-(N-phenylethyl-N-propyl)amino-5-hydroxytetralin·HCl | 65.43466 | 52.77428 | 60.4844 | 51.10147 |
| Piribedil·2HCl | 70.43668 | 61.52793 | 71.47113 | 41.19743 |
| Cabergoline | 76.91545 | 48.07228 | 77.14592 | 45.8402 |
| Fenoldopam·HCl | 77.33538 | 45.27138 | 69.40569 | 63.35173 |
| B-HT 920·2HCl | 74.97842 | 58.97091 | 70.11813 | 54.23016 |
| Ropinirole HCl | 72.18286 | 53.45179 | 78.99604 | 61.9799 |
| PD 168077 maleate | 75.26704 | 56.14917 | 75.76791 | 66.49739 |
| (+)-UH 232 maleate | 82.91486 | 63.85248 | 85.81355 | 65.71164 |
| LY-163,502·2HCl | 88.98983 | 72.85814 | 75.30018 | 86.7894 |
| (-)-Quinpirole·HCl | 84.60746 | 67.1295 | 85.40936 | 95.1307 |
| Pramipexole Dihydrochloride Monohydrate | 87.61601 | 73.21711 | 82.7588 | 89.93775 |
| (±)-PD 128,907·HCl | 83.67743 | 85.45062 | 94.2538 | 75.15364 |
| R(-)-2,11-dihydroxy-10-methoxyapomorphine·HCl | 91.84016 | 92.79975 | 81.85245 | 85.60691 |
| 3-Hydroxyphenethylamine·HCl | 88.58294 | 96.744 | 82.62473 | 99.8315 |
| 4-Hydroxyphenethylamine·HCl | 99.7156 | 93.43022 | 95.69317 | 88.2748 |

### Example 3 - DHX reduces fibroblast activation and matrix deposition

To test whether DHX-mediated inhibition of YAP/TAZ nuclear localization translates into altered mesenchymal cell activation, we first demonstrated that expression of hallmark profibrotic genes *CTGF, COL1A1, ACTA2,* and *FN1* was reduced in IPF patient-derived lung fibroblasts by DHX treatment, recapitulating effects of YAP/TAZ knockdown¹⁻⁴ (Fig. 3a). These effects could be blocked with a DRD1 antagonist (Fig. 3a) as well as DRD1-siRNA (Fig. 7). Stimulating fibroblasts cultured on stiff tissue culture plastic for 72 hours with TGFβ further enhances their myofibroblastic transition, as detected by αSMA+ stress fibers; treating fibroblasts with DHX from 48-72 hours in the presence of TGFP reversed this transition (Fig. 3b). Similarly, DHX dose-dependently reversed fibroblast-mediated, TGFβ-stimulated accumulation of collagen I and fibronectin (Fig. 3c). To validate that this effect is dependent on inhibition of YAP/TAZ, NIH-3T3 cells were employed which stably express a doxycycline-inducible, constitutively active, mutant TAZ (TAZ4SA)^{2,36}. In these cells, DHX had no effect on profibrotic gene expression or extracellular matrix accumulation (Fig. 8). Finally, traction force microscopy (TFM) demonstrated that DHX significantly and dose-dependently reduced the contractile forces generated by fibroblasts (Fig. 3D).

### Example 4 - Extracellular matrix remodeling by DHX

The results above were consistent with DHX not only attenuating key aspects of fibroblast pro-fibrotic activation, but potentially shifting their phenotype toward one that promotes fibrosis clearance and resolution. To test this concept directly, an in vitro matrix remodeling assay was developed. Fibroblasts (NIH-3T3 cells) were first plated at confluence (**A** and **B** in Fig. 3e), following an approach developed for studying cell-derived matrices. Cells on both plates were cultured with TGFβ and ascorbic acid to promote matrix synthesis and deposition. After 72 hours the stiffness of the cells and their cell-derived matrix were measured using atomic force microscopy (AFM). To test the ability of DHX to induce cell-mediated matrix remodeling, TGFβ and ascorbic acid were maintained but also DHX was added to **B** (vehicle control was added to **A**) for an additional 72 hours before probing the matrix again with AFM. While the cells and matrix in control plate **A** continued to stiffen over time, DHX treatment effectively reversed this trend and significantly reduced the observed stiffness. To confirm that DHX introduced a matrix remodeling effect, the matrices were decellularized and plated low passage primary human adult lung fibroblasts for 24 hours onto the decellularized matrices, then RNA was isolated to measure changes in profibrotic gene expression. *CTGF*, *COL1A1*, *ACTA2* and *FN1* expression were all decreased in the cells plated onto the matrix which had previously been treated with DHX, identifying a pharmaco-footprint left behind in the extracellular matrix by cellular remodeling.

Based on these matrix remodeling effects of DHX, it was determined whether efficacy of this pathway extends to inducing matrix degradation/remodeling action in fibroblasts which could reverse the disease rather than simply slow its progression (an important utility of fibroblasts). First, the effect DHX was investigated on expression of genes associated with matrix remodeling (Fig. 3f). IPF patient derived fibroblasts treated with TGFβ showed enhanced expression of matrix crosslinking genes and inhibitors of matrix protease enzymes but also showed reduced expression of several genes associated with matrix clearance (Fig. 3f). In each case, DHX treatment reversed the effects of TGFβ, reducing expression of crosslinking and protease inhibitors but enhancing expression of matrix degradation associated enzymes. There doesn't appear to be a single driver gene by which DHX effects matrix remodeling, suggesting that the observed effect is a broader fibroblast program shift.

### Example 5 - DHX efficacy in vivo

Bleomycin model of pulmonary fibrosis was used to test the efficacy of DHX *in vivo* in experimental fibrosis. Mice were administered bleomycin intratracheally at Day 0. On Day 10 injury and inflammation typically subside and fibrosis is ongoing. At Day 10 mice were randomized into two groups, one receiving DHX (5 mg/kg once daily i.n.) and the other, vehicle control. The Bleo DHX group lost significantly less weight than the Bleo control group (Fig. 4a). At day 24 the mice were sacrificed and compared using histology, hydroxyproline and qPCR. Histologically, the Bleo DHX group was nearly completely protected from lung remodeling compared to the Bleo control group and sham uninjured mice, as assessed by a blinded pathologist (Fig. 4b). Total collagen in the lungs of Bleo DHX mice was nearly identical to sham treated mice, and significantly reduced compared to Bleo control mice (Fig. 4c). Bleomycin increased staining for YAP and TAZ in the lungs and this was attenuated by DHX treatment (Fig. 4d). The Bleo Control group also showed enhanced transcript levels for profibrotic genes *Acta2*, *Ctgf*, *Fn1*, *Col1a1*, and *Col1a2*, as well as *Yap* and *Taz* themselves, all of which were significantly attenuated in the Bleo DHX group (Fig. 4e). To assess whether DHX adversely effects lung remodeling in the absence of fibrosis, we also exposed control mice to DHX following an identical time course and route of exposure. The lungs of Sham DHX mice did not differ from those of control mice using any of these measurements (Fig. 9).

### Example 6 - Effect of DHX on hepatic stellate cells

Based on the efficacy of DHX in attenuating YAP/TAZ nuclear localization across an array of mesenchymal cells, we sought to extend our findings to hepatic stellate cells and liver fibrosis. Preferential expression of *DRD1* in hepatic stellate cells (HSCs) compared to hepatocytes (Fig. 10) was confirmed, with results similar to those obtained for lung tissue in the previous examples. Ability of DHX to reduce TGFβ-mediated HSC expression of SMA and FN protein was then tested by western blotting, and observed significant reversal of both. Efficacy of DHX in the bile duct ligation model of cholestatic injury and liver fibrosis was then tested. Bile duct ligation was performed at Day 0 and treatment with DHX or vehicle began at Day 7 and continued for 14 days. DHX significantly improved histological fibrosis caused by the BDL and exhibited a trend toward reduced collagen deposition (Fig. 10).

Previous work demonstrated that TAZ mediates fibrotic effects in hepatocytes in a model of non-alcoholic steatohepatitis³⁷, that verteporfin (an inhibitor of YAP/TAZ-TEAD interactions) has limited beneficial effects in models of liver fibrosis models¹⁹, and that YAP/TAZ are essential in liver regeneration (nonspecific YAP knockdown in liver promotes hepatocyte necrosis³⁸). Our results are the first to demonstrate the efficacy of a GPCR-based approach to selective inhibition of YAP/TAZ in experimental liver fibrosis.

### Example 7 - DOPA decarboxylase is decreased in IPF, and correlates with worsening disease severity

It was discovered that IPF patient lungs express less dopa decarboxylase (DDC) (enzyme that takes part in dopamine synthesis) than non-IPF lungs and the lower level of DDC expression correlates with decreased lung function consistent with an endogenous, protective role for dopamine signaling that is lost in pulmonary fibrosis (Fig. 33). In support of this, it was also shown that dopamine is antifibrotic in *in vitro* assessments of fibroblast activity (Fig. 34).

Taken together, the experimental results and data presented here demonstrate that GPCR agonism can be used to pharmacologically target YAP and TAZ in selective cell populations to exert beneficial effects on tissue fibrosis. Gaₛ agonism and YAP/TAZ inhibition reverses the matrix deposition and stiffening phenotype of activated fibroblasts toward a matrix remodeling phenotype that promotes fibrosis resolution, showing that Gaₛ agonism and YAP/TAZ inhibition is an valuable approach to treat patients with fibrotic diseases.

### REFERENCES

1 Tschumperlin, D. J., Liu, F. & Tager, A. M. Biomechanical regulation of mesenchymal cell function. Curr Opin Rheumatol 25, 92-100, doi: 10.1097 /BOROb013e32835b13cd (2013).
2 Liu, F. et al. Mechanosignaling through YAP and TAZ drives fibroblast activation and fibrosis. Am J Physiol-Lung C 308, L344-L357, doi: 1 0.1152/ajplung. 00300.2014 (2015).
3 Martin, K. et al. PAK proteins and YAP-1 signalling downstream of integrin beta-1 in myofibroblasts promote liver fibrosis. Nat Commun 7, 12502, doi:10.1038/ncomms12502 (2016).
4 Szeto, S. G. et al. YAP/TAZ Are Mechanoregulators of TGF-beta-Smad Signaling and Renal Fibrogenesis. JAm Soc Nephrol, doi:10.1681/ASN.2015050499 (2016).
5 Piersma, B., Bank, R. A. & Boersema, M. Signaling in Fibrosis: TGF-beta, WNT, and YAP/TAZ Converge. Front Med (Lausanne) 2, 59, doi:10.3389/fmed.2015.00059 (2015).
6 Hansen, C. G., Moroishi, T. & Guan, K. L. YAP and TAZ: a nexus for Hippo signaling and beyond. Trends Cell Biol 25, 499-513, doi:10.1016/j.tcb.2015.05.002 (2015).
7 Zanconato, F., Battilana, G., Cordenonsi, M. & Piccolo, S. YAP/TAZ as therapeutic targets in cancer. Curr Opin Pharmacol 29, 26-33, doi:10.1016/j.coph.2016.05:002 (2016).
8 Zhao, B., Li, L., Lei, Q. & Guan, K. L. The Hippo-YAP pathway in organ size control and tumorigenesis: an updated version. Genes Dev 24, 862-874, doi:24/9/862 [pii] 10.1101/gad.1909210 (2010).
9 Elbediwy, A., Vincent-Mistiaen, Z. I. & Thompson, B. J. YAP and TAZ in epithelial stem cells: A sensor for cell polarity, mechanical forces and tissue damage. Bioessays 38, 644-653, doi:10.1002/bies.201600037 (2016).
10 Lin, C. W. et al. YAP is essential for mechanical force production and epithelial cell proliferation during lung branching morphogenesis. Elife 6, doi:ARTN e21130 10.7554/eLife.21130 (2017).
11 Imajo, M., Ebisuya, M. & Nishida, E. Dual role of YAP and TAZ in renewal of the intestinal epithelium. Nature Cell Biology 17, 7-+, doi:10.1038/ncb3084 (2015).
12 Kim, J. et al. YAP/TAZ regulates sprouting angiogenesis and vascular barrier maturation. J Clin Invest 127, 3441-3461, doi:10.1172/JCI93825 (2017).
13 Wang, L. et al. Integrin-YAP/TAZ-JNK cascade mediates atheroprotective effect of unidirectional shear flow. Nature, doi:10.1038/nature20602 (2016).
14 Tang, Y. & Weiss, S. J. Snail/Slug-YAP/TAZ complexes cooperatively regulate mesenchymal stem cell function and bone formation. Cell Cycle 16, 399-405, doi:10.1080/15384101.2017.1280643 (2017).
15 Levasseur, A., St-Jean, G., Paquet, M., Boerboom, D. & Boyer, A. Targeted disruption of YAP and TAZ impairs the maintenance of the adrenal cortex. Endocrinology, doi:10.1210/en.2017-00098 (2017).
16 Totaro, A. et al. YAP/TAZ link cell mechanics to Notch signalling to control epidermal stem cell fate. Nat Commun 8, 15206, doi:10.1038/ncomms15206 (2017).
17 Grove, M. et al. YAP/TAZ initiate and maintain Schwann cell myelination. Elife 6, doi:10.7554/eLife.20982 (2017).
18 Elbediwy, A. et al. Integrin signalling regulates YAP and TAZ to control skin homeostasis. Development 143, 1674-1687, doi:10.1242/dev.133728 (2016).
19 Martin, K. et al. PAK proteins and YAP-1 signalling downstream of integrin beta-1 in myofibroblasts promote liver fibrosis. Nat Commun 7, 12502, doi:10.1038/ncomms12502 (2016).
20 Zhang, K. et al. omega-3 PUFAs ameliorate liver fibrosis and inhibit hepatic stellate cells proliferation and activation by promoting YAP/TAZ degradation. Sci Rep 6, 30029, doi: 10.1038/srep30029 (2016).
21 Perumal, N., Perumal, M., Halagowder, D. & Sivasithamparam, N. Morin attenuates diethylnitrosamine-induced rat liver fibrosis and hepatic stellate cell activation by co-ordinated regulation of Hippo/Yap and TGF-beta 1/Smad signaling. Biochimie 140, 10-19, doi:10.1016/j.biochi.2017.05.017 (2017).
22 Liang, M. et al. Yap/Taz Deletion in Gli+ Cell-Derived Myofibroblasts Attenuates Fibrosis. J Am Soc Nephrol, doi:10.1681/ASN.2015121354 (2017).
23 Miranda, M. Z. et al. TGF-beta1 regulates the expression and transcriptional activity of TAZ protein via a Smad3-independent, myocardin-related transcription factor-mediated mechanism. J Biol Chem 292, 14902-14920, doi:10.1074/jbc.M117.780502 (2017).
24 Szeto, S. G. et al. YAP/TAZ Are Mechanoregulators of TGF-beta-Smad Signaling and Renal Fibrogenesis. JAm Soc Nephrol 27, 3117-3128, doi:10.1681/ASN.2015050499 (2016).
25 Liang, M. et al. Yap/Taz Deletion in Gli(+) Cell-Derived Myofibroblasts Attenuates Fibrosis. J Am Soc Nephrol 28, 3278-3290, doi:10.1681/ASN.2015121354 (2017).
26 Hauser, A. S., Attwood, M. M., Rask-Andersen, M., Schioth, H. B. & Gloriam, D. E. Trends in GPCR drug discovery: new agents, targets and indications. Nat Rev Drug Discov, doi:10.1038/nrd.2017.178 (2017).
27 Zhou, X., Wang, Z., Huang, W. & Lei, Q. Y. G protein-coupled receptors: bridging the gap from the extracellular signals to the Hippo pathway. Acta Biochim Biophys Sin (Shanghai) 47, 10-15, doi:10.1093/abbs/gmu108 (2015).
28 Yu, F. X. et al. Regulation of the Hippo-YAP pathway by G-protein-coupled receptor signaling. Cell 150, 780-791, doi:10.1016/j.cell.2012.06.037 (2012).
29 Yu, F. X. et al. Protein kinase A activates the Hippo pathway to modulate cell proliferation and differentiation. Genes Dev 27, 1223-1232, doi:10.1101/gad.219402.113 (2013).
30 Kim, M. et al. cAMP/PKA signalling reinforces the LATS-YAP pathway to fully suppress YAP in response to actin cytoskeletal changes. Embo J 32, 1543-1555, doi:10.1038/emboj.2013.102 (2013).
31 Insel, P. A. et al. GPCR expression in tissues and cells: Are the optimal receptors being used as drug targets? Brit J Pharmacol 165, 1613-1616, doi:10.1111/j.1476-5381.2011.01434.x (2012).
32 Flock, T. et al. Selectivity determinants of GPCR-G-protein binding. Nature 545, 317-322, doi:10.1038/nature22070 (2017).
33 Southan, C. et al. The IUPHAR/BPS Guide to PHARMACOLOGY in 2016: towards curated quantitative interactions between 1300 protein targets and 6000 ligands. Nucleic Acids Res 44, D1054-D1068, doi:10.1093/nar/gkv1037 (2016).
34 Wilborn, J. et al. Cultured Lung Fibroblasts Isolated from Patients with Idiopathic Pulmonary Fibrosis Have a Diminished Capacity to Synthesize Prostaglandin E(2), and to Express Cyclooxygenase-2. Journal of Clinical Investigation 95, 1861-1868, doi:Doi 10.1172/Jci117866 (1995).
35 Snead, A. N., He, S. & Insel, P. A. G protein-coupled receptor (GPCR) regulation of cardiac fibrosis. Faseb J 26 (2012).
36 Jorgenson, A. J. et al. TAZ activation drives fibroblast spheroid growth, expression of profibrotic paracrine signals, and context-dependent ECM gene expression. Am J Physiol Cell Physiol 312, C277-C285, doi:10.1152/ajpcell.00205.2016 (2017).
37 Wang, X. B. et al. Hepatocyte TAZ/WWTR1 Promotes Inflammation and Fibrosis in Nonalcoholic Steatohepatitis. Cell Metab 24, 848-862, doi:10.1016/j.cmet.2016.09.016 (2016).
38 Bai, H. et al. Yes-associated protein regulates the hepatic response after bile duct ligation. Hepatology 56, 1097-1107, doi:10.1002/hep.25769 (2012).

## Claims

1. A composition comprising a Gαₛ protein coupled receptor agonist, or a pharmaceutically acceptable salt thereof for use in a method of treating or preventing a fibrotic pathology, the method comprising administering to a subject in need thereof a therapeutically effective amount of said composition,
wherein Gαₛ protein coupled receptor is preferentially expressed in mesenchymal cells as compared to epithelial or endothelial cells, and the agonist is specific for Gαₛ receptor that is expressed preferentially in the mesenchymal cell,
wherein the Gαₛ protein coupled receptor is a dopamine receptor, and
wherein the agonist agonizes both D1 and D5 dopamine receptors,
wherein the agonist is selected from dihydrexidine (DHX), SKF-81297, SKF-82958, SKF-38393, fenoldopam, 6-Br-APB, A-68930, A-77636, CY-208-243, pergolide, R(-)-2,10,11-trihydroxyaporphine, (R)-(-)-apomorphine, R(-)-propylnorapomorphine, R(+)-6-bromo-APB, R(-)-2,10,11-trihydroxy-N-propyl-noraporphine, 6,7-ADTN, mesulergine, N-methyldopamine, 4-hydroxyphenethylamine, 3-hydroxyphenethylamine, pramipexole, PD-168077, (±)-PD 128-907, (±)-2-(N-phenylethyl-N-propyl)amino-5-hydroxytetralin, bromocriptine, ropinirole, LY-163-502, dipropyldopamine, B-HT 920, piribedil, (+)-UH 232, (-)-quinpirole, R(-)-2,11-dihydroxy-10-methoxyapomorphine, or a pharmaceutically acceptable salt thereof,
or wherein the agonist is a compound of Formula (I): 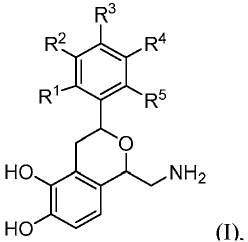 or a pharmaceutically acceptable salt thereof, wherein:
each of R¹, R², R³, R⁴ and R⁵ is independently selected from H, OH, C₁₋₆ alkoxy, C₁₋₆ haloalkoxy, HO-C₁₋₃ alkyl, NH₂-C₁₋₃ alkyl, HS-C₁₋₃ alkyl, SH, NH₂, C₁₋₆ alkylamino and di(C₁₋₆ alkyl)amino, optionally wherein at least one of R¹, R², R³, R⁴ and R⁵ is selected from OH, C₁₋₆ alkoxy, C₁₋₆ haloalkoxy, HO-C₁₋₃ alkyl, NH₂-C₁₋₃ alkyl, HS-C₁₋₃ alkyl, SH, NH₂, C₁₋₆ alkylamino and di(C₁₋₆ alkyl)amino.

2. The composition for use of claim 1, wherein at least two of R¹, R², R³, R⁴ and R⁵ are independently selected from OH, C₁₋₆ alkoxy, C₁₋₆ haloalkoxy, HO-C₁₋₃ alkyl, NH₂-C₁₋₃ alkyl, HS-C₁₋₃ alkyl, SH, NH₂, C₁₋₆ alkylamino and di(C₁₋₆ alkyl)amino.

3. The composition for use of claim 1, wherein at least one of R¹, R², R³, R⁴ and R⁵ is selected from NH₂ and OH.

4. The composition for use of claim 1, wherein the compound of Formula (I) is selected from any one of the following compounds: 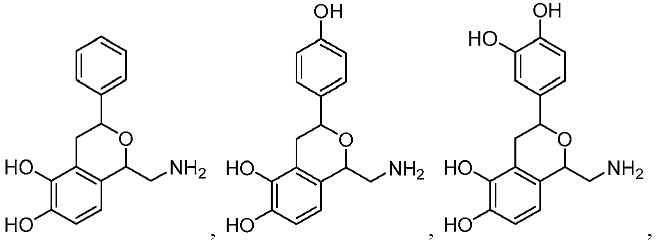 and 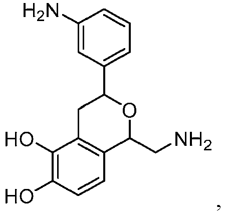 or a pharmaceutically acceptable salt thereof.

5. The composition for use of any one of claims 1-4, wherein the mesenchymal cell is a fibroblast or a stellate cell.

6. The composition for use of any one of claims 1-5, wherein the fibrotic pathology is selected from interstitial lung disease (ILD), pulmonary fibrosis (PF), idiopathic pulmonary fibrosis (IPF), liver tissue fibrosis, cardiac fibrosis, kidney fibrosis, and skin tissue fibrosis.

7. The composition for use of any one of claims 1-6, further comprising administering to the subject a therapeutically effective amount of an additional therapeutic agent useful in treating a fibrotic pathology, optionally wherein the additional therapeutic agent is dopamine, or a pharmaceutically acceptable salt thereof.

8. An *in vitro* method of:
• agonizing a Gα_{S} protein coupled receptor in a cell; and/or
• promoting YAP/TAZ phosphorylation in a cell; and/or
• inhibiting YAP/TAZ function in a cell; and/or
• inhibiting expression of a profibrotic gene in a cell; and/or
• reducing nuclear localization of YAP/TAZ in a cell; and/or
• inhibiting expressing of α-smooth muscle actin (*αSMA*) in a cell; and/or
• inhibiting extra-cellular matrix production and deposition by a cell; and/or
• enhancing extra-cellular matrix degradation by a cell;
the method comprising contacting the cell with an effective amount of a compound of Formula (I): 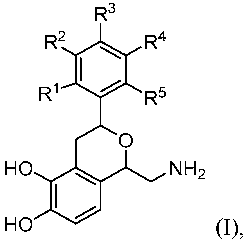 or a pharmaceutically acceptable salt thereof, wherein:
each of R¹, R², R³, R⁴ and R⁵ is independently selected from H, OH, C₁₋₆ alkoxy, C₁₋₆ haloalkoxy, HO-C₁₋₃ alkyl, NH₂-C₁₋₃ alkyl, HS-C₁₋₃ alkyl, SH, NH₂, C₁₋₆ alkylamino and di(C₁₋₆ alkyl)amino,
with the proviso that the compound of Formula (I) is not any one of the following compounds:

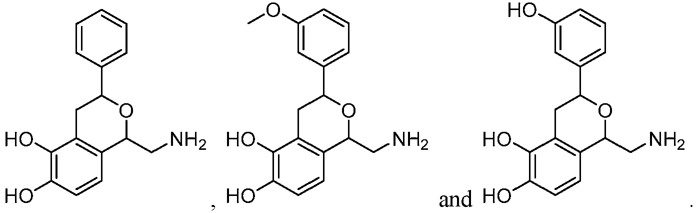

9. The method of claim 8, wherein at least two of R¹, R², R³, R⁴ and R⁵ are OH, or wherein at least one of R¹, R², R³, R⁴ and R⁵ is NH₂.

10. The method of claim 8, wherein the compound of Formula (I) is selected from any one of the following compounds: 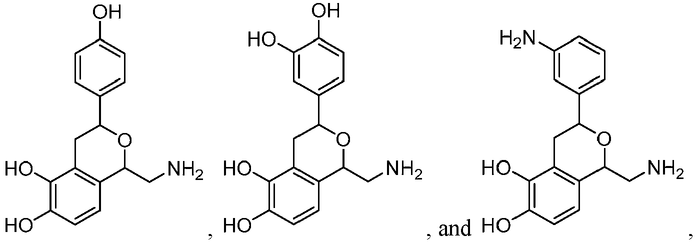 or a pharmaceutically acceptable salt thereof.

11. A compound of Formula (I): 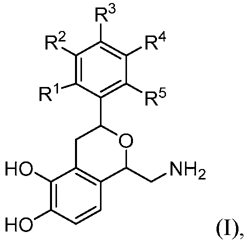 or a pharmaceutically acceptable salt thereof, wherein:
each of R¹, R², R³, R⁴ and R⁵ is independently selected from H, OH, C₁₋₆ haloalkoxy, HO-C₁₋₃ alkyl, NH₂-C₁₋₃ alkyl, HS-C₁₋₃ alkyl, SH, NH₂, C₁₋₆ alkylamino and di(C₁₋₆ alkyl)amino,
with the proviso that the compound of Formula (I) is not any one of the following compounds:

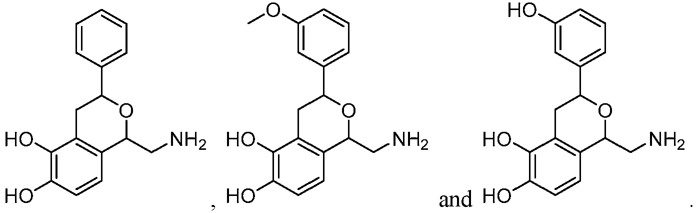

12. The compound of claim 11, wherein the compound of Formula (I) is selected from any one of the following compounds: 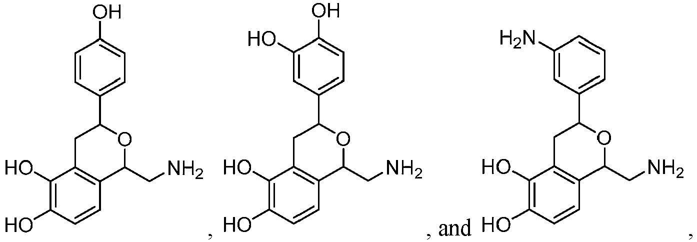 or a pharmaceutically acceptable salt thereof.

13. The compound of claim 11, wherein R³ is OH.

14. The compound of claim 11, wherein at least two of R¹, R², R³, R⁴ and R⁵ are OH.

15. The compound of claim 11, wherein at least one of R¹, R², R³, R⁴ and R⁵ is NH₂.

## Patentansprüche

1. Zusammensetzung, umfassend einen Agonisten eines Gαₛ-Protein-gekoppelten Rezeptors oder ein pharmazeutisch unbedenkliches Salz davon zur Verwendung bei einem Verfahren zur Behandlung oder Prävention einer fibrotischen Pathologie, wobei das Verfahren die Verabreichung einer therapeutisch wirksamen Menge der Zusammensetzung an ein Individuum, bei dem diesbezüglicher Bedarf besteht, umfasst,
wobei der Gαₛ-Protein-gekoppelte Rezeptor bevorzugt in Mesenchymzellen im Vergleich zu Epithel- oder Endothelzellen exprimiert wird und der Agonist für den Gαₛ-Rezeptor, der bevorzugt in der Mesenchymzelle exprimiert wird, spezifisch ist,
wobei es sich bei dem Gαₛ-Protein-gekoppelten Rezeptor um einen Dopaminrezeptor handelt und
wobei der Agonist sowohl D1- als auch D5-Dopaminrezeptoren agonisiert,
wobei der Agonist aus Dihydrexidin (DHX), SKF-81297, SKF-82958, SKF-38393, Fenoldopam, 6-Br-APB, A-68930, A-77636, CY-208-243, Pergolid, R(-)-2,10,11-Trihydroxyaporphin, (R)-(-)-Apomorphin, R(-)-Propylnorapomorphin, R(+)-6-Brom-APB, R(-)-2,10,11-Trihydroxy-N-propylnoraporphin, 6,7-ADTN, Mesulergin, N-Methyldopamin, 4-Hydroxyphenethylamin, 3-Hydroxyphenethylamin, Pramipexol, PD-168077, (±)-PD 128-907, (±)-2-(N-Phenylethyl-N-propyl)amino-5-hydroxytetralin, Bromocriptin, Ropinirol, LY-163-502, Dipropyldopamin, B-HT 920, Piribedil, (+)-UH 232, (-)-Quinpirol, R(-)-2,11-Dihydroxy-10-methoxyapomorphin oder einem pharmazeutisch unbedenklichen Salz davon ausgewählt ist
oder wobei es sich bei dem Agonisten um eine Verbindung der Formel (I): 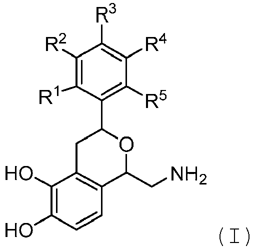 oder ein pharmazeutisch unbedenkliches Salz davon handelt, wobei:
R¹, R², R³, R⁴ und R⁵ jeweils unabhängig aus H, OH, C₁₋₆-Alkoxy, C₁₋₆-Halogenalkoxy, HO-C₁₋₃-Alkyl, NH₂-C₁₋₃-Alkyl, HS-C₁₋₃-Alkyl, SH, NH₂, C₁₋₆-Alkylamino und Di (C₁₋₆-alkyl)amino ausgewählt sind, gegebenenfalls wobei mindestens eines von R¹, R², R³, R⁴ und R⁵ aus OH, C₁₋₆-Alkoxy, C₁₋₆-Halogenalkoxy, HO-C₁₋₃-Alkyl, NH₂-C₁₋₃-Alkyl, HS-C₁₋₃-Alkyl, SH, NH₂, C₁₋₆-Alkylamino und Di (C₁₋₆-alkyl)amino ausgewählt ist.

2. Zusammensetzung zur Verwendung nach Anspruch 1, wobei mindestens zwei von R¹, R², R³, R⁴ und R⁵ unabhängig aus OH, C₁₋₆-Alkoxy, C₁₋₆-Halogenalkoxy, HO-C₁₋₃-Alkyl, NH₂-C₁₋₃-Alkyl, HS-C₁₋₃-Alkyl, SH, NH₂, C₁₋₆-Alkylamino und Di (C₁₋₆-alkyl) amino ausgewählt sind.

3. Zusammensetzung zur Verwendung nach Anspruch 1, wobei mindestens eines von R¹, R², R³, R⁴ und R⁵ aus NH₂ und OH ausgewählt ist.

4. Zusammensetzung zur Verwendung nach Anspruch 1, wobei die Verbindung der Formel (I) ausgewählt ist aus einer der folgenden Verbindungen: 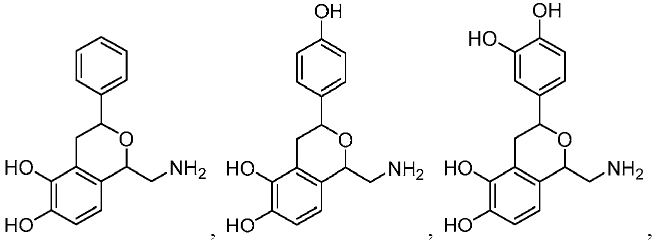 und 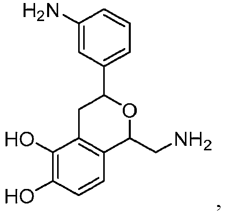 oder einem pharmazeutisch unbedenklichen Salz davon.

5. Zusammensetzung zur Verwendung nach einem der Ansprüche 1-4, wobei es sich bei der Mesenchymzelle um einen Fibroblasten oder eine Sternzelle handelt.

6. Zusammensetzung zur Verwendung nach einem der Ansprüche 1-5, wobei die fibrotische Pathologie aus interstitieller Lungenerkrankung (Interstitial Lung Disease, ILD), Lungenfibrose (Pulmonary Fibrosis, PF), ideopathischer Lungenfibrose (Idiopathic Pulmonary Fibrosis, IPF), Lebergewebefibrose, Herzfibrose, Nierenfibrose und Hauptgewebefibrose ausgewählt ist.

7. Zusammensetzung zur Verwendung nach einem der Ansprüche 1-6, ferner umfassend die Verabreichung einer therapeutisch wirksame Menge eines zusätzlichen therapeutischen Mittels, das bei der Behandlung einer fibrotischen Pathologie nützlich ist, an das Individuum, gegebenenfalls wobei es sich bei dem zusätzlichen therapeutischen Mittel um Dopamin oder ein pharmazeutisch unbedenkliches Salz davon handelt.

8. In-vitro-Verfahren zur:
• Agonisierung eines Gαₛ-Protein-gekoppelten Rezeptors in einer Zelle und/oder
• Förderung der YAP/TAZ-Phosphorylierung in einer Zelle und/oder
• Inhibierung der YAP/TAZ-Funktion in einer Zelle und/oder
• Inhibierung der Expression eines profibrotischen Gens in einer Zelle und/oder
• Reduzierung der Kernlokalisation von YAP/TAZ in einer Zelle und/oder
• Inhibierung der Expression von α-Glattmuskel-Actin *(αSMA)* in einer Zelle und/oder
• Inhibierung der Produktion und Ablagerung von extrazellulärer Matrix durch eine Zelle und/oder
• Verstärkung des Abbaus von extrazellulärer Matrix durch eine Zelle;
wobei das Verfahren das Inkontaktbringen der Zelle mit einer wirksamen Menge einer Verbindung der Formel (I): 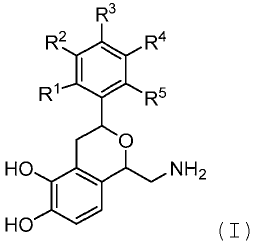
oder eines pharmazeutisch unbedenklichen Salz davon umfasst, wobei:
R¹, R², R³, R⁴ und R⁵ jeweils unabhängig aus H, OH, C₁₋₆-Alkoxy, C₁₋₆-Halogenalkoxy, HO-C₁₋₃-Alkyl, NH₂-C₁₋₃-Alkyl, HS-C₁₋₃-Alkyl, SH, NH₂, C₁₋₆-Alkylamino und Di (C₁₋₆-alkyl)amino ausgewählt sind,
mit der Maßgabe, dass es sich bei der Verbindung der Formel (I) nicht um eine der folgenden Verbindungen handelt:

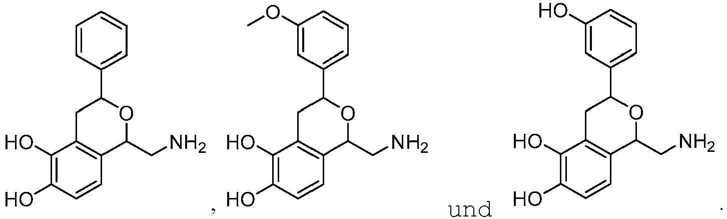

9. Verfahren nach Anspruch 8, wobei mindestens zwei von R¹, R², R³, R⁴ und R⁵ für OH stehen oder wobei mindestens eines von R¹, R², R³, R⁴ und R⁵ für NH₂ steht.

10. Verfahren nach Anspruch 8, wobei die Verbindung der Formel (I) ausgewählt ist aus einer der folgenden Verbindungen: 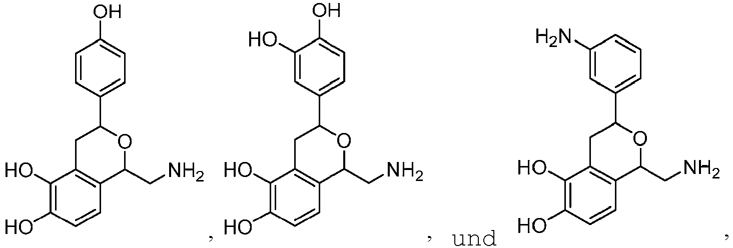 oder einem pharmazeutisch unbedenklichen Salz davon.

11. Verbindung der Formel (I): 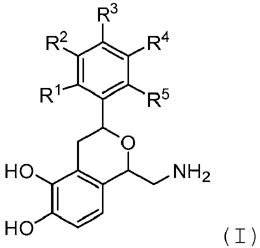 oder pharmazeutisch unbedenkliches Salz davon, wobei:
R¹, R², R³, R⁴ und R⁵ jeweils unabhängig aus H, OH, C₁₋₆-Halogenalkoxy, HO-C₁₋₃-Alkyl, NH₂-C₁₋₃-Alkyl, HS-C₁₋₃-Alkyl, SH, NH₂, C₁₋₆-Alkylamino und Di (C₁₋₆-alkyl) amino ausgewählt sind,
mit der Maßgabe, dass es sich bei der Verbindung der Formel (I) nicht um eine der folgenden Verbindungen handelt:

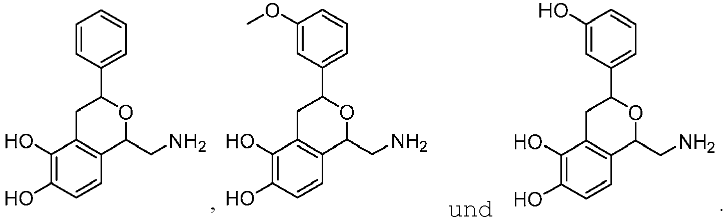

12. Verbindung nach Anspruch 11, wobei die Verbindung der Formel (I) ausgewählt ist aus einer der folgenden Verbindungen: 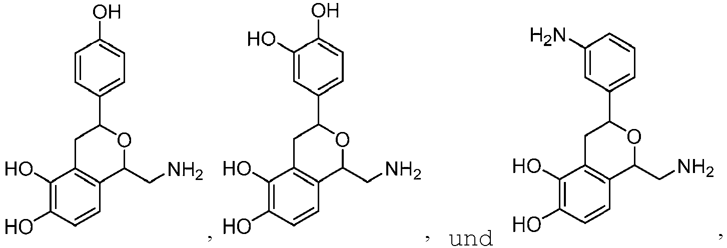 oder einem pharmazeutisch unbedenklichen Salz davon.

13. Verbindung nach Anspruch 11, wobei R³ für OH steht.

14. Verbindung nach Anspruch 11, wobei mindestens zwei von R¹, R², R³, R⁴ und R⁵ für OH stehen.

15. Verbindung nach Anspruch 11, wobei mindestens eines von R¹, R², R³, R⁴ und R⁵ für NH₂ steht.

## Revendications

1. Composition comprenant un agoniste du récepteur couplé aux protéines Gαₛ, ou un sel pharmaceutiquement acceptable correspondant pour une utilisation dans un procédé de traitement ou de prévention d'une pathologie fibrotique, le procédé comprenant une administration à un sujet qui en a besoin d'une quantité thérapeutique efficace de ladite composition,
dans laquelle le récepteur couplé aux protéines Gαₛ est exprimé de préférence dans des cellules mésenchymateuses par rapport aux cellules épithéliales ou endothéliales, et l'agoniste est spécifique pour le récepteur Gαₛ qui est exprimé de préférence dans la cellule mésenchymateuse,
dans laquelle le récepteur couplé aux protéines Gαₛ est un récepteur de dopamine, et
dans laquelle l'agoniste agonise à la fois les récepteurs de dopamine D1 et D5,
dans laquelle l'agoniste est sélectionné parmi dihydrexidine (DHX), SKF-81297, SKF-82958, SKF-38393, fénoldopam, 6-Br-APB, A-68930, A-77636, CY-208-243, le pergolide, R(-)-2,10,11-trihydroxyaporphine, (R)-(-)-apomorphine, R(-)-propylnorapomorphine, R(+)-6-bromo-APB, R(-)-2,10,11-trihydroxy-N-propyl-noraporphine, 6,7-ADTN, mésulergine, N-méthyldopamine, 4-hydroxyphénéthylamine, 3-hydroxyphénéthylamine, pramipexole, PD-168077, (±)-PD 128-907, (±)-2-(N-phényléthyl- N-propyl)amino-5-hydroxytétraline, bromocriptine, ropinirole, LY-163-502, dipropyldopamine, B-HT 920, piribédil, (+)-UH 232, (-)-quinpirole, R(-)-2,11 -dihydroxy-10-méthoxyapomorphine, ou un sel pharmaceutiquement acceptable correspondant,
ou dans laquelle l'agoniste est un composé de Formule (I) 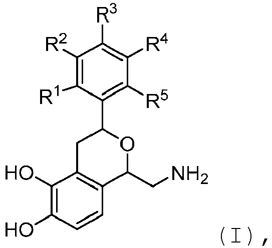 ou un sel pharmaceutiquement acceptable correspondant, chacun parmi R¹, R², R³, R⁴ et R⁵ étant indépendamment choisi parmi H, OH, C₁₋₆ alcoxy, C₁₋₆ halogénoalcoxy, HO-C₁₋₃ alkyle, NH₂-C₁₋₃ alkyle, HS-C₁₋₃ alkyle, SH, NH₂, C₁₋₆ alkylamino et di (C₂₋₆ alkyl)amino, éventuellement, au moins l'un parmi R¹, R², R³, R⁴ et R⁵ étant choisi parmi OH, C₁₋₆ alcoxy, C₁₋₆ halogénoalcoxy, HO-C₁₋₃ alkyle, NH₂-C₁₋₃ alkyle, HS-C₁₋₃ alkyle, SH, NH₂, C₁₋₆ alkylamino et di (C₁₋₆ alkyl)amino.

2. Composition pour une utilisation selon la revendication 1, au moins deux parmi R¹, R², R³, R⁴ et R⁵ étant indépendamment choisis parmi OH, C₁₋₆ alcoxy, C₁₋₆ halogénoalcoxy, HO-C₁₋₃ alkyle, NH₂-C₁₋₃ alkyle, HS-C₁₋₃ alkyle, SH, NH₂, C₁₋₆ alkylamino et di (C₁₋₆ alkyl)amino.

3. Composition pour une utilisation selon la revendication 1, au moins l'un parmi R¹, R², R³, R⁴ et R⁵ étant choisi parmi NH₂ et OH.

4. Composition pour une utilisation selon la revendication 1, le composé de formule (I) étant choisi parmi l'un quelconque des composés suivants : 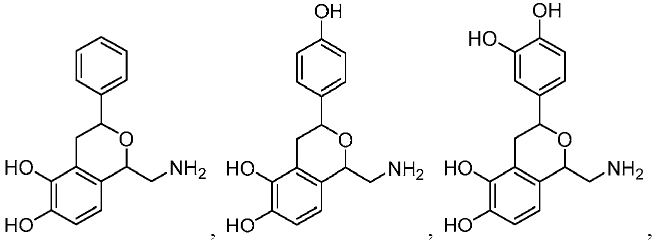 et 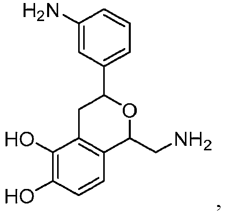 ou un sel pharmaceutiquement acceptable correspondant.

5. Composition pour une utilisation selon l'une quelconque des revendications 1-4, dans laquelle la cellule mésenchymateuse est un fibroblaste ou une cellule de stellate.

6. Composition pour une utilisation selon l'une quelconque des revendications 1-5, dans laquelle la pathologie fibrotique est sélectionnée parmi la maladie pulmonaire interstitielle (MPI), la fibrose pulmonaire (FP), la fibrose pulmonaire idiopathique (FPI), la fibrose des tissus hépatiques, la fibrose cardiaque, la fibrose rénale et la fibrose des tissus cutanés.

7. Composition pour une utilisation selon l'une quelconque des revendications 1-6, comprenant en outre l'administration au sujet d'une quantité thérapeutiquement efficace d'un agent thérapeutique supplémentaire utile dans le traitement d'une pathologie fibrotique, dans laquelle éventuellement l'agent thérapeutique supplémentaire est la dopamine, ou un sel pharmaceutiquement acceptable correspondant.

8. Procédé *in vitro* de :
• agonisation d'un récepteur couplé aux protéines Gαₛ dans une cellule ; et/ou
• favorisation de la phosphorylation YAP/TAZ dans une cellule ; et/ou
• inhibition de la fonction YAP/TAZ dans une cellule ; et/ou
• inhibition de l'expression d'un gène profibrotique dans une cellule ; et/ou
• réduction de la localisation nucléaire de YAP/TAZ dans une cellule ; et/ou
• inhibition de l'expression de l'α-actine du muscle lisse *(αSMA)* dans une cellule ; et/ou
• inhibition de la production et du dépôt de matrice extra-cellulaire par une cellule ; et/ou
• amélioration de la dégradation de la matrice extra-cellulaire par une cellule ;
le procédé comprenant la mise en contact de la cellule avec une quantité efficace d'un composé de formule (I) : 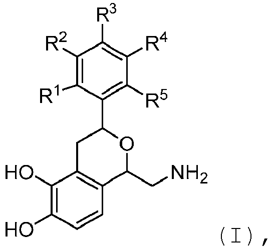
ou un sel pharmaceutiquement acceptable correspondant, chacun parmi R¹, R², R³, R⁴ et R⁵ étant indépendamment choisi parmi H, OH, C₁₋₆ alcoxy, C₁₋₆ halogénoalcoxy, HO-C₁₋₃ alkyle, NH₂-C₁₋₃ alkyle, HS-C₁₋₃ alkyle, SH, NH₂, C₁₋₆ alkylamino et di (C₁₋₆ alkyl)amino,
à condition que le composé de la formule (I) ne soit pas l'un quelconque des composés suivants :

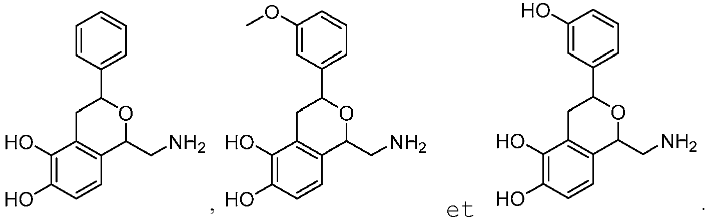

9. Procédé selon la revendication 8, au moins deux parmi R¹, R², R³, R⁴ et R⁵ étant OH, ou, au moins l'un parmi R¹, R², R³, R⁴ et R⁵ étant NH₂.

10. Procédé selon la revendication 8, le composé de formule (I) étant choisi parmi l'un quelconque des composés suivants : 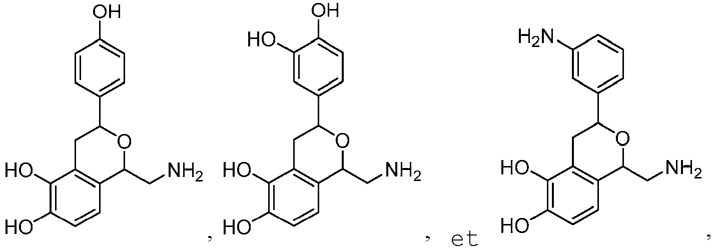 ou un sel pharmaceutiquement acceptable correspondant.

11. Composé de formule (I) : 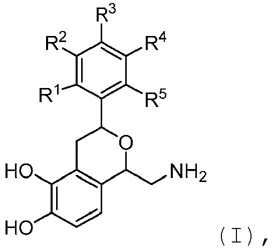 ou un sel pharmaceutiquement acceptable correspondant, chacun parmi R¹, R², R³, R⁴ et R⁵ étant indépendamment choisi parmi H, OH, C₁₋₆ halogénoalcoxy, HO-C₁₋₃ alkyle, NH₂-C₁₋₃ alkyle, HS-C₁₋₃ alkyle, SH, NH₂, C₁₋₆ alkylamino et di (C₁₋₆ alkyl) amino,
à condition que le composé de la formule (I) ne soit pas l'un quelconque des composés suivants :

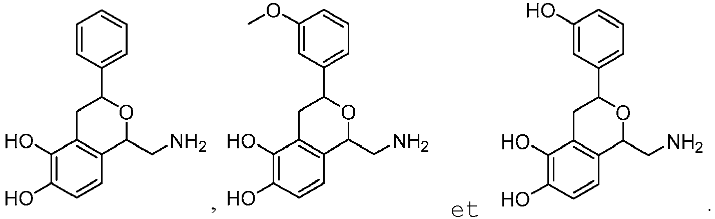

12. Composé selon la revendication 11, le composé de formule (I) étant choisi parmi l'un quelconque des composés suivants : 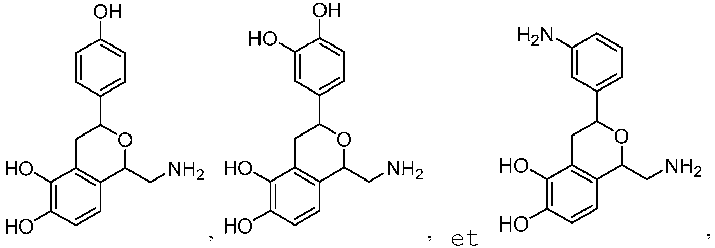 ou un sel pharmaceutiquement acceptable correspondant.

13. Composé selon la revendication 11, R³ étant OH.

14. Composé selon la revendication 11, au moins deux parmi R¹, R², R³, R⁴ et R⁵ étant OH.

15. Composé selon la revendication 11, au moins l'un parmi R¹, R², R³, R⁴ et R⁵ étant NH₂.
